# EUROPEAN PATENT SPECIFICATION

(11) **EP 3 545 104 B1**
(45) Date of publication and mention of the grant of the patent: **24.02.2021**
(21) Application number: 17837944.2
(22) Date of filing: 23.11.2017
(51) Int. Cl.: C12Q 1/68

(54) **TANDEM BARCODING OF TARGET MOLECULES FOR THEIR ABSOLUTE QUANTIFICATION AT SINGLE-ENTITY RESOLUTION**
TANDEM-BARCODIERUNG VON ZIELMOLEKÜLEN ZU DEREN ABSOLUTER QUANTIFIZIERUNG BEI EINHEITSAUFLÖSUNG
CODAGE À BARRES EN TANDEM DE MOLÉCULES CIBLES POUR LEUR QUANTIFICATION ABSOLUE À UNE RÉSOLUTION D'ENTITÉ UNIQUE

(30) Priority: 23.11.2016 EP 16306543
(43) Date of publication of application: 02.10.2019
(73) Proprietor: Université de Strasbourg, 67000 Strasbourg (FR); Centre National de la Recherche Scientifique, 75016 Paris (FR)
(72) Inventor: RYCKELYNCK, Michaël, 67100 Strasbourg (FR); BAUDREY, Stéphanie, 67380 Lingolsheim (FR)
(74) Representative: Cabinet Becker et Associés
(86) International application number: PCT/EP2017/080264
(87) International publication number: WO 2018/096058

(56) References cited:
- WO-A1-2013/123442
- WO-A1-2015/164212
- WO-A1-2016/044227
- D. A. JAITIN ET AL: "Massively Parallel Single-Cell RNA-Seq for Marker-Free Decomposition of Tissues into Cell Types", SCIENCE, vol. 343, no. 6172, 14 February 2014 (2014-02-14), pages 776-779, XP055111761, ISSN: 0036-8075, DOI: 10.1126/science.1247651 -& DIEGO ADHEMAR JAITIN ET AL: "Supplementary Material: Massively Parallel Single-Cell RNA-Seq for Marker-Free Decomposition of Tissues into Cell Types", INTERNET CITATION, 14 February 2014 (2014-02-14), pages 1-35, XP002722806, ISSN: 0036-8075 Retrieved from the Internet: URL:http://www.sciencemag.org/content/supp l/2014/02/12/343.6172.776.DC1/Jaitin-SM.pd f [retrieved on 2014-04-04]
- ISLAM S ET AL: "Quantitative single-cell RNA-seq with unique molecular identifiers", NATURE METHODS,, vol. 11, no. 2, 1 February 2014 (2014-02-01), pages 163-166, XP002733949, ISSN: 1548-7091, DOI: 10.1038/NMETH.2772 [retrieved on 2013-12-22]
- ASSAF ROTEM ET AL: "High-Throughput Single-Cell Labeling (Hi-SCL) for RNA-Seq Using Drop-Based Microfluidics", PLOS ONE, vol. 10, no. 5, 22 May 2015 (2015-05-22), page e0116328, XP055227089, DOI: 10.1371/journal.pone.0116328 cited in the application
- ALON S ET AL: "Barcoding bias in high-throughput multiplex sequencing of miRNA", GENOME RESEARCH, COLD SPRING HARBOR LABORATORY PRESS, US, vol. 21, no. 9, 1 September 2011 (2011-09-01), pages 1506-1511, XP002720457, ISSN: 1088-9051, DOI: 10.1101/GR.121715.111 [retrieved on 2011-07-12]
- EVAN Z. MACOSKO ET AL: "Highly Parallel Genome-wide Expression Profiling of Individual Cells Using Nanoliter Droplets", CELL, vol. 161, no. 5, 1 May 2015 (2015-05-01), pages 1202-1214, XP055323004, US ISSN: 0092-8674, DOI: 10.1016/j.cell.2015.05.002 cited in the application
- MARIE-JEANNE ARGUEL ET AL: "A cost effective 5' selective single cell transcriptome profiling approach with improved UMI design", NUCLEIC ACIDS RESEARCH, vol. 45, no. 7, 9 December 2016 (2016-12-09), pages e48-e48, XP055367026, ISSN: 0305-1048, DOI: 10.1093/nar/gkw1242
- RAPOLAS ZILIONIS ET AL: "Single-cell barcoding and sequencing using droplet microfluidics", NATURE PROTOCOLS, vol. 12, no. 1, 8 December 2016 (2016-12-08), pages 44-73, XP055367764, GB ISSN: 1754-2189, DOI: 10.1038/nprot.2016.154

### SEQUENCE LISTING

<110> UNIVERSITE DE STRASBOURG CENTRE NATIONAL DE LA RECHERCHE SCIENTIFIQUE
<120> Tandem barcoding of target molecules for their absolute quantification at single-entity resolution
<130> B2367PC
<160> 37
<170> Patent In version 3.5
<210> 1
   <211> 62
   <212> DNA
   <213> Artificial Sequence
<220>
   <223> Template UEI-DraIII
<400> 1
   gagcggataa caatttcaca caggcacggg gtgtgagatc acagatcgga agagcgtcgt 60
   gt 62
<210> 2
   <211> 24
   <212> DNA
   <213> Artificial Sequence
<220>
   <223> UEI-Fwd
<400> 2
   gagcggataa caatttcaca cagg 24
<210> 3
   <211> 19
   <212> DNA
   <213> Artificial Sequence
<220>
   <223> UEI-Rev
<400> 3
   acacgacgct cttccgatc 19
<210> 4
   <211> 142
   <212> DNA
   <213> Artificial Sequence
<220>
   <223> Template Calibrator-N15-AlwNI
<220>
   <221> misc_feature
   <222> (86)..(110)
   <223> n is a, c, g, or t
<400> 4
   tcgtcggcag cgtcagatgt gtataagaga cagggaagac gtagcaaggg agtagccaat 60
   gtgaattgag agccttaagc tgtatnnnnn nnnnnnnnnn nnnnnnnnnn caggggctgg 120
   tcgtgactgg gaaaaccctg gc 142
<210> 5
   <211> 23
   <212> DNA
   <213> Artificial Sequence
<220>
   <223> Calib-Rev
<400> 5
   gccagggttt tcccagtcac gac 23
<210> 6
   <211> 19
   <212> DNA
   <213> Artificial Sequence
<220>
   <223> Illu-2-Fwd
<400> 6
   tcgtcggcag cgtcagatg 19
<210> 7
   <211> 67
   <212> DNA
   <213> Artificial Sequence
<220>
   <223> Template UEI-N15-DraIII*
<220>
   <221> misc_feature
   <222> (34)..(48)
   <223> n is a, c, g, or t
<400> 7
   gagcggataa caatttcaca caggcacggg gtgnnnnnnn nnnnnnnnga tcggaagagc 60
   gtcgtgt 67
<210> 8
   <211> 149
   <212> DNA
   <213> Artificial Sequence
<220>
   <223> Template Calibrator-N15-AlwNI-lg
<220>
   <221> misc_feature
   <222> (103)..(117)
   <223> n is a, c, g, or t
<400> 8
   tcgtcggcag cgtcagatgt gtataagaga cagggaagac gtagcaaggg agtagccaat 60
   gtgaattgag agccttaagc tgtatcagga ccagagagat gannnnnnnn nnnnnnncag 120
   gggctggtcg tgactgggaa aaccctggc 149
<210> 9
   <211> 53
   <212> DNA
   <213> Artificial Sequence
<220>
   <223> Illu1 rev UEI
<400> 9
   gtctcgtggg ctcggagatg tgtataagag acagacacga cgctcttccg atc 53
<210> 10
   <211> 19
   <212> DNA
   <213> Artificial Sequence
<220>
   <223> Illu1
<400> 10
   gtctcgtggg ctcggagat 19
<210> 11
   <211> 90
   <212> DNA
   <213> Artificial Sequence
<220>
   <223> Template-AlwNI-N15-Calib-DraIII
<220>
   <221> misc_feature
   <222> (44)..(58)
   <223> n is a, c, g, or t
<400> 11
   ggaagacgta gcaagggagt agccaatgtg aattcacaca gtgnnnnnnn nnnnnnnnca 60
   ggggctggtc gtgactggga aaaccctggc 90
<210> 12
   <211> 24
   <212> DNA
   <213> Artificial Sequence
<220>
   <223> Calib-Fwd
<400> 12
   ggaagacgta gcaagggagt agcc 24
<210> 13
   <211> 80
   <212> DNA
   <213> Artificial Sequence
<220>
   <223> RTmimicks
<220>
   <221> misc_feature
   <222> (36)..(43)
   <223> n is a, c, g, or t
<400> 13
   ctgcgcggat cccggaagcg aggccagctg gctgcnnnnn nnnttggctg tctcttatac        60
   acatctgacg ctgccgacga                                                    80
<210> 14
   <211> 38
   <212> DNA
   <213> Artificial Sequence
<220>
   <223> antiSBACA-AlwN1
<400> 14
   gcagccagct ggcctcgctt ccgggatccg cgcagaca 38
<210> 15
   <211> 101
   <212> DNA
   <213> Artificial Sequence
<220>
   <223> Template UEI-N15-DraIII-Illu1
<220>
   <221> misc_feature
   <222> (34)..(48)
   <223> n is a, c, g, or t
<400> 15
   gagcggataa caatttcaca caggcacggg gtgnnnnnnn nnnnnnnnga tcggaagagc        60
   gtcgtgtctg tctcttatac acatctccga gcccacgaga c                           101
<210> 16
   <211> 539
   <212> RNA
   <213> Staphylococcus aureus
<400> 16
   auuaauacga cucacuauac cuagaucaca gagaugugau ggaaaauagu ugaugaguug     60
   uuuaauuuua agaauuuuua ucuuaauuaa ggaaggagug auuucaaugg cacaagauau    120
   cauuucaaca aucggugacu uaguaaaaug gauuaucgac acagugaaca aauucacuaa    180
   aaaauaagau gaauaauuaa uuacuuucau uguaaauuug uuaucuacgu auaguacuaa    240
   aaguaugagu uauuaagcca ucccaacuua auaaccaugu aaaauuagca agugaguaac    300
   auuugcuagu agaguuaguu uccuuggacu cagugcuaug uauuuuucuu aauuaucauu    360
   acagauaauu auuucuagca uguaagcuau cguaaacaac aucgauuuau cauuauuuga    420
   uaaauaaaau uuuuuucaua auuaauaaca uccccaaaaa uagauugaaa aaauaacugu    480
   aaaacauucc cuuaauaaua aguauggucg ugagccccuc ccaagcucgc ggccuuuug      539
<210> 17
   <211> 10
   <212> DNA
   <213> Artificial Sequence
<220>
   <223> empty
<220>
   <221> misc_feature
   <222> (1)..(10)
   <223> n is a, c, g, or t
<400> 17
   nnnnnnnnnn 10
<210> 18
   <211> 55
   <212> DNA
   <213> Artificial Sequence
<220>
   <223> RT-UMI-RNAIII*
<220>
   <221> misc_feature
   <222> (36)..(41)
   <223> n is a, c, g, or t
<400> 18
   ctgcgcggat cccggaagcg aggccagctg gctgcnnnnn nttgggaggg gctca 55
<210> 19
   <211> 10
   <212> DNA
   <213> Artificial Sequence
<220>
   <223> empty
<220>
   <221> misc_feature
   <222> (1)..(10)
   <223> n is a, c, g, or t
<400> 19
   nnnnnnnnnn 10
<210> 20
   <211> 35
   <212> DNA
   <213> Artificial Sequence
<220>
   <223> SB-alone
<400> 20
   ctgcgcggat cccggaagcg aggccagctg gctgc 35
<210> 21
   <211> 63
   <212> DNA
   <213> Artificial Sequence
<220>
   <223> RNAIII-Fwd
<400> 21
   tcgtcggcag cgtcagatgt gtataagaga cagataactg taaaacattc ccttaataat 60
   aag 63
<210> 22
   <211> 867
   <212> RNA
   <213> Artificial Sequence
<220>
   <223> Gfp mRNA
<400> 22
   augaccagcu acccauacga uguuccagau uacgcuaucg aaggccgcgg ccgccaucau 60
   caucaucauc augauaucgg uaccaguaaa ggagaagaac uuuucacugg aguuguccca 120
   auucuuguug aauuagaugg ugauguuaau gggcacaaau uuucugucag uggagagggu 180
   gaaggugaug caacauacgg aaaacuuacc cuuaaauuua uuugcacuac uggaaaacua 240
   ccuguuccau ggccaacacu ugucacuacu uucgcguaug gucuucaaug cuuugcgaga 300
   uacccagauc auaugaaaca gcaugacuuu uucaagagug ccaugcccga agguuaugua 360
   caggaaagaa cuauauuuuu caaagaugac gggaacuaca agacacgugc ugaagucaag 420
   uuugaaggug auacccuugu uaauagaauc gaguuaaaag guauugauuu uaaagaagau 480
   ggaaacauuc uuggacacaa auuggaauac aacuauaacu cacacaaugu auacaucaug 540
   gcagacaaac aaaagaaugg aaucaaaguu aacuucaaaa uuagacacaa cauugaagau 600
   ggaagcguuc aacuagcaga ccauuaucaa caaaauacuc caauuggcga uggcccuguc 660
   cuuuuaccag acaaccauua ccuguccaca caaucugccc uuucgaaaga ucccaacgaa 720
   aagagagacc acauggcccu ucuugaguuu guaacagcug cugggauuac acauggcaug 780
   gaugaacuau acaaagagaa uucagagcuc ggauccacuc gagaugcauu agaacaaaaa 840
   uuauuaucag aagaagauuu aaauuaa 867
<210> 23
   <211> 69
   <212> DNA
   <213> Artificial Sequence
<220>
   <223> gfp-mut2-RT-UMI-AlwNI*
<220>
   <221> misc_feature
   <222> (36)..(43)
   <223> n is a, c, g, or t
<400> 23
   ctgcgcggat cccggaagcg aggccagctg gctgcnnnnn nnncaaataa atttaagggt 60
   aagttttcc 69
<210> 24
   <211> 26
   <212> DNA
   <213> Artificial Sequence
<220>
   <223> Gfp-Fwd-25nt
<400> 24
   tgaattagat ggtgatgtta atgggc 26
<210> 25
   <211> 70
   <212> DNA
   <213> Artificial Sequence
<220>
   <223> Template-UEI-DraIII-N2x5
<220>
   <221> misc_feature
   <222> (34)..(35)
   <223> n is a, c, g, or t
<220>
   <221> misc_feature
   <222> (38)..(39)
   <223> n is a, c, g, or t
<220>
   <221> misc_feature
   <222> (42)..(43)
   <223> n is a, c, g, or t
<220>
   <221> misc_feature
   <222> (46)..(47)
   <223> n is a, c, g, or t
<220>
   <221> misc_feature
   <222> (50)..(51)
   <223> n is a, c, g, or t
<400> 25
   gagcggataa caatttcaca caggcacggg gtgnnacnng annctnngcn ngatcggaag 60
   agcgtcgtgt 70
<210> 26
   <211> 76
   <212> DNA
   <213> Artificial Sequence
<220>
   <223> Template-UEI-DraIII-N4443
<220>
   <221> misc_feature
   <222> (34)..(37)
   <223> n is a, c, g, or t
<220>
   <221> misc_feature
   <222> (41)..(44)
   <223> n is a, c, g, or t
<220>
   <221> misc_feature
   <222> (48)..(51)
   <223> n is a, c, g, or t
<220>
   <221> misc_feature
   <222> (55)..(57)
   <223> n is a, c, g, or t
<400> 26
   gagcggataa caatttcaca caggcacggg gtgnnnnaca nnnngagnnn ntctnnngat 60
   cggaagagcg tcgtgt 76
<210> 27
   <211> 85
   <212> DNA
   <213> Artificial Sequence
<220>
   <223> Template-Calibrator-AlwNI
<400> 27
   ggaagacgta gcaagggagt agccaatgtg aattcacaca gtgtctacaa gtacaggggc 60
   tggtcgtgac tgggaaaacc ctggc 85
<210> 28
   <211> 93
   <212> DNA
   <213> Artificial Sequence
<220>
   <223> Template-Calibrator-AlwNI-N2x5
<220>
   <221> misc_feature
   <222> (44)..(45)
   <223> n is a, c, g, or t
<220>
   <221> misc_feature
   <222> (48)..(49)
   <223> n is a, c, g, or t
<220>
   <221> misc_feature
   <222> (52)..(53)
   <223> n is a, c, g, or t
<220>
   <221> misc_feature
   <222> (56)..(57)
   <223> n is a, c, g, or t
<220>
   <221> misc_feature
   <222> (60)..(61)
   <223> n is a, c, g, or t
<400> 28
   ggaagacgta gcaagggagt agccaatgtg aattcacaca gtgnntcnnt anncannagn      60
   ncaggggctg gtcgtgactg ggaaaaccct ggc                                   93
<210> 29
   <211> 99
   <212> DNA
   <213> Artificial Sequence
<220>
   <223> Template-Calibrator-AlwNI-N4443
<220>
   <221> misc_feature
   <222> (44)..(47)
   <223> n is a, c, g, or t
<220>
   <221> misc_feature
   <222> (51)..(54)
   <223> n is a, c, g, or t
<220>
   <221> misc_feature
   <222> (58)..(61)
   <223> n is a, c, g, or t
<220>
   <221> misc_feature
   <222> (65)..(67)
   <223> n is a, c, g, or t
<400> 29
   ggaagacgta gcaagggagt agccaatgtg aattcacaca gtgnnnntct nnnnacannn      60
   ngagnnncag gggctggtcg tgactgggaa aaccctggc                             99
<210> 30
   <211> 90
   <212> DNA
   <213> Artificial Sequence
<220>
   <223> Template-Calibrator-AlwNI-N15
<220>
   <221> misc_feature
   <222> (44)..(58)
   <223> n is a, c, g, or t
<400> 30
   ggaagacgta gcaagggagt agccaatgtg aattcacaca gtgnnnnnnn nnnnnnnnca 60
   ggggctggtc gtgactggga aaaccctggc 90
<210> 31
   <211> 100
   <212> DNA
   <213> Artificial Sequence
<220>
   <223> Tag-NaBAb-AlwNI
<220>
   <221> misc_feature
   <222> (32)..(39)
   <223> n is a, c, g, or t
<400> 31
   cacacaggaa acagctatga cccagtgtct gnnnnnnnnt gtggacgctg tctcttatac 60
   acatctgacg ctgccgacga cgtcgtgact gggaaaaccc 100
<210> 32
   <211> 20
   <212> DNA
   <213> Artificial Sequence
<220>
   <223> BG-M13-Fwd**
<400> 32
   gggttttccc agtcacgacg 20
<210> 33
   <211> 22
   <212> DNA
   <213> Artificial Sequence
<220>
   <223> Alexa488-M13-Rev
<400> 33
   cacacaggaa acagctatga cc 22
<210> 34
   <211> 38
   <212> DNA
   <213> Artificial Sequence
<220>
   <223> DNA based probe specific to RNAIII of Staphylococcus aureus
<400> 34
   gcagccagct ggcctcgctt ccgggatccg cgcagggg 38
<210> 35
   <211> 55
   <212> DNA
   <213> Artificial Sequence
<220>
   <223> DNA based probe specific to RNAIII of Staphylococcus aureus
<220>
   <221> misc_feature
   <222> (36)..(41)
   <223> n is a, c, g, or t
<400> 35
   gatcgcggat cccggaagcg aggccagctg gctgcnnnnn nttgggaggg gctca 55
<210> 36
   <211> 62
   <212> DNA
   <213> Artificial Sequence
<220>
   <223> Example of entity identification sequence
<220>
   <221> misc_feature
   <222> (34)..(43)
   <223> n is a, c, g, or t
<400> 36
   gagcggataa caatttcaca caggcacggg gtgnnnnnnn nnngatcgga agagcgtcgt 60
   gt 62
<210> 37
   <211> 62
   <212> DNA
   <213> Artificial Sequence
<220>
   <223> example of entity identification sequence
<220>
   <221> misc_feature
   <222> (20).. (29)
   <223> n is a, c, g, or t
<400> 37
   acacgacgct cttccgatcn nnnnnnnnnc accccgtgcc tgtgtgaaat tgttatccgc        60
   tc                                                                        62

## Description

### FIELD OF THE INVENTION

The present invention relates to methods and systems for labelling nucleic acids and other biological molecules from entities, e.g. cells, within emulsion droplets in high throughput regimes while preserving the integrity of the single-entity information.

### BACKGROUND OF THE INVENTION

Single cell analytics are gaining popularity due to the insight that taking into account the heterogeneity of a population of cell may be of capital interest to understand the function and behaviour of diverse biological systems. Individual cells of a population of clonal unicellular organisms or of multicellular organisms, even among cells of the same tissue or cell-type, often exhibit heterogeneous gene regulation or protein expression patterns. It is thus increasingly recognized that traditional analytical approaches that analyse large populations of cells yield ensemble views that, although informative, only reflect the dominant biological mechanism and fail to identify cell-to-cell variations.

RNA levels are considered a useful marker of phenotypic heterogeneity and, as a consequence, considerable efforts were done to analyse RNA content in single cells. Probe-dependent methods including fluorescence in situ hybridization (FISH) or reporter fusions to fluorescent proteins, was replaced with the probe-independent RNA-seq technique in which cellular RNA molecules are converted into cDNA and subsequently sequenced in parallel using next-generation sequencing technology. Single-cell RNA-seq requires the isolation of individual cells, the conversion of cellular RNA into cDNA and the massively parallel sequencing of cDNA libraries.

The rapid expansion of microfluidic devices has resulted in the development of valve-based microfluidic chips wherein cells are isolated in nano-liter reaction chambers (Streets et al., 2014). However, this approach remains limited not only by the cost but also because the number of single cells that can be currently processed with said chips remains at less than one hundred per run. Alternatively, microfluidic droplets also provide a compartment in which cells can be isolated. Typically, droplets of one phase are generated in another, immiscible phase by exploiting capillary instabilities in a microfluidic two-phase flow. The addition of a surfactant to either or both of the phases stabilizes the droplets against coalescence and allows them to function as discrete microreactors.

Furthermore, as single-cell RNA-seq analysis may require the profiling of several thousands if not millions of representative individual cells, barcoding strategies have been developed to reduce sequencing costs and increase throughput. Using unique cellular identifiers, it has made possible to pool up a multitude of cells for simultaneous sequencing since each read could subsequently be assigned to its original cell through the unique cellular barcode (Islam et al., 2012).

The main technical challenge when combining barcoding strategy and compartmentalization of cells into droplets is to ensure that each droplet carries a different barcode and thus that the integrity of the single cell information is preserved.

Several methods have been described to address this problem. Each cell may be coencapsulated with a distinctly barcoded particle, such as bead (Macosko et al. 2015) or hydrogel microsphere (Klein et al., 2015), in a nano-liter scale droplet. Each of these particles contains more than 10⁸ individual primers that share the same "cell barcode". In such a method, in order to ensure that a droplet comprises only one particle, the number of droplets created greatly exceeds the number of particles or cells injected, so that a droplet will generally contain zero or one cell and zero or one particle (Macosko et al. 2015; Klein et al., 2015).

Alternatively, a barcode-library emulsion may be produced using a microfluidic device consisting of 96 drop-makers creating millions of drops containing a high concentration of a single one of the 96 barcodes (Rotem et al. 2015). Each cell-bearing drop is then paired and fused with one barcode- drop. However, to ensure that each cell-bearing drop is fused with at most one barcode drop, only half of the cell-bearing drops actually fuse with a barcode drop. Furthermore, cases where two cell-bearing drops fuse with a single barcode drop or where two barcode drops fuse with a single cell-bearing drop, introduce errors in the resultant labelling and are a potential source of noise (Rotem et al. 2015).

Thus, whatever the method used to ensure that each droplet carries a different barcode, this results in a limited droplet occupancy, a reduced useful fraction of droplets, and thus a reduced throughput.

Furthermore, even if RNA levels have been recognized as useful marker for phenotypic heterogeneity, current methods provide limited information since levels of protein or other biological molecules cannot be assessed with the same system. Indeed, to date, quantification of the protein expression at the single cell level, which is critical for complete characterization of the phenotypic states, is generally based on fluorescence imaging methods.

Consequently, there is a great need for new methods and devices allowing high-throughput quantification of RNA, proteins and other biological molecules of interest at the single entity level.

### SUMMARY OF THE INVENTION

The present invention provides a new method of labelling any target molecules from a plurality of entities in high throughput regimes while preserving the integrity of the single-entity information.

Accordingly, in a first aspect, the present invention relates to a method of labelling a plurality of molecular targets from a plurality of entities while preserving the integrity of the single-entity information, said method comprising
providing a first set of emulsion droplets comprising droplets containing labelled molecular targets, wherein each of these droplets contains a plurality of molecular targets originating from no more than one entity and wherein, in each of these droplets, each molecular target is labelled with a molecular identification DNA sequence comprising (i) a unique molecular identification (UMI) barcode which is different for each molecular target and (ii) an overhang or an overhang producing restriction site;
providing a second set of emulsion droplets comprising droplets containing entity identification sequences, wherein each of these droplets contains at least one entity identification sequence which is a DNA sequence, preferably a double stranded DNA sequence, comprising a unique entity identification (UEI) barcode which is different for each droplet of the second set, and an overhang producing restriction site;
fusing droplets of the first set with droplets of the second set wherein a droplet of the first set is fused with no more than one droplet of the second set; and
ligating UEI barcodes to labelled molecular targets after restriction enzyme digestion, and
optionally breaking the emulsion.

The method may further comprise
encapsulating a plurality of entities within emulsion droplets, each droplet containing no more than one entity, and optionally lysing said entities within the droplets to release molecular targets;
labelling said molecular targets with probes, each probe comprising
a capture moiety capable of specific binding or ligation to a molecular target or to an adaptor linked to said molecular target, and
a DNA moiety comprising (i) a region proximal to the capture moiety and comprising the unique molecular identification (UMI) sequence and (ii) a region distal from the capture moiety and comprising an overhang or an overhang producing restriction site,
thereby obtaining the first set of emulsion droplets.

The method may further comprise
(i) encapsulating a plurality of entity identification sequences within emulsion droplets, each droplet containing no more than one entity identification sequence, with an amplification reaction mixture, and
   amplifying the entity identification sequences within droplets
   thereby obtaining the second set of emulsion droplets, or
(ii) encapsulating a plurality of entity identification sequences within emulsion droplets in the presence of UEI-calibrators, wherein at least some droplets comprise one or several entity identification sequences and one or several UEI-calibrators, and wherein said UEI-calibrators are DNA sequences, preferably double stranded DNA sequences, comprising a unique calibrator barcode which is different for each UEI-calibrator and for each droplet, and one or two overhang producing restriction sites; and
amplifying entity identification sequences and/or UEI-calibrators within droplets;
thereby obtaining the second set of emulsion droplets.

Preferably, after fusion of droplets of the first and second sets, (i) UEI calibrator barcodes and UEI barcodes and (ii) UEI barcodes and labelled molecular targets are assembled through restriction enzyme digestion and ligation of compatible overhangs of (i) UEI calibrators and entity identification sequences and of (ii) entity identification sequences and labelled molecular targets.

Alternatively, after fusion of droplets of the first and second sets, (i) UEI calibrator barcodes, UEI barcodes and labelled molecular targets may be assembled through restriction enzyme digestion and ligation of compatible overhangs of (i) UEI calibrators and labelled molecular targets and (ii) UEI calibrators and entity identification sequences, or of i) UEI calibrators and entity identification sequences and (ii) entity identification sequences and labelled molecular targets.

Alternatively, entity identification sequences and UEI-calibrators may be assembled through their compatible overhangs before amplification and, after fusion of droplets of the first and second sets, the amplified fragment comprising UEI calibrator and UEI barcodes is ligated to labelled molecular targets through compatible overhangs of i) UEI calibrators and labelled molecular targets or (ii) entity identification sequences and labelled molecular targets.

Preferably, at least some of molecular targets are nucleic acids and at least some probes comprise
a capture moiety which is a single stranded DNA region which drives the specific recognition of a nucleic acid molecular target through conventional Watson-Crick base-pairing interactions and
a DNA moiety comprising a 3' single stranded region comprising the unique molecular identification (UMI) sequence and a 5'double-stranded region comprising the overhang or overhang producing restriction site.

Preferably, said nucleic acid molecular targets are labelled using said probes as priming sites for a DNA polymerase synthetizing complementary strands of molecular targets.

In particular embodiments, at least some of molecular targets are RNA molecules and the DNA polymerase is a reverse transcriptase.

In some embodiments, at least some probes comprise a capture moiety which is
(i) a binding moiety that specifically binds to a molecular target and is directly bound to the DNA moiety,
(ii) a chimeric protein comprising a first domain that specifically binds to a molecular target and a second domain that binds to the DNA moiety, or
(iii) a binding moiety that binds specifically to a molecular target and a protein bridge, said protein bridge comprising a first domain that binds to the binding moiety and a second domain that binds to the DNA moiety.

Preferably, (i) the binding moiety or the first domain of the chimeric protein is selected from the group consisting of an antibody, a ligand of a ligand/anti-ligand couple, a peptide aptamer, a nucleic acid aptamer, a protein tag, or a chemical probe (e.g. suicide substrate) reacting specifically with a molecular target or a class of molecular targets, preferably is an antibody, (ii) the first domain of the protein bridge is an immunoglobulin-binding bacterial protein, preferably is domains A to E of protein A, and/or (iii) the second domain of the protein bridge or the chimeric protein is selected from the group consisting of SNAP-tag, CLIP-tag or Halo-Tag, preferably is a SNAP-tag.

In some preferred embodiments, at least some probes comprise a capture moiety comprising an antibody moiety specific to a molecular target and a protein bridge, said protein bridge comprising a first domain that binds to a Fc region of the antibody moiety and a second domain that binds to the DNA moiety, preferably a SNAP-tag.

The present invention also relates to a method of quantifying one or several molecular targets from a plurality of entities with single-entity resolution, said method comprising
labelling said molecular targets according to the method of the invention;
capturing said labelled molecular targets,
amplifying sequences comprising UMI and UEI barcodes, and optionally UEI-calibrator barcodes
sequencing amplified sequences.

The sequencing of UMI and UEI barcodes, and optionally UEI-calibrator barcodes, allows to unambiguously assign each molecular target to a droplet/entity and thus to quantify the content of molecular targets in each droplet/entity.

Preferably, the entity is a cell, or a particle or an oil-in-water emulsion droplet exposing molecular targets on its outer surface. More preferably, the entity is a cell.

When the entity is a particle or an emulsion droplet exposing molecular targets on its outer surface, the method may further comprise
- labelling molecular targets with probes as defined herein, and
- encapsulating entities attached to labelled molecular targets within emulsion droplets, each droplet containing no more than one entity,
thereby obtaining the first set of emulsion droplets.

The present invention further relates to the use of a kit to label a plurality of molecular targets from a plurality of entities according to the method of the invention or to quantify one or several molecular targets from a plurality of entities with single-entity resolution according to the method of the invention, wherein the kit comprises
a microfluidic device comprising
- a first emulsion re-injection module or on-chip droplet generation module;
- a second emulsion re-injection module or on-chip droplet generation module
- a droplet-pairing module, and
- a module coupling droplet fusion to injection,
wherein emulsion re-injection modules and/or on-chip droplet generation modules are in fluid communication and upstream to the droplet-pairing module, the droplet-pairing module is in fluid communication and upstream to the module coupling droplet fusion to injection,
and optionally,
- one or several probes as defined herein; and/or
- one or several entity identification sequences as defined herein; and/or
- one or several UEI-calibrators as defined herein; and/or
- one or several primers suitable to amplify entity identification sequences and/or UEI-calibrators; and/or
- an aqueous phase and/or an oil phase; and/or
- a leaflet providing guidelines to use such a kit.

Alternatively, the invention relates to the use of a kit to label a plurality of molecular targets from a plurality of entities according to the method of the invention or to quantify one or several molecular targets from a plurality of entities with single-entity resolution according to the method of the invention, wherein the kit comprises
- one or several probes as defined herein; and/or
- one or several entity identification sequences as defined herein; and/or
- one or several UEI-calibrators as defined herein; and/or
- one or several primers suitable to amplify entity identification sequences and/or UEI-calibrators; and/or
- an aqueous phase and/or an oil phase; and/or
- a microfluidic device, preferably a microfluidic device of the invention, and optionally
- a leaflet providing guidelines to use such a kit.

### BRIEF DESCRIPTION OF THE DRAWINGS

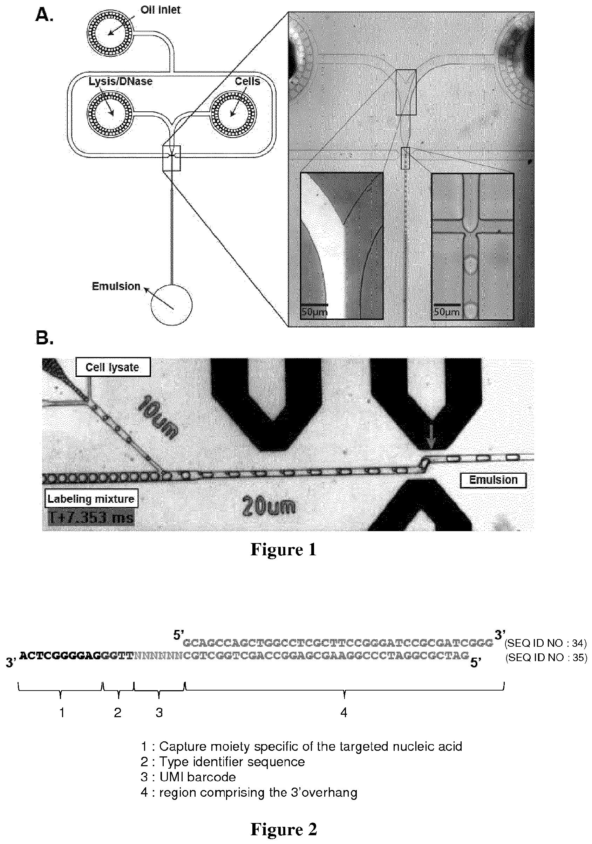

**Figure 1** **:** Droplet-based microfluidics platforms for single cell molecular labeling. A. Single cell individualization and lysis. An aqueous stream containing the cells is combined with a stream of aqueous solution containing a lysis agent and optionally a double strand specific DNase. The emulsion is generated, collected and incubated to allow cell lysis and DNA degradation to occur. **B.** Droplet fusion. Droplets containing cell lysate are reinjected into a droplet fusion microfluidic chip and synchronized with on-chip generated droplets containing labeling mixture (reverse transcription mixture, DNA probes, antibodies...). Pairs of droplets are then fused when passing between a pair of electrodes at the fusion point (arrow).
**Figure 2****:** Exemplary embodiment of synthetic DNA-based probe for targeted labeling of nucleic acids. In this example, the sequence of the probe designed to quantify RNA III of *Staphylococcus aureus.*

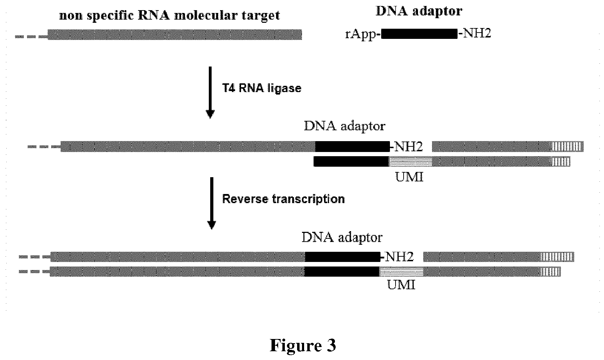

**Figure 3****:** Exemplary embodiment with a DNA probe comprising a capture moiety driving the specific recognition of a DNA adaptor linked to RNA molecular targets. The pre-adenylated adaptor (5'-App oligonucleotide) acts as a substrate for T4 ligase and is thus ligated to RNA molecules. The capture moiety of the probe then specifically hybridizes with the DNA adaptor.

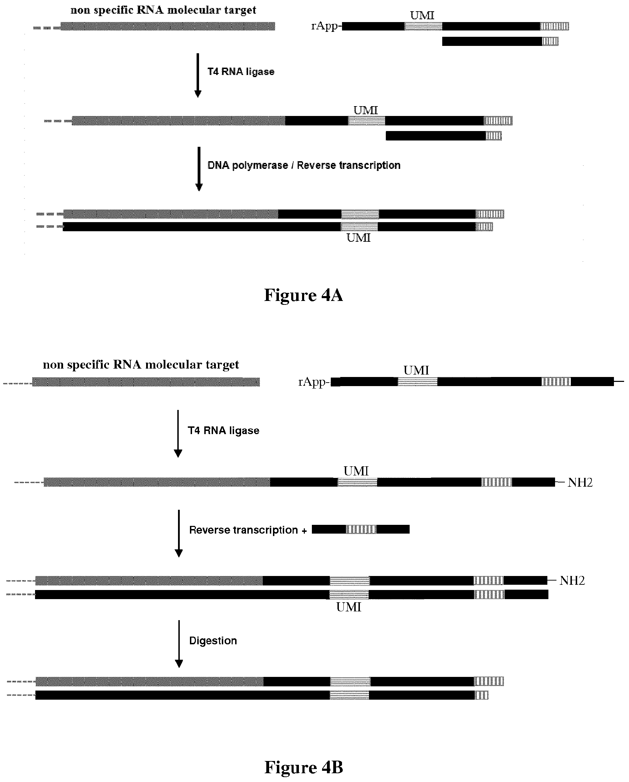

**Figure 4****:** Exemplary embodiments with a DNA probe comprising a capture moiety which is a 5' single stranded DNA region comprising 5',5'-adenyl pyrophosphoryl moiety (App) onto its 5'-end. Such moiety acts as a substrate for T4 ligase and is thus ligated to RNA molecules.

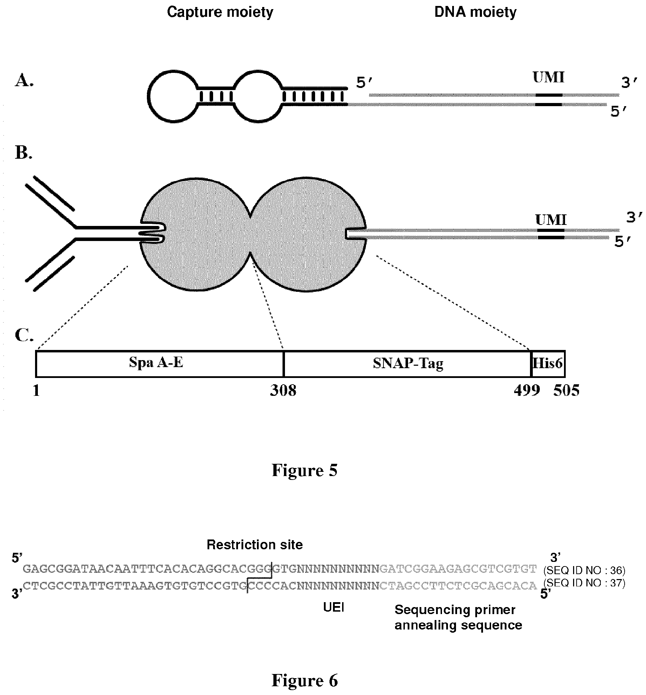

**Figure 5****:** Exemplary embodiment of chimeric probe. **A.** Aptamer-based probe. This probe is composed of a RNA or DNA aptamer specific of the target molecule and fused to a synthetic DNA labeling moiety. **B.** Chimeric probe comprising a capture moiety comprising a protein bridge (SNAP-Tag and protein A) and an antibody specific of the molecular target. **C.** Schematic organization of the capture moiety.
**Figure 6****:** Exemplary embodiment of entity identification sequence. The entity identification sequence comprises a constant region, a unique restriction site , a UEI barcode and a sequencing primer annealing sequence.

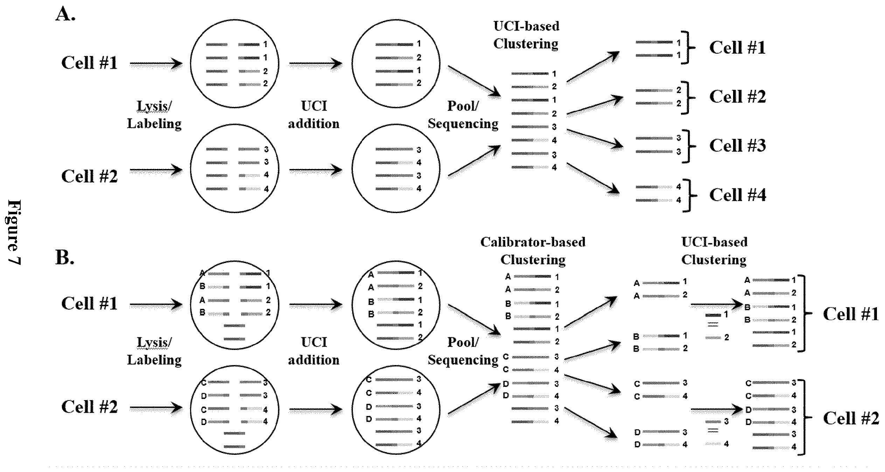

**Figure 7****:** Multiple encapsulations of entity identification sequences (UEI): consequences and correction using UEI-calibrators. Two scenarios are exemplified using the simplified case of 2 UEI and 2 calibrators encapsulated into the same compartment. **A.** Multiple encapsulations of UEI without calibrators. Two cells are encapsulated into distinct droplets together with 2 different UEI. Upon UEI addition, target molecules from the same cell are associated with 2 different UEI. This will lead to the misassignment of target molecules to clusters representing only a sub-fraction of the cell and the integrity of the single-cell information will be compromised. **B.** Multiple encapsulations of UEI in the presence of calibrators. Two cells are encapsulated into distinct droplets together with 2 different UEI and 2 different calibrators. Upon UEI addition, both target molecules from the same cell and Calibrators are associated with 2 different UEI. At the end of the process, a first analysis defines the clusters of UEI associated with the same set of Calibrators. Target molecules labeled by UEI belonging to the same cluster are then clustered together and the integrity of the single-cell information is preserved.

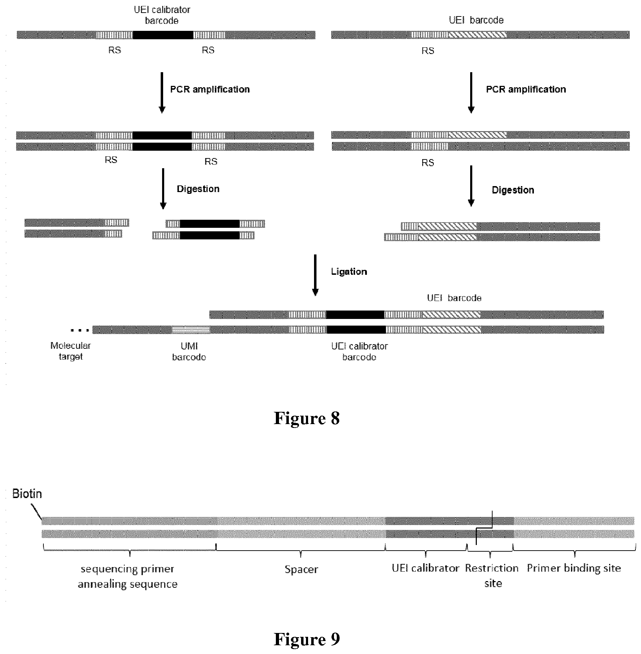

**Figure 8****:** Exemplary embodiment with UEI-calibrators comprise two overhang producing restriction sites (RS), a first restriction site generating an overhang compatible with overhangs of digested entity identification sequences comprising UEI barcodes and a second restriction site generating an overhang compatible with overhangs of labelled molecular targets. In this embodiment, the digestion/ligation step used for UEI addition to labelled molecular targets, leads to the formation of tripartite molecules comprising a labelled molecular target, a UEI barcode and a UEI-calibrator barcode.
**Figure 9****:** Exemplary embodiment of UEI-Calibrator. The molecule is shown as a PCR-amplification product (double-stranded DNA). The UEI-Calibrator comprises a sequencing primer annealing sequence, a spacer, a UEI-calibrator barcode (UEI calibrator), a unique restriction site and a constant region comprising a primer binding site allowing amplification of UEI calibrator.

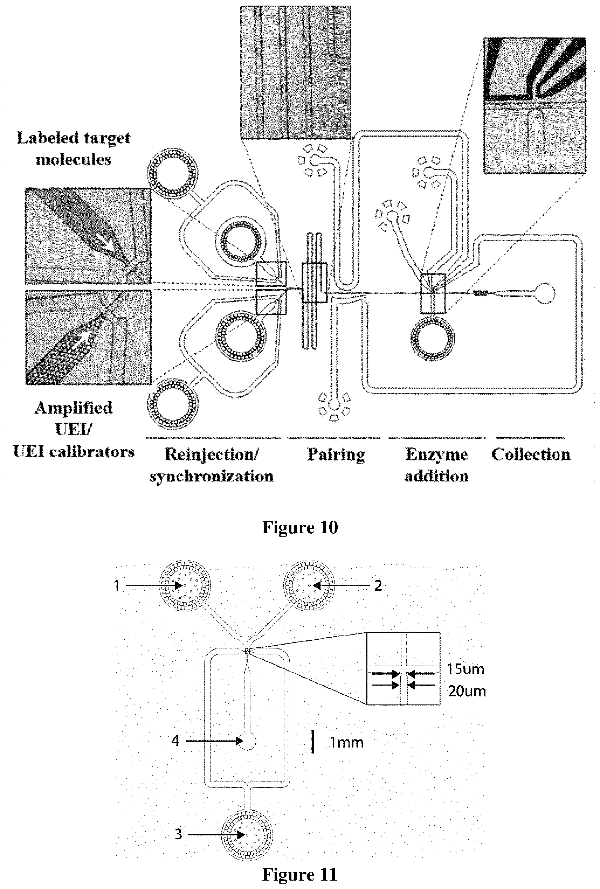

**Figure 10****:** Exemplary embodiment of a microfluidic device suitable to implement the method of the invention. The first set and second set of emulsion droplets are reinjected into the device (left micrographs), spaced with oil streams and synchronized at the junction of both reinjection channels. Synchronized droplets are allowed to circulate into a long channel having a width larger than the small droplet but narrower than the large droplet. As a consequence, the small droplet catches the large one and pairs of droplets (middle micrograph) are formed at the exit of the channel. Finally pairs of droplets are at the injection point where the enzyme solution is infused and the presence of electrodes trigger both droplets fusion and enzyme delivery (right micrograph).
**Figure 11****:** Co-flow droplet generator. The key dimensions of the microfluidic device are indicated. The depth of the channels was 10 µm.

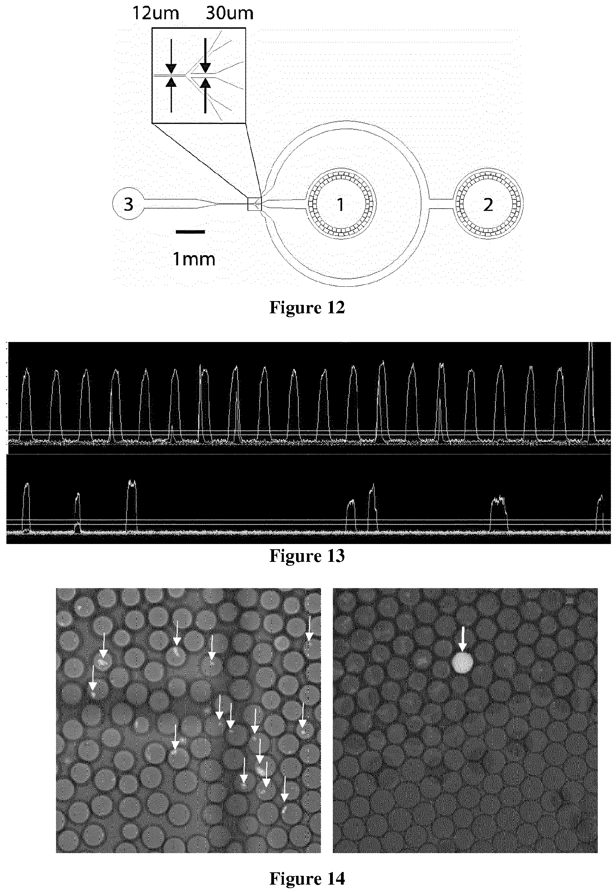

**Figure 12****:** Droplet fluorescence analyzer. The key dimensions of the microfluidic device are indicated. The depth was 15 µm. Fluorescence measurement point is indicated by the open arrow.
**Figure 13****:** Fluorescence profile of orange-labelled droplets containing intact or lysed bacteria. Top panel: fluorescence profile of droplet containing intact bacteria. Each orange peak corresponds to a droplet. Green spikes observed into each orange peak corresponds to a fluorescent particle, therefore an intact bacterium. Bottom panel: fluorescence profile of droplet containing lyzed bacteria. Each orange peak corresponds to a droplet. Moreover, the presence of homogeneous green having the same width as the orange peak (e.g. the second peak from the left) indicates that the fluorescently-labelled nucleic acids have been released into the droplet, so that the bacterium has been lyzed.
**Figure 14****.** Bright-field and green fluorescence imaging of the water-in-oil droplets. The bacteria (green particles, arrows) encapsulated in the presence of CutSmart® buffer but in the absence of B-PER™ are shown on the left side whereas the bacteria encapsulated in the presence of B-PER™ are shown on the right side. Note that the lower number of fluorescent droplets after bacteria lysis is due to the more than a thousand-fold dilution of the fluorescence in the droplets following bacteria lysis, making these droplets difficult to distinguish from the background.

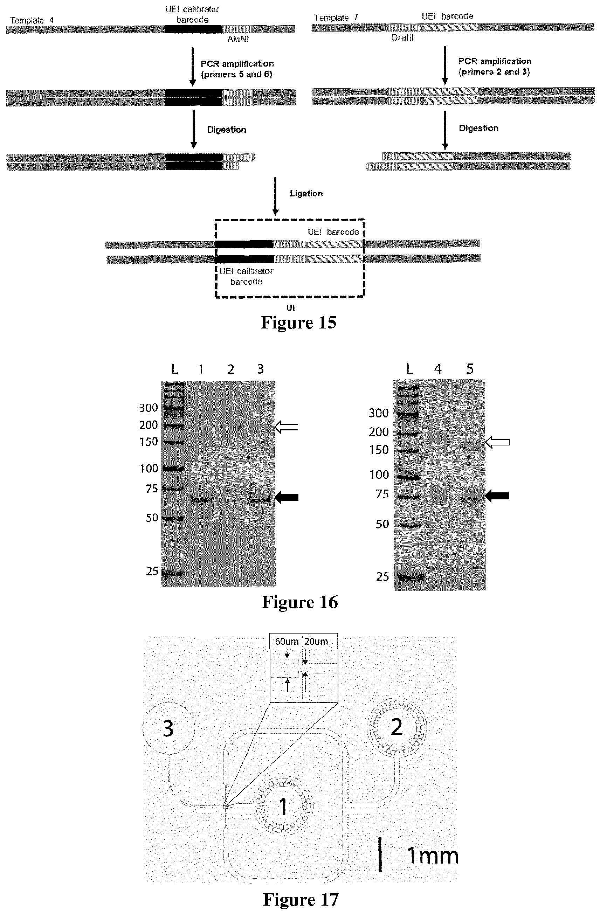

**Figure 15****:** Main steps in Unique Identifiers (UI) preparation.
**Figure 16****:** Analysis of PCR amplification and (co)-amplification of UEI-Calibrators and UEIs. Left panel: analysis of the PCR amplification of the UEIs (lane 1), the UEI-Calibrators (lane 2) or of both together (lane 3). Right panel: analysis of the PCR co-amplification of UEI-Calibrators and UEIs in bulk (lane 4) and in droplets (lane 5). The position of the expected size for UEI-Calibrators and of the UEIs amplification products size are labelled respectively by an open and a closed arrow. The lane L corresponds to the low range ladder (SM1203, Fermentas). Both gels were 8% native polyacrylamide-TBE 1x gels.
**Figure 17****:** Droplet generator. The key dimensions of the microfluidic device are indicated and the channels were 40 µm deep.

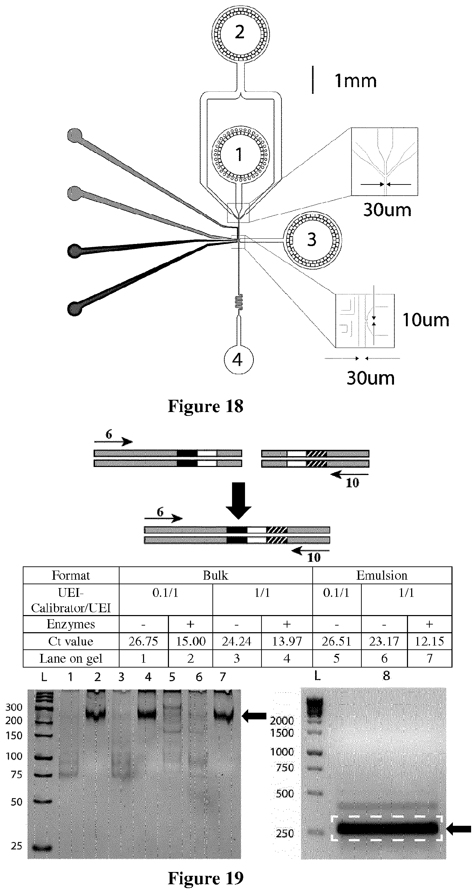

**Figure 18****.** Droplet picoinjector. Key dimensions are indicated and the channels were 40 µm deep. Ground and positive electrodes are shown in light and dark gray respectively.
**Figure 19****.** Analysis of DNA labelling efficiency. Top panel: The proper formation of an UI following the recombination of a UEI-Calibrator-bearing DNA (black square) with an UEI-bearing DNA (dashed squares) at the level of a restriction site (open square) brings annealing sites of primer 6 and 10 on the same DNA allowing for qPCR to take place. Middle panel: The Ct values are given for experiments performed both in bulk and in emulsion at both UEI-Calibrator/UEI ratios. Moreover, labelling reactions were performed in the presence (+) or in the absence (-) of restriction/ligation enzymes. Finally, for each reaction the number of the corresponding lane on analysis gel is given. Bottom left panel: analysis of qPCR products on 8% native polyacrylamide-TBE 1x. The lane L corresponds to the low range ladder (SM1203, Fermentas). The position of the product of expected size is indicated by the black arrow. Bottom right panel: gel purification of indexed library. The library of indexed UIs was purified on a 1% native agarose gel in TBE. The lane L corresponds to the 1 kb ladder (SM1163, Fermentas). The position of the product of expected size is indicated by the black arrow. The white dotted line box shows the band recovered for sequencing.

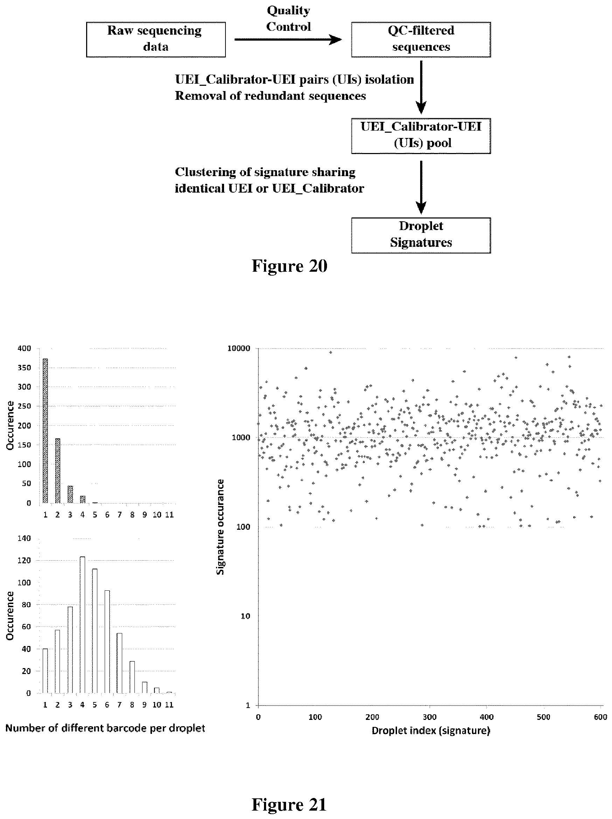

**Figure 20****.** Bioinformatics algorithm used to analyze sequencing data.
**Figure 21****:** Barcodes distribution and signature occurrence in droplets. Left panel: Distribution of UEI-Calibrators and UEIs in droplets. The distribution of the number of different barcode sequences per droplet is shown for the UEI-Calibrator (gray dashed bars) as well as for UEIs (open bars). Right panel: upon UIs clustering in Signatures, the occurrence of signature in the sequence pool was determined.

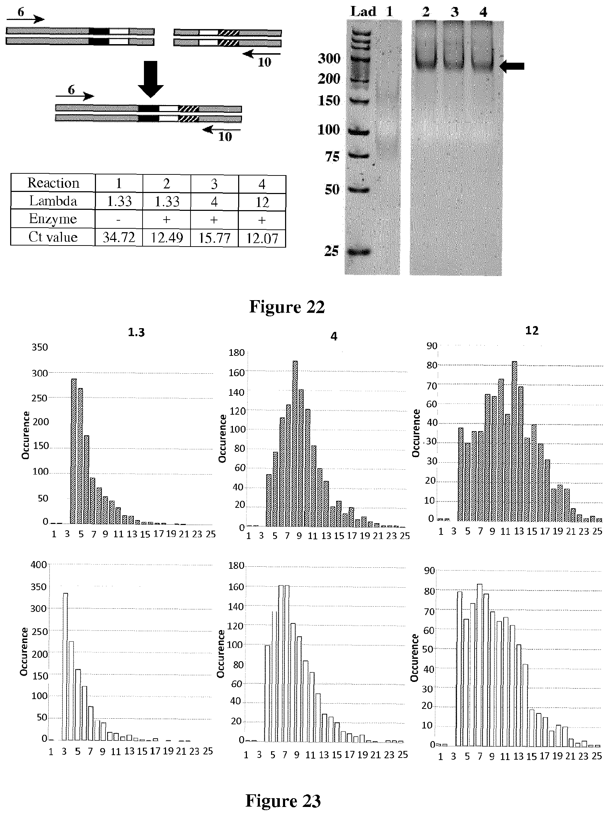

**Figure 22****.** Analysis of UI formation at various barcode lambda values. Left panel: qPCR analysis of UI formation. The proper formation of an UI upon the recombination of a UEI-Calibrator-bearing DNA (black square) with an UEI-bearing DNA (dashed squares) at the level of a restriction site (open square) brings annealing sites of primer 6 and 10 on the same DNA allowing for qPCR to take place. Ct values are given for the different lambda (number of different UEI-Calibrators and UEIs per droplet) tested. Right panel: analysis of qPCR products on 8% native polyacrylamide-TBE 1x. The lane L corresponds to the low range ladder (SM1203, Fermentas). The position of the product of expected size is indicated by the black arrow.
**Figure 23****.** Distribution profile of UEI-Calibrators and UEIs in the droplets. Occurrence at values 1 and 2 were intentionally removed as they contained significant sequencing noise.

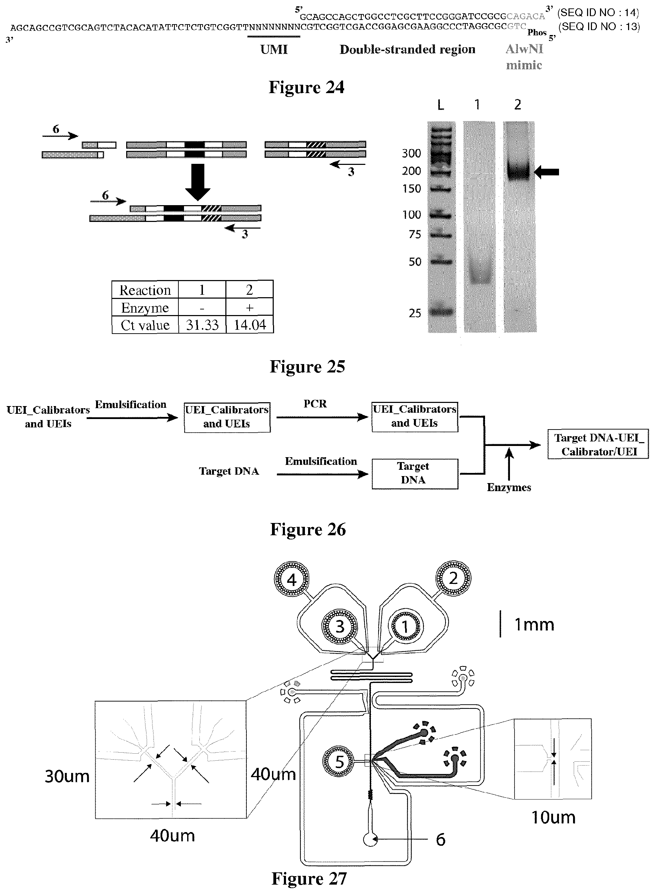

**Figure 24****:** Scheme representation of the target DNA used.
**Figure 25****:** Analysis of UI formation and grafting to a target DNA. Left panel: qPCR analysis of UI formation and grafting to a target DNA. The proper formation of an UI upon the recombination of a UEI-Calibrator-bearing DNA (black square) with an UEI-bearing DNA (dashed squares) at the level of a restriction site (open square) as well as the attachment of the UI at an extremity of the target DNA (polka-dotted squares) mimicking a digested product brings annealing sites of primer 6 and 3 on the same DNA allowing for qPCR to take place. Ct values are given in the table. Right panel: analysis of qPCR products on 8% native polyacrylamide-TBE 1x. The lane L corresponds to the low range ladder (SM1203, Fermentas). The position of the product of expected size is indicated by the black arrow.
**Figure 26****.** Schematic workflow of UI preparation and target DNA labelling in droplets.
**Figure 27****.** Barcoding chip. Key dimensions are indicated and the channels were 40 µm deep. Ground and positive electrodes are shown in light and dark gray respectively.

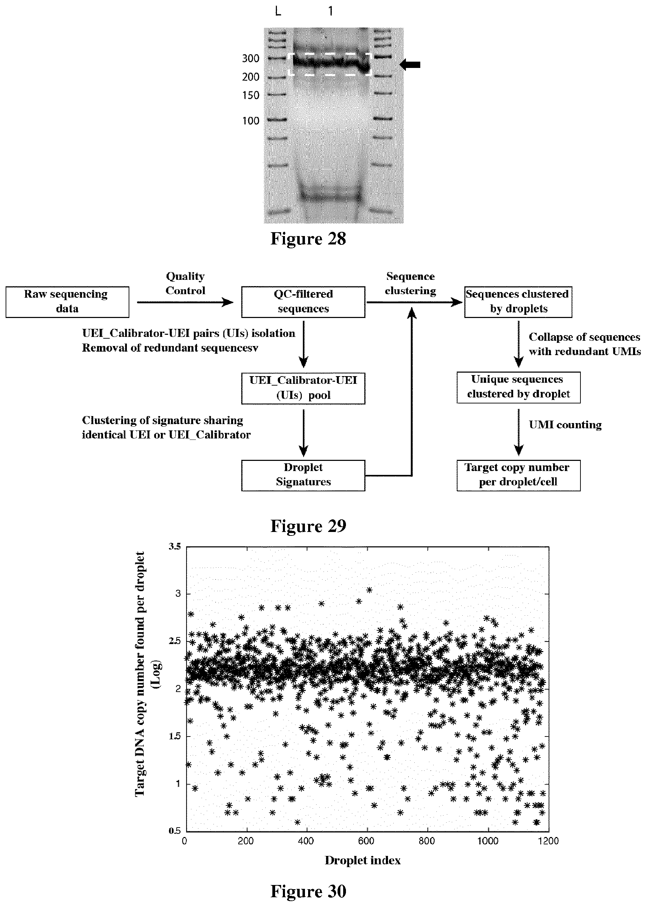

**Figure 28****.** Purification gel of the Target DNA-UI indexed library. The indexed library was purified on a 1% native agarose gel in TBE. The lane L corresponds to the 1 kb ladder (SM1163, Fermentas). The position of the product of expected size is indicated by the black arrow. The white dotted line box shows the band recovered for sequencing.
**Figure 29****.** Bioinformatics algorithm used to deconvolute sequencing data and determine the copy number of target molecules per droplet/cell.
**Figure 30****.** Droplet-to-droplet reliability. The number of DNA molecules identified per droplet is represented for each droplet. Note that droplets with low DNA content (between 1.8 and 0.5) were likely to be noise but were kept in the analysis to not bias it.

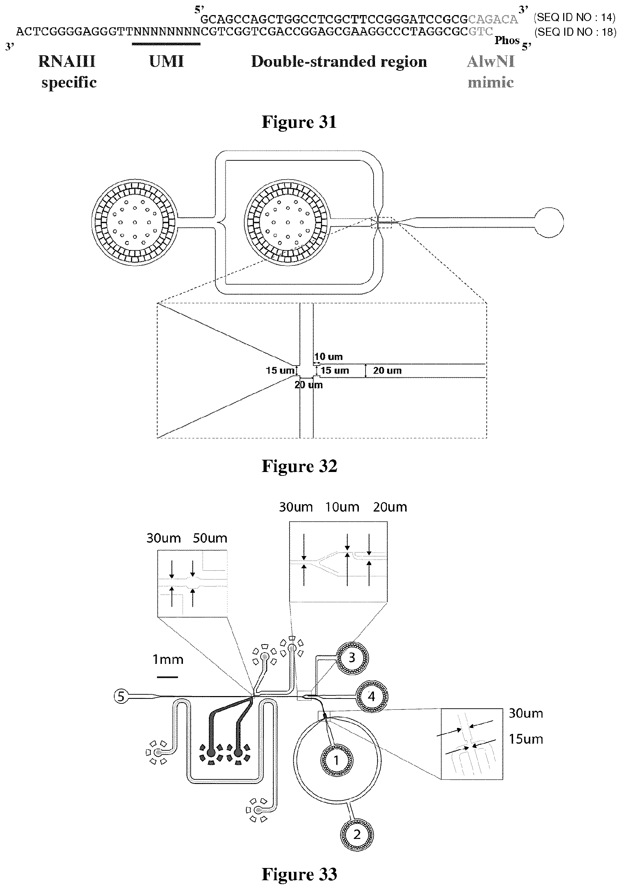

**Figure 31****.** Scheme representation of the primer used to reverse transcribed the RNA-III.
**Figure 32****.** 2 pL droplet generator. The key dimensions of the devices are indicated. The channels were 10 µm deep.
**Figure 33****.** Microfluidic droplet fuser. Key dimensions are indicated and the channels were 15 µm deep. Ground and positive electrodes are shown in light and dark gray respectively.

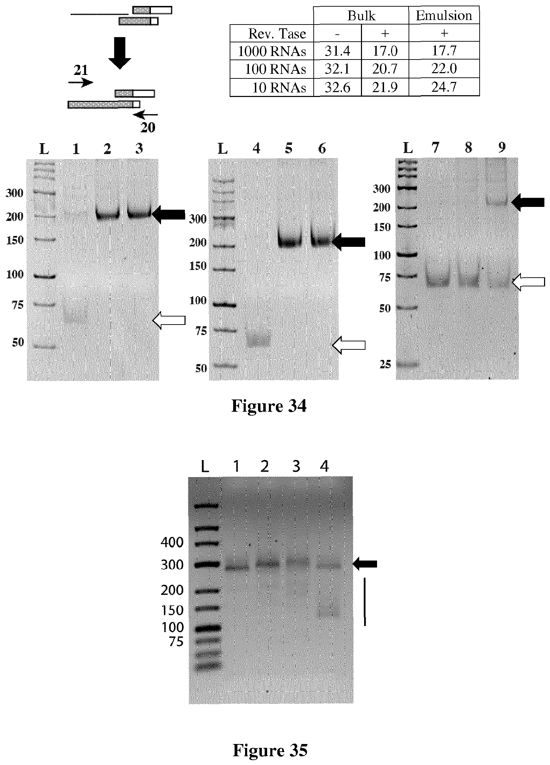

**Figure 34****.** Analysis of reverse transcription products. Top: upon reverse transcription, the RT primer is extended and contain annealing site of primer 21. The generated cDNA contains both primer-binding sites (20 and 21) and can be detected by qPCR. Ct values are given in the table. Bottom: analysis of qPCR products on 8% native polyacrylamide-TBE 1x. Gels on the left, the middle and the right correspond respectively to the experiment started with 1000, 100 and 10 RNA per droplet. Lanes 1, 4 and 7 are the negative controls, lanes 2, 5 and 8 correspond to the experiment performed in bulk and lanes 3, 6 and 9 correspond to the reaction performed in droplets. The lanes L correspond to the low range ladder (SM1203, Fermentas). The position of the product of expected size is indicated by the black arrows whereas small parasitic side products are shown by the open arrows.
**Figure 35****.** Analysis of PCR products on 1% agarose gel-TBE. UI were initially prepared with random region-free (lane1), N2x5 (lane 2), N4443 (lane 3) and N15 (lane 4) templates. The position of the product of expected size is indicated by the black arrow. The black vertical bar shows the short parasitic side products.

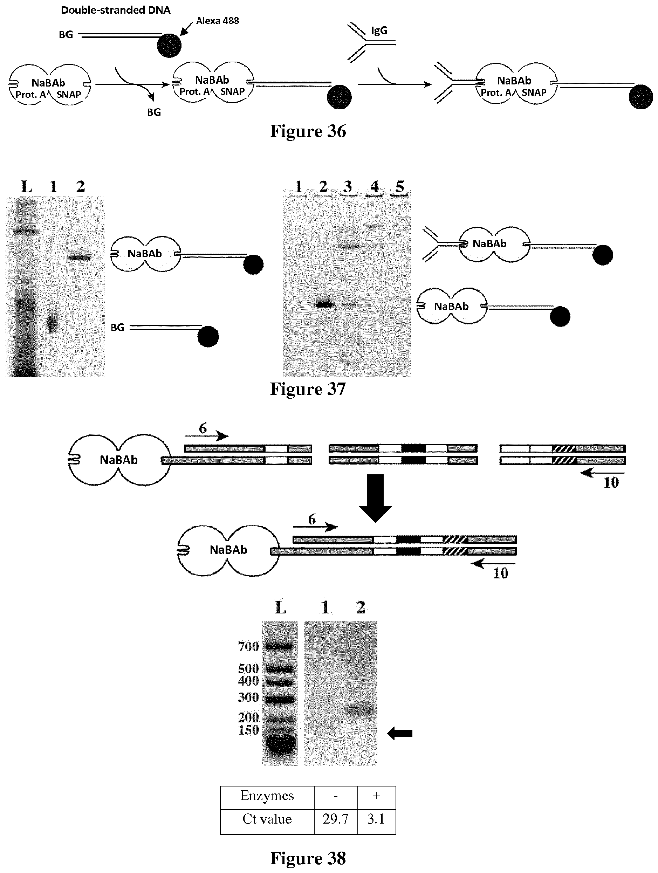

**Figure 36****.** Workflow of the preparation of NaBAb-DNA/IgG complex.
**Figure 37****.** Preparation of NaBAb-DNA/IgG complex. Left panel: Incubation of BG-labelled fluorescent DNA with (lane 2) and without (lane 1) NaBAb protein. L indicates a molecular weight ladder. Right panel: NaBAb protein was incubated alone (lane 1), with BG-labelled fluorescent DNA (lane 2) as well as an increasing concentration of IgG (62.5 µg/mL on lane 3, 112 µg/mL on lane 4 and 225 µg/mL on lane 5). Upon incubation, the reaction products were loaded on a native polyacrylamide gel and the position of DNA molecule was revealed by imaging gel fluorescence (emitted by the Alexa488 conjugated with the DNA) without further staining.
**Figure 38****.** UI attachment to NaBAb-DNA complex. Left panel: qPCR analysis of UI grafting to DNA-labelled protein. The proper formation of an UI upon the recombination of a UEI-Calibrator-bearing DNA (black square) with an UEI-bearing DNA (dashed squares) at the level of a restriction site (open square) as well as the attachment of the UI, via a compatible restriction site at the extremity of a DNA covalently attached to a protein brings annealing sites of primer 6 and 10 on the same DNA allowing for qPCR to take place. Ct values are given in the table. Right panel: analysis of qPCR products on 1% agarose gel-TBE 1x. The lane L corresponds to the low range ladder (SM1203, Fermentas). The position of the product of expected size is indicated by the black arrow.

### DETAILED DESCRIPTION OF THE INVENTION

The inventors conceived a new method of labelling any target molecules from a plurality of entities in high throughput regimes, i.e. allowing the analysis of several thousands of entities per run, while preserving the integrity of the single-entity information. This method is based on a tandem molecular barcoding in which all molecular targets (nucleic acids, proteins,...) are labelled (i) with a first unique barcode (unique molecular identification barcode or UMI barcode) which is different for each molecular target from an entity, and (ii) with a tag sequence coding the entity from which the molecular target originates, i.e. unique entity identification barcode or UEI barcode which is different for each entity but identical for all molecular targets originating from the same entity. Once this tandem barcoding is performed, the absolute quantification of all molecular targets with a single-entity resolution may be carried out in a single run of next-generation sequencing making this method highly sensitive and cost-effective.

In a first aspect, the present invention relates to a method of labelling a plurality of molecular targets from a plurality of entities, said method comprising
providing a first set of emulsion droplets comprising droplets containing labelled molecular targets, wherein each of these droplets contains a plurality of molecular targets originating from no more than one entity and wherein, in each of these droplets, each molecular target is labelled with a molecular identification DNA sequence comprising (i) a unique molecular identification (UMI) barcode which is different for each molecular target and (ii) an overhang or an overhang producing restriction site;
providing a second set of emulsion droplets comprising droplets containing entity identification sequences, wherein each of these droplets contains at least one entity identification sequence which is a DNA sequence, preferably a double-stranded DNA sequence, comprising a unique entity identification (UEI) barcode which is different for each droplet of the second set, and an overhang producing restriction site;
fusing droplets of the first set with droplets of the second set wherein a droplet of the first set is fused with no more than one droplet of the second set; and
ligating UEI barcodes to labelled molecular targets, after restriction enzyme digestion.

The method of the invention may be used to label molecular targets from any type of entities.

As used herein, the term "entity" refers to any entity comprising or exposing on its surface, molecular targets as defined below. In particular, this term refers to a cell, or refers to a particle or an emulsion droplet, preferably an oil-in-water emulsion droplet, exposing molecular targets on its outer surface.

As used herein, the term "cell" refers to a prokaryotic cell or a eukaryotic cell such as animal, plant, fungal or algae cell. The population of cells to be processed may be homogenous, i.e. comprising only one cellular type, or may be heterogeneous, i.e. comprising several cellular types. In some embodiments, the population of cells is obtained from a tissue sample, preferably an animal tissue sample, more preferably from a pathological sample such as a tumor sample. In some other embodiments, the population of cells is a population of bacterial, fungal or algae cells, preferably of bacterial or fungal cells. This population may comprise bacteria, fungi or algae of the same species or bacteria, fungi or algae of different species.

The terms "particle" and "bead" are used herein interchangeably and refer to any solid support, preferably a spherical solid support, of 50 nm to 10 µm in size which is suitable to expose one or several molecular targets on its outer surface. In particular, these terms may refer to polymer beads (e.g. polyacrylamide, agarose, polystyrene), latex beads, magnetic beads or hydrogel beads. Methods for covalent or non-covalent binding of molecular targets such as nucleic acids or proteins, to beads are well known by the skilled person and various techniques are commercially available. In particular, this binding may be carried out through reactive groups on the surface of the particle. For example, nucleic acids may be attached to the surface by carbodiimide-mediated end-attachment of 5'-phosphate and 5'-NH2 modified nucleic acids to respectively amino and carboxyl beads. Proteins may also be covalently or non-covalently attached to beads via any suitable method such as using sulphate, amidine, carboxyl, carboxyl/sulphate or chloromethyl modified beads.

The term "entity" may also refer to an emulsion droplet, preferably an oil-in-water emulsion droplet, exposing molecular targets on its outer surface. Molecular targets may be covalently or non-covalently attached to the droplet through reactive groups exposed on the surface of the droplets such as nitrilotriacetate which can specifically interact with his-tagged proteins, or through any other functional moiety which is able to covalently or non-covalently interact with a molecular target of interest. The skilled person may use any known method to produce such emulsion droplets exposing molecular targets on its outer surface, in particular methods described in international patent application WO 2017/174610.

The method of the invention allows labelling molecular targets from a high number of entities in a single run. Thus, as used herein, the term "plurality of entities" refers to at least 1,000 entities, preferably at least 5,000 entities, more preferably at least 10,000 entities, and even more preferably at least 50,000 entities.

As used herein, the term "target molecule" or "molecular target" refers to any kind of molecules, and in particular any kind of molecules which may be possibly present in a cell. The molecular target can be a biomolecule, i.e. a molecule that is naturally present in living organisms, or a chemical compound that is not naturally found in living organism such as pharmaceutical drugs, toxicants, heavy metals, pollutants, etc... Preferably, the molecular target is a biomolecule. Examples of biomolecules include, but are not limited to, nucleic acids, e.g. DNA or RNA molecules, proteins such as antibodies, enzymes or growth factors, lipids such as fatty acids, glycolipids, sterols or glycerolipids, vitamins, hormones, neurotransmitters, and carbohydrates, e.g., mono-, oligo- and polysaccharides. The terms "polypeptide", "peptide" and "protein" are used interchangeably to refer to a polymer of amino acid residues, and are not limited to a minimum length. The protein may comprise any post-translational modification such as phosphorylation, acetylation, amidation, methylation, glycosylation or lipidation. As used herein, the term "nucleic acid" or "polynucleotide" refers to a polymeric form of nucleotides of any length, either ribonucleotides or deoxyribonucleotides.

One of the main advantage of the method of the invention, is the possibility to label, in a single run, different types of molecular targets such as proteins and nucleic acids. Thus, the term "plurality of molecular targets" may refer to different copies of the same molecule, e.g. different copies of the same mRNA or of the same protein, or may refer to different copies of different molecules, e.g. different copies of a mRNA and different copies of a protein.

In an embodiment, molecular targets are different copies of the same molecule. Preferably the molecule is biomolecule, more preferably a nucleic acid or a protein, even more preferably a RNA molecule or a protein. In another embodiment, molecular targets are different copies of different molecules. Preferably said molecules are biomolecules, more preferably are nucleic acids and/or proteins, even more preferably RNA molecules and/or proteins. In a particular embodiment, molecular targets are different copies of at least two different nucleic acids, preferably RNA. In another particular embodiment, molecular targets are different copies of at least two different proteins. In a further embodiment, molecular targets are different copies of one or several nucleic acid, preferably RNA, and different copies of one or several proteins.

In preferred embodiments, the method of the invention is implemented using one or several microfluidic systems, i.e. at least one step of the method is implemented using a microfluidic system. In some embodiments, the method is implemented using several microfluidic systems, for example a microfluidic system to generate the first set of emulsion droplets, a microfluidic system to generate the second set of emulsion droplets and a microfluidic system to fuse the two sets, to incorporate UEI barcodes and optionally to conduct some subsequent steps. In some other embodiments, the method is implemented using a microfluidic system wherein the first set of emulsion droplets and/or the second set of emulsion droplets are generated and wherein droplets of the two sets are fused.

As used herein, the terms "emulsion droplet", "droplet" and "microfluidic droplet" are used interchangeably and may refer to a water-in-oil emulsion droplet (also named w/o droplet) , i.e. an isolated portion of an aqueous phase that is completely surrounded by an oil phase, an oil-in-water emulsion droplet (also named o/w droplet), i.e. an isolated portion of an oil phase that is completely surrounded by an aqueous phase, a water-in-oil-in-water emulsion droplet (also named w/o/w droplet) consisting of an aqueous droplet inside an oil droplet, i.e. an aqueous core and an oil shell, surrounded by an aqueous carrier fluid, or an oil-in-water-in-oil emulsion droplet (also named o/w/o droplet) consisting of an oil droplet inside an aqueous droplet, i.e. an oil core and an aqueous shell, surrounded by an oil carrier fluid. Preferably, this term refers to a w/o emulsion droplet.

A droplet may be spherical or of other shapes depending on the external environment. Typically, the droplet has a volume of less than 100 nL, preferably of less than 10 nL, and more preferably of less than 1 nL. For instance, a droplet may have a volume ranging from 2 pL to 1 nL, preferably from 2 to 500 pL, more preferably from 2 to 100 pL. Preferably, the droplets have a homogenous distribution of diameters, i.e., the droplets may have a distribution of diameters such that no more than about 10%, about 5%, about 3%, about 1%, about 0.03%, or about 0.01% of the droplets have an average diameter greater than about 10%, about 5%, about 3%, about 1%, about 0.03%, or about 0.01% of the average diameter of the droplets. Preferably, the emulsion is a monodispersed emulsion, i.e. an emulsion comprising droplets of the same volume. Techniques for producing such a homogenous distribution of diameters are well-known by the skilled person (see for example WO 2004/091763).

The aqueous phase is typically water or an aqueous buffer solution, such as but not limited to Tris-HCl buffer, Tris-acetate buffer, phosphate buffer saline (PBS) or acetate buffer. Preferably, the aqueous phase is an aqueous buffer solution. Optionally, the aqueous phase may comprise bovine serum albumin or additive such as Pluronic. In preferred embodiments, the aqueous phase is chosen in order to be compatible with enzymatic reactions performed during the process of the invention, such as enzymatic digestion, amplification, ligation, etc... An example of such aqueous phase includes, but is not limited to, CutSmart restriction enzyme buffer (New England Biolabs).

The oil phase used to generate the emulsion droplets may be selected from the group consisting of fluorinated oil such as FC40 oil (3M®), FC43 (3M®), FC77 oil (3M®), FC72 (3M®), FC84 (3M®), FC70 (3M®), Novec-7500 (3M®), Novec-7100 (3M®), perfluorohexane, perfluorooctane, perfluorodecane, Galden-HT135 oil (Solvay Solexis), Galden-HT170 oil (Solvay Solexis), Galden-HT110 oil (Solvay Solexis), Galden-HT90 oil (Solvay Solexis), Galden-HT70 oil (Solvay Solexis), Galden PFPE liquids, Galden® SV Fluids or H-Galden® ZV Fluids; and hydrocarbon oils such as Mineral oils, Light mineral oil, Adepsine oil, Albolene, Cable oil, Baby Oil, Drakeol, Electrical Insulating Oil, Heat-treating oil, Hydraulic oil, Lignite oil, Liquid paraffin, Mineral Seal Oil, Paraffin oil, Petroleum, Technical oil, White oil, Silicone oils or Vegetable oils. Preferably, the oil phase is fluorinated oil such as Novec-7500, FC40 oil, Galden-HT135 oil or FC77 oil, more preferably is Novec-7500. The skilled person may easily select suitable phase oil to implement the methods of the invention.

The emulsion droplets comprise one or several surfactants. Said surfactant(s) can aid in controlling or optimizing droplet size, flow and uniformity and stabilizing aqueous emulsions. Suitable surfactants for preparing the emulsion droplets used in the present invention are typically non-ionic and contain at least one hydrophilic head and one or several lipophilic tails, preferably one (diblock surfactant) or two (triblock surfactant) lipophilic tails. Said hydrophilic head(s) and the tail(s) may be directly linked, or linked via a spacer moiety. Examples of suitable surfactants include, but are not limited to, sorbitan-based carboxylic acid esters such as sorbitan monolaurate (Span 20), sorbitan monopalmitate (Span 40), sorbitan monostearate (Span 60) and sorbitan monooleate (Span 80); block copolymers of polyethylene glycol and polypropylene glycol such as the triblock copolymer EA-surfactant (RainDance Technologies), DMP (dimorpholino phosphate)-surfactant (Baret, Kleinschmidt, et al., 2009) and Jeffamine-surfactant; polymeric silicon-based surfactants such as Abil EM 90; triton X-100; and fluorinated surfactants such as PFPE-PEG and perfluorinated polyethers (e.g., Krytox-PEG, DuPont Krytox 157 FSL, FSM, and/or ESH). In the context of the invention, preferred surfactants are fluorinated surfactants, i.e. fluorosurfactants.

In some particular embodiments, the emulsion droplets comprise one or several functionalized surfactants at their interface. As used herein, a "functionalized surfactant" refers to a surfactant which bears at least one functional moiety either on one of its hydrophilic head(s) or lipophilic tail(s), preferably on a hydrophilic head. As used herein, a "functional moiety" is virtually any chemical or biological entity which provides the surfactant with a function of interest. For instance, the functional moiety can enable to create a covalent or non covalent interaction between the surfactant and a molecular target of interest. Thanks to the use of such functionalized surfactants, molecular targets may be exposed on the surface of emulsion droplets. The interface of these droplets may comprise only functionalized surfactant(s) or a mix of functionalized and non-functionalized surfactants. The ratio between functionalized and non-functionalized surfactants may vary and can be easily adapted by the skilled person. For example, functionalized surfactant may represent from 1 to 100% (w/w) of total surfactants, preferably from 2 to 80% (w/w), and more preferably from 5 to 50% (w/w).

The total amount of surfactant in the carrier oil is preferably chosen in order to ensure stability of the emulsion and prevent spontaneous coalescence of droplets. Typically, the carrier oil comprises from 0.5 to 10% (w/w), preferably from 1 to 8% (w/w), and more preferably from 2 to 5% (w/w) of surfactant.

The emulsion can be prepared by any method known by the skilled artisan. Preferably, the emulsion can be prepared on a microfluidic system.

### Providing the first set of emulsion droplets comprising labelled molecular targets

The first set of emulsion droplets comprises droplets containing labelled molecular targets, wherein each of these droplets contains a plurality of molecular targets originating from no more than one entity and wherein, in each of these droplets, molecular targets are labelled with a molecular identification DNA sequence.

The first set of emulsion droplets may be a w/o or o/w/o emulsion depending on the nature of the entities. In some embodiments, the entities are cells or particles and emulsion droplets of the first set are w/o emulsion droplets. In some other embodiments, the entities are o/w emulsion droplets exposing molecular targets on their outer surfaces and emulsion droplets of the first set are o/w/o emulsion droplets.

Preferably, the first set of emulsion droplets comprises at least 10,000 droplets, preferably at least 100,000 droplets, preferably at least 500,000 droplets, and even more preferably at least 1,000,000 droplets.

In some embodiments, the first set of emulsion droplets is obtained by
- encapsulating entities within emulsion droplets, each droplet containing no more than one entity, and optionally lysing said entities within the droplets to release molecular targets and
- labelling said molecular targets.

In particular embodiments, entities are particles or o/w emulsion droplets exposing molecular targets on their outer surfaces, and the first set of emulsion droplets is obtained by
- encapsulating entities within emulsion droplets, each droplet containing no more than one entity, and
- labelling said molecular targets.

In some particular embodiments wherein entities are particles or o/w emulsion droplets exposing molecular targets on their outer surfaces, labelling of molecular targets may be performed in bulk, and then entities attached to labelled molecular targets may be encapsulated.

Thus, when the entity is a particle or an emulsion droplet exposing molecular targets on its outer surface, the method may comprise
- labelling said molecular targets, and
- encapsulating entities attached to labelled molecular targets within emulsion droplets, each droplet containing no more than one entity,
thereby obtaining the first set of emulsion droplets.

In preferred embodiments, entities are cells and the first set of emulsion droplets is obtained by
- encapsulating entities within emulsion droplets, each droplet containing no more than one entity,
- lysing said entities within the droplets to release molecular targets, and
- labelling said molecular targets.

To ensure single-entity resolution, entities have to be confined and isolated from the beginning to the end of the barcoding process. Encapsulation of entities into microfluidic droplets is a convenient way to isolate said entities and is particularly suitable to high throughput regimes.

Those of ordinary skill in the art is aware of techniques for encapsulating cells or particles within microfluidic droplets (see, for example, the international patent application WO 2004/091763). If cells are adherent or from tissue, they may be first dissociated and optionally filtered or centrifuged to remove clumps of two or more cells before encapsulation. Cells may typically be suspended in an aqueous buffer such as PBS buffer.

Methods for producing monodisperse w/o/w double emulsions, i.e. for encapsulating o/w droplets are also well known by the skilled person and microfluidic systems generating such emulsions are commercially available.

During encapsulation, the entity number density (entities per unit volume) has to be adjusted to minimize incidences of two or more entities becoming captured in the same droplet. In particular, entities may be encapsulated at a density of less than 1 entity per droplet, preferably at a density of less than 0.2 entity per droplet, in order to prevent co-encapsulation of two or more entities. In preferred embodiments, the entity number density and the average occupancy is adjusted in order to ensure that most, preferably at least 98%, or all of the droplets have only zero or one entity present in them.

Using any well-known microfluidic method to encapsulate entities into microfluidic droplets, entity-bearing droplets may be produced at high frequency, e.g. ranging from 0.5 kHz to 15 kHz, preferably from 1 kHz to 10 kHz, more preferably from 1 kHz to 5 kHz.

In embodiments wherein entities are cells, after encapsulation, cells may be lysed within the droplets in order to release molecular targets. Cell lysis may be performed using any method known by the skilled person such as using physical, chemical or biological means. In particular, cells may be lysed using radiation (e.g. UV, X or γ-rays) or laser (see e.g. Rau et al., 2004). The lysis may also be induced by osmotic shock or by addition of a detergent or enzyme (see, e.g. Kintses et al., 2012; Novak et al., 2011; Brown & Audet, 2008). The lysis may also be induced by heat shock.

In some embodiments, the lysis is induced by a lysis agent. Preferably, the lysis agent comprises one or several components altering the osmotic balance, one or several detergents and/or one or several enzymes. More preferably, the lysis agent is Triton X-100, BugBuster® reagent (Merck Millipore), Nonidet P40™ (MP BioMedical), M-PER™ (Thermo Scientific) or B-PER™ (Thermo Scientific).

In an embodiment, the lysis agent is directly added to the aqueous phase of the droplets before encapsulation. In such embodiment, an aqueous stream containing the cells may be combined with a stream of aqueous solution containing the lysis agent just before generation of droplets (see, e.g. Fig. 1A). The emulsion may be then generated, collected and incubated to allow cell lysis.

In another embodiment, the lysis agent is introduced inside the droplet after droplet generation by any known technique such as pico-injection or droplet fusion. The emulsion may be then collected and incubated to allow cell lysis.

Typically, the emulsion is incubated from 5 minutes to 1 hour and at a temperature ranging from 4°C to 25°C to allow cell lysis.

Alternatively, the lysis may be induced by a heat treatment. Typically, in this case, the emulsion may be incubated from 5 minutes to 1 hour and at a temperature up to 95°C to allow cell lysis.

The skilled person can easily adapted the incubation temperature during the lysis to the used method.

In some embodiments wherein the w/o interface of the first set droplets comprises functionalized surfactant(s), some or all molecular targets released by cell lysis, may be bound by said surfactant(s) and concentrated onto the inner w/o interface of droplets. Possibly, these w/o droplets can be convert into o/w droplets using droplet inversion as presented in international patent application WO 2017/174610.

In some embodiments wherein entities are particles or o/w emulsion droplets exposing molecular targets on their outer surfaces, molecular targets can be released from said particles or from the surface of said o/w emulsion droplets by the action of a cleaving agent (e.g. restriction enzyme).

Optionally, depending on the nature of molecular targets, one or several additional reagents may be added to the aqueous phase before collection and incubation of the first set emulsion. Examples of such additional reagents may include, but are not limited to DNases, RNases, proteases, protease inhibitors and/or nuclease inhibitors.

In some embodiments, molecular targets are RNA and one or several additional reagents, preferably comprising one or several DNases and/or one or several proteases, are added to the aqueous phase. In some other embodiments, molecular targets are proteins and/or RNA and additional reagents, preferably comprising one or several DNases, are added to the aqueous phase.

Additional reagent(s) and lysis agent may be added simultaneously to the aqueous phase, i.e. directly added to the aqueous phase of the droplets just before encapsulation or after droplet generation by any known technique such as pico-injection or droplet fusion. Alternatively, additional reagent(s) and lysis agent may be added sequentially. In particular, the lysis agent may be added to the aqueous phase before encapsulation by co-flowing a flow of an aqueous solution containing the entities and a flow of a solution containing the lysis agent, and additional reagent(s) may be added after encapsulation, and *vice-versa,* or the lysis agent and additional reagent(s) may be added sequentially after encapsulation, e.g. separate pico-injection or droplet fusion.

In preferred embodiments, additional reagent(s) and lysis agent are added simultaneously to the aqueous phase, i.e. directly added to the aqueous phase of the droplets just before encapsulation (see, e.g. Fig. 1A).

After encapsulation, and optionally cell lysis and /or elimination/degradation of some non-targeted molecules, as detailed above, molecular targets are labelled with a molecular identification DNA sequence comprising (i) a unique molecular identification (UMI) barcode which is different for each molecular target and (ii) an overhang or an overhang producing restriction site.

The "UMI sequence" or "UMI barcode" is a randomized nucleotide sequence assigning a unique barcode to each molecular target and thus allows further performing the digital detection/counting of molecular targets initially present into or onto the entity, their absolute quantification and correcting for amplification biases. Indeed, in a droplet, each probe carries a unique identification number (the UMI) and therefore counting the number of different UMI gives the absolute number of labelled molecular targets. Preferably, the UMI sequence is a randomized nucleotide sequence having a length of at least 5 nucleotides, preferably a length from 5 to 15 nucleotides, more preferably a length from 5 to 10 nucleotides. The randomized sequence can be a stretch of contiguous randomized nucleotides or a stretch of semi-randomized nucleotides (i.e. contiguous randomized nucleotides spaced by constant nucleotides). Typical examples of a stretch of semi-randomized nucleotides are stretches where several randomized dinucleotides are spaced by constant dinucleotides, or stretches where several randomized trinucleotides are spaced by constant trinucleotides. Preferably, the UMI sequence is a stretch of semi-randomized nucleotides, in particular a stretch where several randomized dinucleotides are spaced by constant dinucleotides.

An overhang is required to add the UEI barcode and optionally the UEI-calibrator barcode, as detailed below, after fusion with the droplets of the second set. This overhang may be a 3'overhang or a 5'overhang, preferably is a 3'overhang.

The molecular identification DNA sequence may comprise an overhang (3' or 5' overhang) compatible with a cohesive end generated by a restriction enzyme, or may comprise an overhang producing restriction site.

In some embodiments, the molecular identification DNA sequence comprises an overhang, preferably a 3' overhang, compatible with a cohesive end generated by a restriction enzyme. The choice of this configuration ensures that no complementary strand will be synthesized by the filling activity of a polymerase so that the extremity will stay competent for the later UEI addition. In addition, this overhang is compatible with the cleavage product of the restriction enzyme which is used to digest double stranded DNA sequences bearing UEI barcodes or UEI calibrators and comprised in the droplets of the second set as detailed below. Using digestion and ligation, this overhang allows UEI, and optionally UEI-calibrator, addition to each UMI sequence.

In some other embodiments, the molecular identification DNA sequence comprises an overhang producing restriction site, i.e. a restriction site generating a 3' or 5'overhang, preferably 3'overhang, which is compatible with the cleavage product of the restriction enzyme which is used to digest double stranded DNA sequences bearing UEI barcodes or UEI calibrators and comprised in the droplets of the second set. In a particular embodiment, the overhang producing restriction site on the molecular identification DNA sequence is recognized by the same enzyme than the overhang producing restriction site on double stranded DNA sequences bearing UEI calibrators (see below). In particular, overhangs on labelled molecular targets may be generated while digesting DNA sequences bearing UEI barcodes and DNA sequences bearing UEI calibrators after fusion of droplets of the first and second sets.

Alternatively, in embodiments wherein entities particles or o/w emulsion droplets exposing molecular targets on their outer surfaces, molecular targets may be released from said particles or from the surface of said o/w emulsion droplets by the action of a restriction enzyme, thereby generating a 3' or 5'overhang. Optionally, the molecular identification DNA sequence may further comprise a type identifier sequence which is a short predefined sequence, preferably having a length from 4 to 8 nucleotides, coding for the nature (e.g. nucleic acid or protein) and/or the identity (e.g. GFP mRNA) of the molecular target. Preferably, the type identifier sequence is proximal to the UMI sequence, more preferably directly adjacent to the UMI sequence.

In preferred embodiments, labelling of molecular targets with UMI barcodes is performed using probes, each probe comprising
a capture moiety capable of specific binding to a molecular target or specific ligation to a molecular target or an adaptor linked to said molecular target and
a DNA moiety comprising the molecular identification DNA sequence, i.e. (i) a region proximal to the capture moiety and comprising the unique molecular identification (UMI) sequence, and optionally a type identifier sequence, and (ii) a region distal from the capture moiety and comprising an overhang, preferably an overhang compatible with a cohesive end generated by a restriction enzyme, or an overhang producing restriction site. Preferably the region distal from the capture moiety comprises a 3'overhang or a 3'overhang producing restriction site.

In embodiments wherein the region distal from the capture moiety comprises a 3'overhang, the 5'end is a phosphorylated 5' end.

Preferably, the restriction site or overhang is separated from the UMI sequence by at least 10 nucleotides, preferably at least 20 nucleotides, and more preferably 20 to 40 nucleotides. Preferably, this separating region has a melting temperature of at least 50°C, more preferably at least 55°C, is GC rich in order to form stable duplexes, does not exhibit any sequence identity with a nucleic acid found in the organism from which the cell is originated and does not contain one of the restriction sites later used for labelling. In preferred embodiments, this region is identical for all probes.

In a particular embodiment, the DNA moiety comprises, from the capture moiety to the restriction site or overhang, a type identifier sequence of 4 to 8 nucleotides, preferably of 4 nucleotides, a UMI sequence of 5 to 15 nucleotides, preferably of 8 nucleotides, and a region of 20 to 40 nucleotides comprising the restriction site or overhang.

The nature of the capture moiety and the structure of the DNA moiety may differ according to type and/or the chemical structure of molecular targets. Since molecular targets of different types and/or chemical structures may be labelled simultaneously, probes contained in the same droplet may have different structures.

In some embodiments, at least some of molecular targets are nucleic acids and at least some probes specific of said nucleic acids are DNA probes comprising
a capture moiety which is a single stranded DNA region which drives the specific recognition of a nucleic acid molecular target, or the specific recognition of a nucleic acid adaptor linked to said molecular target, through conventional Watson-Crick base-pairing interactions, and
a DNA moiety comprising (i) a 3' single stranded region proximal to the capture moiety and comprising the unique molecular identification (UMI) sequence, and optionally a type identifier sequence, and (ii) a 5'double-stranded region distal from the capture moiety and comprising an overhang, preferably an overhang compatible with a cohesive end generated by a restriction enzyme, or an overhang producing restriction site.

Preferably, the DNA moiety comprises (i) a 3' single stranded region proximal to the capture moiety and comprising the unique molecular identification (UMI) sequence, and optionally a type identifier sequence, and (ii) a 5'double-stranded region distal from the capture moiety and comprising a 3'overhang, preferably compatible with a cohesive end generated by a restriction enzyme.

These DNA probes may be produced by any method known by the skilled person such as chemical synthesis.

An exemplary illustration of such a probe is presented in Figure 2.

In these embodiments, the length of the capture moiety has to be sufficient to allow the specific recognition of the target molecule through hybridization. Preferably, the capture moiety is a single stranded DNA region of at least 8 nucleotide long, preferably of 8 to 25 nucleotide long, more preferably of 10 to 15 nucleotide long.

The melting temperature (Tm) of the perfect hybrid formed upon association of the capture moiety with the molecular target is preferably adjusted (e.g. by modulating the length of the sequence specific to the target nucleic acid) in order to be ranged between 30°C and 70°C, preferably between 30°C and 60°C, more preferably between 40°C and 60°C, and even more preferably between 40°C and 50°C.

Preferably, the difference between melting temperature (Tm) of all probes specific to nucleic acid molecular targets, is lower than 3°C, more preferably lower than 2°C and even more preferably lower than 1°C.

The capture moiety may be specific to a particular DNA or RNA or may be complementary to a sequence region common to all RNAs, e.g. the capture moiety may be a poly-T tract which is complementary to the poly-A tails of eukaryotic mRNAs or complementary to a nucleic acid adaptor, preferably a DNA adaptor, added to all RNA, for instance through the action of an RNA ligase.

Thus, in some embodiments, the capture moiety drives the specific recognition of a nucleic acid adaptor linked to the molecular targets of interest, e.g. all RNAs. Such adaptor may be, for example, pre-adenylated oligonucleotides (5'-App oligos) which act as substrates for T4 ligases and thus can be ligated to any RNA molecule. Typically such adaptor may be a single stranded DNA region of at least 8 nucleotide long, preferably of 8 to 25 nucleotide, more preferably of 10 to 15 nucleotide long. An exemplary illustration of such embodiment is presented in Figure 3.

In some other embodiments, at least some of molecular targets are nucleic acids and at least some probes specific of said nucleic acids are DNA probes comprising
a capture moiety which is a single stranded DNA region which is able to ligate to a nucleic acid molecular target, and
a DNA moiety comprising (i) a 5' single stranded region proximal to the capture moiety and comprising the unique molecular identification (UMI) sequence, and optionally a type identifier sequence, and (ii) a 3'double-stranded region distal from the capture moiety and comprising an overhang, preferably an overhang compatible with a cohesive end generated by a restriction enzyme, or an overhang producing restriction site.

Preferably, the capture moiety is a 5' single stranded DNA region comprising 5',5'-adenyl pyrophosphoryl moiety (App) onto its 5'-end. Such moiety may act as substrate for T4 ligases and thus can be ligated to the 3' end any RNA molecule. In this embodiment, the 5'App extremity of the capture moiety directly interacts with the RNA molecular target and ligates to said target in the presence of T4 ligase. Exemplary illustrations of such embodiment are presented in Figures 4A and 4B.

These DNA probes may be produced by any method known by the skilled person such as chemical synthesis.

Preferably, the capture moiety is specific to a particular DNA or RNA molecule, more preferably is specific to a particular RNA molecule.

In some embodiments, nucleic acid molecular targets may be labelled using probes as described above, and in particular the capture moiety of said probes, as priming sites for a DNA polymerase synthetizing complementary strands of molecular targets. DNA and/or RNA molecular targets are converted into barcoded complementary DNA (cDNA) upon reverse transcription or other DNA polymerization reaction.

In some embodiments, some molecular targets are RNA molecules and the DNA polymerase is a reverse transcriptase. Upon hybridization of the probe, through its capture moiety, to the targeted RNA or the adaptor, reverse transcription can occur and first strand of complementary DNA (cDNA) can be synthesized, said cDNA comprising the UMI sequence of the DNA moiety of the probe.

It will be recognized that DNA polymerization reaction or reverse transcription requires appropriate conditions, for example the presence of an appropriate buffer and DNA polymerase enzyme, temperatures appropriate for annealing of the probes to targeted RNAs or DNAs and the activity of the enzyme and optionally presence of DTT. These conditions mainly depend on the polymerase and may be adapted according to the supplier guidance.

Preferably, in these embodiments, the method further comprises, after lysis step when necessary,
contacting molecular targets with a labelling mixture comprising said DNA probes as described above, at least one DNA polymerase, a dNTP mix (dATP, dCTP, dGTP, dTTP) and optionally an appropriate buffer, and
converting RNA molecular targets into cDNA comprising a molecular identification DNA sequence (UMI) using polymerization reaction.

Alternatively, or additionally, at least some probes may be chimeric molecules made of synthetic DNA oligonucleotides, i.e. the DNA moiety, covalently associated to a second molecule, i.e. the capture moiety, targeting specifically and with high affinity the target molecule and making possible to specifically label any molecule with a signal amplifiable and readable. These probes may be specific of any type of molecular targets, preferably are specific of protein molecular targets.

The capture moiety of these probes may be
(i) a binding moiety that specifically binds to a molecular target and is directly bound to the DNA moiety,
(ii) a chimeric protein comprising a first domain that specifically binds to a molecular target and a second domain that binds to the DNA moiety, or
(iii) a binding moiety that binds specifically to a molecular target and a protein bridge, said protein bridge comprising a first domain that binds to the binding moiety and a second domain that binds to the DNA moiety.

The term "specifically binding" or "specifically binds" is used herein to indicate that this moiety has the capacity to recognize and interact specifically with the molecular target of interest, while having relatively little detectable reactivity with other structures present in the aqueous phase such as other molecular targets that can be recognized by other probes. There is commonly a low degree of affinity between any two molecules due to non-covalent forces such as electrostatic forces, hydrogen bonds, Van der Waals forces and hydrophobic forces, which is not restricted to a particular site on the molecules, and is largely independent of the identity of the molecules. This low degree of affinity can result in non-specific binding. By contrast when two molecules bind specifically, the degree of affinity is much greater than such non-specific binding interactions. In specific binding a particular site on each molecule interacts, the particular sites being structurally complementary, with the result that the capacity to form non-covalent bonds is increased. Specificity can be relatively determined by binding or competitive assays, using e.g., Biacore instruments. The affinity of a molecule X for its partner Y can generally be represented by the dissociation constant (Kd). In preferred embodiments, the Kd representing the affinity between the capture moiety and the molecular target of interest is from 1.10⁻⁷M or lower, preferably from 1.10⁻⁸M or lower, and even more preferably from 1.10⁻⁹M or lower.

In some embodiments, at least some probes comprise a capture moiety which is a binding moiety that specifically binds to a molecular target and is directly bound to the DNA moiety. Preferably the binding moiety is covalently bound to the DNA moiety.

Examples of binding moieties include, but are not limited to, antibodies, ligands of ligand/anti-ligand couples, peptide and nucleic acid aptamers, protein tags, or chemical probes (e.g. suicide substrate) reacting specifically with a molecular target or a class of molecular targets.

Examples of ligand/anti-ligand couples include, but are not limited to, antibody/antigen or ligand/receptor. In particular, in some embodiments, the molecular target is an antibody and the binding moiety is an antigen recognized by said antibody, or *vice-versa.* In some other embodiments, the molecular target is a receptor and the binding moiety is a ligand recognized by said receptor, or *vice-versa.*

A multitude of protein tags are well-known by the skilled person (see for example Young et al. Biotechnol. J. 2012, 7, 620-634) and may be used in the present invention. Examples of such protein tags include, but are not limited to, biotin (for binding to streptavidin or avidin derivatives), glutathione (for binding to proteins or other substances linked to glutathione-S-transferase), lectins (for binding to sugar moieties), c-myc tag, hemaglutinin antigen (HA) tag, thioredoxin tag, FLAG tag, polyArg tag, polyHis tag, Strep-tag, OmpA signal sequence tag, calmodulin-binding peptide, chitin-binding domain, cellulose-binding domain, S-tag, and Softag3, and the like.

A multitude of chemical probes are well-known by the skilled person (see for example Niphakis and Cravatt, Ann. Rev. of Biochem. 2014, 83, 341-77 and Willems et al. Bioconjugate Chem. 2014, 25, 1181-91) and may be used in the present invention. Examples of chemical probes include, but are not limited to, electrophile or photoreactive Activity-Based Probes (ABP), suicide substrate-based ABP and inhibitors-based ABP.

In a particular embodiment, at least some probes are aptamer-based probes, i.e. probes comprising a nucleic acid or peptide aptamer as capture moiety. Similar to antibodies, aptamers interact with their targets by recognizing a specific three-dimensional structure. Aptamers can specifically recognize a wide range of targets, such as proteins, nucleic acids, ions or small molecules such as drugs and toxins.

Peptides aptamers consist of a short variable peptide loop attached at both ends to a protein scaffold such as the bacterial protein thioredoxin-A. Typically, the variable loop length is composed of ten to twenty amino acids. Peptide aptamer specific of a target of interest may be selected using any method known by the skilled person such as the yeast two-hybrid system or Phage Display. Peptides aptamers may be produced by any method known by the skilled person such as chemical synthesis or production in a recombinant bacterium followed by purification.

Preferably, at least some probes comprise a nucleic acid aptamer as capture moiety. Nucleic acid aptamers are a class of small nucleic acid ligands that are composed of RNA or single-stranded DNA oligonucleotides and have high specificity and affinity for their targets. Systematic Evolution of Ligands by EXponential enrichment (SELEX) technology to develop nucleic acid aptamers specific of a target of interest, is well known by the skilled person and may be used to obtain aptamers specific of a particular molecular target. Nucleic acid aptamers may be produced by any method known by the skilled person such as chemical synthesis or *in vitro* transcription for RNA aptamers. Preferably, nucleic acid aptamers used as capture moiety are selected from the group consisting of DNA aptamers, RNA aptamers, XNA aptamers (nucleic acid aptamer comprising xeno nucleotides) and spiegelmers (which are composed entirely of an unnatural L-ribonucleic acid backbone). An exemplary illustration of such a probe is presented in Figure 5A.

In another particular embodiment, at least some probes are antibody-based probes, i.e. probes comprising an antibody as capture moiety.

The term "antibody" herein is used in the broadest sense and specifically covers monoclonal antibodies, polyclonal antibodies, antibody fragments, and derivatives thereof, so long as they specifically bind to the molecular target of interest. In particular, the antibody may be a full length monoclonal or polyclonal antibody, preferably a full length monoclonal antibody. Preferably, this term refers to an antibody with heavy chains that contain an Fc region. By "Fc", "Fc fragment" or "Fc region", used herein is meant the polypeptide comprising the constant region of an antibody excluding the first constant region immunoglobulin domain. Thus Fc refers to the last two constant region immunoglobulin domains of IgA, IgD, and IgG, and the last three constant region immunoglobulin domains of IgE and IgM, and the flexible hinge N-terminal to these domains. Preferably, the antibody is a full length monoclonal or polyclonal IgG antibody, preferably a full length monoclonal IgG antibody. A large number of specific and high affinity monoclonal antibodies are currently available on the market.

As used herein, the term "antibody fragment" refers to a protein comprising a portion of a full length antibody, generally the antigen binding or variable domain thereof. Examples of antibody fragments include Fab, Fab', F(ab)₂, F(ab')₂, F(ab)₃, Fv (typically the VL and VH domains of a single arm of an antibody), single-chain Fv (ScFv), dsFv, Fd (typically the VH and CH1 domains) and dAb (typically a VH domain) fragments, nanobodies, minibodies, diabodies, triabodies, tetrabodies, kappa bodies, linear antibodies, and other antibody fragments that retain antigen-binding function (e.g. Holliger and Hudson, Nat Biotechnol. 2005 Sep;23(9): 1126-36). Antibody fragments can be made by various techniques, including but not limited to proteolytic digestion of intact antibody as well as recombinant host cells (e.g. *E. coli* or phage). These techniques are well-known by the skilled person and are extensively described in the literature. Preferably, the antibody fragment is selected from the group consisting of Fab', F(ab)₂, F(ab')₂, F(ab)₃, Fv, single-chain Fv (ScFv) fragments and nanobodies.

The term "antibody derivative", as used herein, refers to an antibody provided herein, e.g. a full-length antibody or a fragment of an antibody, wherein one or more of the amino acids are chemically modified, e.g. by alkylation, PEGylation, acylation, ester or amide formation or the like. In particular, this term may refer to an antibody provided herein that is further modified to contain additional nonproteinaceous moieties that are known in the art and readily available.

In some embodiments, the capture moiety is selected from the group consisting of monoclonal and polyclonal antibodies, Fab', F(ab)₂, F(ab')₂, F(ab)₃, Fv, single-chain Fv (ScFv) fragments and nanobodies, and derivatives thereof. In some preferred embodiments, the capture moiety is selected from the group consisting of a monoclonal antibody, a ScFv fragment or a nanobody.

In some embodiments, at least some probes comprise a capture moiety which is a chimeric protein comprising a first domain that specifically binds to a single molecular target and a second domain that binds to a single DNA moiety. Preferably the second domain is covalently bound to the DNA moiety.

The first domain of the chimeric protein specifically binds to a single molecular target. Examples of first domains include, but are not limited to, antibodies, ligands of ligand/anti-ligand couples, peptide and nucleic acid aptamers, protein tags, and chemical probes, as described above. Preferably, the first domain of the chimeric protein is selected from the group consisting of antibodies and peptide aptamers, more preferably is a monoclonal antibody.

In a particular embodiment, the first domain of the chimeric protein is an antibody, preferably selected from the group consisting of monoclonal and polyclonal antibodies, Fab', F(ab)₂, F(ab')₂, F(ab)₃, Fv, single-chain Fv (ScFv) fragments and nanobodies, and derivatives thereof. More preferably, the first domain of the chimeric protein is selected from the group consisting of a monoclonal antibody, a ScFv fragment or a nanobody, and even more preferably from the group consisting of a ScFv fragment or a nanobody.

The second domain that covalently binds to the DNA moiety may be any domain allowing covalently grafting of a single nucleic acid. Examples of such domains include, but are not limited to, SNAP-tag® (New England Biolabs), CLIP- tag® (New England Biolabs), Halo- tag® (Promega). Preferably, the second domain is a SNAP-tag®. The SNAP-tag is a 20 kDa mutant of the DNA repair protein O⁶-alkylguanine-DNA alkyltransferase that reacts specifically and rapidly with benzylguanine (BG) derivatives leading to irreversible covalent association of the SNAP-tag with the DNA moiety attached to BG.

The chimeric protein used as capture moiety and comprising the first and second domains may be produced as fusion protein using any well-known recombinant engineering technology, before to be covalently associated to the DNA moiety of the probe.

In some embodiments, at least some probes comprise a capture moiety comprising (i) a binding moiety that specifically binds to a molecular target and (ii) a protein bridge, said protein bridge comprising a first domain that binds to the binding moiety and a second domain that binds to the DNA moiety. Preferably the second domain is covalently bound to the DNA moiety. The first domain may be covalently or non-covalently bound to the binding moiety, preferably non-covalently bound. In embodiments wherein the first domain non-covalently binds to the binding moiety, the non-covalent interaction is preferably turned into covalent interaction by cross-linking the first domain and the binding moiety.

The second domain of the protein bridge may be as described above for the chimeric protein, i.e. any domain allowing covalently grafting of a single nucleic acid. Preferably, the second domain of the protein bridge is a SNAP-tag®.

The first domain of the protein bridge may be any domain allowing covalent or non-covalent interaction with the binding moiety, preferably non covalent interaction. Examples of such domain includes, but are not limited to, immunoglobulin-binding bacterial proteins such as protein A, protein A/G, protein G and protein L.

In an embodiment, the first domain of the protein bridge is an immunoglobulin-binding bacterial protein and the binding moiety is an antibody, preferably an antibody containing a Fc region, more preferably a full length monoclonal or polyclonal IgG antibody, preferably a full length monoclonal IgG antibody. The immunoglobulin-binding bacterial protein is preferably selected from protein A, protein A/G, protein G and protein L, one or several IgG-binding domains thereof, and functional derivatives thereof.

Protein A is a cell surface protein found in *Staphylococcus aureus.* It has the property of binding the Fc region of a mammalian antibody, in particular of IgG class antibodies. The amino-terminal region of this protein contains five highly homologous IgG-binding domains (termed E, D, A, B and C), and the carboxy terminal region anchors the protein to the cell wall and membrane. All five IgG-binding domains of protein A bind to IgG via the Fc region and in principle, each of these domains is sufficient for binding to the Fc-portion of an IgG.

Thus, in a particular embodiment, the first domain of the protein bridge is selected from the group consisting of domains A, B, C D and E of protein A, combinations thereof and functional derivatives thereof retaining IgG binding functionality of wild-type protein A. Preferably, the first domain of the protein bridge comprises domains A to E of protein A.

The protein bridge may be produced as fusion protein using any well-known recombinant engineering technology, before to be covalently associated to the DNA moiety of the probe.

Preferably, the first domain is located at the N-terminal part of the protein bridge and the second domain is located at the C-terminal part of the protein bridge.

Optionally, the protein bridge may further comprise at the C-terminal extremity an affinity tag (e.g. a polyhistidine-tag) to facilitate its purification.

In a particular embodiment, the protein bridge comprises an immunoglobulin-binding bacterial protein, preferably domains A to E of protein A, as first domain, a SNAP-tag® as second domain and a monoclonal or polyclonal IgG antibody as binding moiety, preferably a monoclonal IgG antibody. An exemplary illustration of such a probe is presented in Figure 5B and C.

The binding moiety may be covalently or non-covalently bound to the protein bridge. In some embodiments, the protein bridge can be cross-linked with the binding moiety to ensure long-term physical link.

In chimeric probes described above, the DNA moiety consists of a double stranded DNA molecule comprising an overhang, preferably compatible with a cohesive end generated by a restriction enzyme, or an overhang producing restriction site as described above for DNA probes. Preferably, the overhang comprised in the DNA moiety or generated by the restriction site is a 3'overhang. As described above, the DNA moiety comprises a unique molecular identification (UMI) sequence, and optionally a type identifier sequence.

Preferably, in chimeric probes, the DNA moiety further comprises a sequencing primer annealing sequence which is proximal to the capture moiety. After labelling of molecular targets, this sequence allows direct amplification using sequencing primers.

Preferably, in embodiments wherein at least some probes are chimeric probes, the method further comprises, after lysis step when necessary, contacting molecular targets with a labelling mixture comprising at least one chimeric probe.

The labelling mixture comprising probes, and optionally reagents to perform DNA polymerization reaction, may be added to the aqueous phase of the droplets before encapsulation of cells (i.e. by direct inclusion in the mixture or *via* a co-flow) or after droplet generation by any known technique such as pico-injection or droplet fusion. Preferably, the labelling mixture is added after incubation allowing cell lysis and/or elimination/degradation of some non-targeted molecules.

In a particular embodiment, the labelling mixture is encapsulated into w/o droplets and these droplets are fused with droplets comprising molecular targets. The content of each droplet comprising the labelling mixture should be substantially identical. Methods to produce such emulsion are well known by the skilled person as well as methods of generating, synchronizing and fusing emulsion droplets, in particular on a microfluidic device.

### Providing the second set of emulsion droplets comprising cell identification sequences

The second set of emulsion droplets comprises droplets containing entity identification sequences, wherein each of these droplets contains at least one entity identification sequence.

The entity identification sequence is a double stranded DNA sequence of 40 to 100 nucleotide long, preferably of 50 to 70 nucleotide long, comprising a unique entity identification (UEI) barcode which is different for each droplet of the second set, and an overhang producing restriction site, preferably a 3 'overhang producing restriction site.

The "UEI sequence" or "UEI barcode" is a randomized nucleotide sequence assigning the same barcode to each molecular target originating from the same entity. Preferably, the UEI sequence is a randomized nucleotide sequence having a length of at least 8 nucleotides, preferably a length from 8 to 20 nucleotides, more preferably a length from 8 to 15 nucleotides. The randomized sequence can be a stretch of contiguous randomized nucleotides or a stretch of semi-randomized nucleotides (i.e. contiguous randomized nucleotides spaced by constant nucleotides). Typical examples of a stretch of semi-randomized nucleotides are stretches where several randomized dinucleotides are spaced by constant dinucleotides, or stretches where several randomized trinucleotides are spaced by constant trinucleotides. Preferably, the UEI sequence is a stretch of semi-randomized nucleotides, in particular a stretch where several randomized dinucleotides are spaced by constant dinucleotides.

The restriction site comprised in the entity identification sequence may generate, upon digestion with the corresponding restriction enzyme, an overhang compatible with the overhangs of labelled molecular targets, or an overhang compatible with the overhangs of UEI calibrators as described below, and thus allows addition of UEI sequence to the molecular identification DNA sequence. Preferably, this restriction site is a non-palindromic cleavage site.

Optionally, the entity identification sequence may further comprise a sequencing primer annealing sequence adjacent to the UEI barcode and at the opposite end of the restriction site.

In preferred embodiments, the entity identification sequence comprises a constant region allowing for amplification of the UEI-bearing DNA, adjacent to the restriction site and at the opposite end of the UEI barcode and the sequencing primer annealing sequence when present. Preferably, this constant region has a length of 15 to 35 nucleotides, preferably of 20 to 30 nucleotides. Preferably, this constant region has a melting temperature comprised between 50°C and 70°C.

An exemplary illustration of such entity identification sequence is presented in Figure 6.

Preferably, all entity identification sequences have to same sequence except for the UEI barcode sequence, i.e. they exhibit the same sequencing primer annealing sequence, the same restriction site and the same constant region.

Whereas the UEI sequence has to be different for each droplet, it also has to be present in large number of identical copies in each droplet.

Thus, in an embodiment, the method further comprises encapsulating a plurality of entity identification sequences within emulsion droplets, each droplet containing no more than one entity identification sequence, with an amplification reaction mixture, and amplifying the entity identification sequences within droplets, thereby obtaining the second set of emulsion droplets. This way, more than one million of copies of the same entity identification sequence can be generated in each droplet and later serve to barcode the previously labeled molecular targets with an identical and unique UEI sequence. Entity identification sequences may be encapsulated in single or double stranded form, preferably in double stranded form.

The amplification reaction mixture comprises all reagents required to perform DNA amplification into the droplets, i.e. typically a DNA polymerase, primers, buffers, dNTPs, salts (e.g. MgCl₂), etc... Primers are designed in order to allow the complete amplification of the entity identification sequence. In certain embodiments, amplification relies on alternating cycles of heating and cooling (i.e., thermal cycling) to achieve successive rounds of replication (e.g., PCR). Methods of amplifying genetic elements compartmentalized in emulsion droplets are well-know and widely practiced by the skilled person (see for example, Chang et al. Lab Chip. 2013 Apr 7;13(7):1225-42; Zanoli and Spoto, Biosensors 2013, 3, 18-43). In particular, the amplification may be performed by any known technique such as polymerase chain reaction (PCR), nucleic acid sequence-based amplification (NASBA), loop-mediated isothermal amplification (LAMP), helicase-dependent amplification (HDA), rolling circle amplification (RCA), multiple displacement amplification (MDA) and recombinase polymerase amplification (RPA). The suitable method can be easily chosen by the skilled person depending on the nature of the encapsulated genetic element and the molecular target. Preferably, entity identification sequences are amplified into the droplets by PCR amplification.

Preferably, in this embodiment, in order to prevent co-encapsulation of more than one entity identification sequence in one droplet, and thus to preserve single-entity resolution, the DNA solution comprising entity identification sequences is strongly diluted, e.g. in the amplification reaction mixture, such that, according to Poisson statistics driving molecule distribution, droplet occupancy, i.e. the percentage of droplets comprising a entity identification sequence, is limited to less than 20%, preferably to less than 10%. Thus, in particular embodiments, the method further comprises, before encapsulating entity identification sequences and amplification reaction mixture within emulsion droplets, diluting entity identification sequences, preferably into the amplification reaction mixture, in order to obtain a droplet occupancy of less than 20%, preferably less than 10%.

In order to increase droplet occupancy, encapsulation of several entity identification sequence in one droplet may be tolerated. In this case, the inventors found that single-entity resolution can be preserved by adding UEI-calibrator molecules to entity identification sequences. Thus, in a preferred embodiment, the method further comprises
encapsulating a plurality of entity identification sequences within emulsion droplets in the presence of UEI-calibrators and an amplification reaction mixture, wherein at least some droplets comprise one or several entity identification sequences and one or several UEI-calibrators; and
amplifying entity identification sequences and/or UEI-calibrators within droplets, preferably entity identification sequences and UEI-calibrators using a multiplex reaction;
thereby obtaining the second set of emulsion droplets.

Entity identification sequences and UEI-calibrators may be encapsulated in single or double stranded form, preferably in double stranded form.

Preferably, for multiplex reaction, a higher amount of primers specific of the entity identification sequences is used relative to those specific for the UEI-calibrators.

The possibility of co-encapsulating several entity identification sequences in the same droplets leads to a dramatic increase of droplet occupancy. Indeed, adjusting the dilution of the entity identification sequence solution in order to co-encapsulate five entity identification sequences per droplets, leads to a droplet occupancy close to 100%.

Preferably, dilutions of entity identification sequence solution and UEI-calibrator solution are adjusted in order to co-encapsulate 2 to 10 entity identification sequences per droplet, preferably 4 to 6 entity identification sequences per droplet, and 2 to 10 UEI-calibrators per droplet, preferably 4 to 6 UEI-calibrators per droplet.

UEI-calibrators are DNA sequences, preferably double stranded DNA sequences, comprising a unique calibrator barcode which is different for each UEI-calibrator and for each droplet, and one or two overhang producing restriction sites, preferably generating overhangs compatible with overhangs of digested entity identification sequences and/or labelled molecular targets.

Preferably, these overhang producing restriction sites are non-palindromic cleavage sites. Preferably, these restriction sites generate 3' overhangs. Preferably, these restriction sites are different from the restriction site generating compatible overhangs on entity identification sequences and/or the restriction site generating compatible overhangs on labelled molecular targets.The "UEI-calibrator sequence" or "UEI-calibrator barcode" is a randomized nucleotide sequence having a length of at least 15 nucleotides, preferably a length from 15 to 40 nucleotides, more preferably a length from 15 to 20 nucleotides. The randomized sequence can be a stretch of contiguous randomized nucleotides or a stretch of semi-randomized nucleotides (i.e. contiguous randomized nucleotides spaced by constant nucleotides). Typical examples of a stretch of semi-randomized nucleotides are stretches where several randomized dinucleotides are spaced by constant dinucleotides, or stretches where several randomized trinucleotides are spaced by constant trinucleotides. Preferably, the UEI-calibrator barcode is a stretch of semi-randomized nucleotides, in particular a stretch where several randomized dinucleotides are spaced by constant dinucleotides.

In an embodiment, UEI-calibrators comprise an overhang producing restriction site which generates, upon digestion with the corresponding restriction enzyme, an overhang compatible with overhangs of digested entity identification sequences comprising UEI barcodes. In this embodiment, since both types of molecules, i.e. UEI-calibrator sequences and entity identification sequences, carry restriction sites generating compatible extremities, the subsequent digestion/ligation step used for UEI addition to labelled molecular targets, leads to the formation of different chimeras between UEI and UEI-calibrator barcodes. Indeed, whereas a large fraction of UEI barcodes will stay available for the barcoding of the labeled molecular targets originating from the entity, a sub fraction of UEI barcodes will get associated to each of the UEI-calibrator barcodes. These pairs of sequences constitute a signature unique to each droplet and allow reassigning each analyzed molecule to its original entity, despite the presence of multiple entity identification sequences in some droplets (see e.g. Figure 7). Optionally, in this embodiment, UEI-calibrators may further comprise a sequencing primer annealing sequence adjacent to the UEI-calibrator barcode and at the opposite end of the restriction site generating overhangs compatible with digested entity identification sequences. Optionally, UEI-calibrators may further comprise a binding tag allowing specific capture of the molecule at the extremity proximal to the sequencing primer annealing sequence. Preferably, the binding tag is selected from biotin or digoxigenin, more preferably is biotin.

Preferably, in this embodiment, UEI-calibrators comprise a constant region adjacent to the restriction site and at the opposite end of the UEI-calibrator barcode and the sequencing primer annealing sequence when present. This constant region allows amplifying UEI-calibrators, preferably using PCR amplification. In particular, this region may comprise a primer binding site. Preferably, this constant region has a length of 10 to 35 nucleotides, preferably of 15 to 25 nucleotides. Preferably this constant region is orthogonal to that of entity identification sequences in order to prevent unwilling hybridization. UEI-calibrators may further comprise an additional region comprised between the UEI-calibrator barcode and the sequencing primer annealing sequence when present. This region acts as a spacer between the sequencing primer annealing sequence and the UEI-calibrator barcode and may be used to adjust the length of the amplified sequences comprising UEI and UEI-calibrator barcodes to the length of the amplified sequences comprising UMI and UEI barcodes to limit potent amplification biases. An exemplary illustration of such UEI-calibrator is presented in Figure 9.

In another embodiment, UEI-calibrators comprise two overhang producing restriction sites, a first restriction site generating an overhang compatible with overhangs of digested entity identification sequences comprising UEI barcodes and a second restriction site generating an overhang compatible with overhangs of labelled molecular targets. In this embodiment, the subsequent digestion/ligation step used for UEI addition to labelled molecular targets, leads to the formation of tripartite molecules comprising a labelled molecular target, an UEI sequence and a UEI-calibrator barcode (see e.g. Figure 8).

In this embodiment, UEI-calibrators may comprise constant regions adjacent to each of the two overhang producing restriction sites. These constant regions allow amplifying UEI-calibrators, preferably using PCR amplification. In particular, these regions may comprise primer binding sites. Preferably, these constant regions have a length of 10 to 35 nucleotides, preferably of 15 to 25 nucleotides. Preferably these constant regions are orthogonal to that of entity identification sequences in order to prevent unwilling hybridization. An exemplary illustration of such UEI-calibrator is presented in Figure 9.

Preferably, all UEI-calibrators have to same sequence except for the UEI-calibrator barcode sequence, i.e. they exhibit, the same restriction site, the same constant region(s) and optionally the same sequencing primer annealing sequence.

As described above, multiplex amplification of entity identification sequences and UEI-calibrators within droplets may be performed using any method known by the skilled person, preferably multiplex PCR. The amplification reaction mixture comprises all reagents required to perform DNA amplification into the droplets, i.e. typically a DNA polymerase, primers, buffers, dNTPs, salts (e.g. MgCl₂), etc... and primers are designed in order to allow the complete amplification of entity identification sequences and UEI calibrators.

Preferably, in embodiments producing chimeras between UEI and UEI-calibrator barcodes, in order to ensure a large over-representation of entity identification sequences and allow the proper barcoding of molecular targets after droplet fusion, the amplification reaction mixture comprises a higher amount of primers specific of the entity identification sequences relative to those specific for the UEI-calibrators. More preferably, the amplification reaction mixture comprises at least 5 times, preferably at least 10 times, and more preferably at least 100 times, higher amount of primers specific of the cell identification sequences relative to those specific for the UEI-calibrators.

In embodiments producing tripartite molecules comprising a labelled molecular target, an UEI sequence and a UEI-calibrator barcode, the amplification reaction mixture may comprise similar amounts of primers specific of the entity identification sequences and primers specific for the UEI-calibrators.

Optionally, UEI sequence and UEI-calibrators may be assembled through their compatible overhangs before amplification. In this case, amplification reaction therefore directly amplify a fragment comprising UEI sequence and UEI-calibrators. After fusion with the first set of droplets, said fragment is ligated to labelled molecular targets.

### Droplet fusion and incorporation of UEI barcodes, and optionally UEI-calibrator barcodes, into labelled molecular targets

As described above, the first set of emulsion droplets comprises molecular targets labelled with molecular identification sequence comprising UMI barcodes, and the second set of emulsion droplets comprises entity identification sequences comprising UEI barcodes, and optionally UEI calibrators.

The method of the invention comprises fusing droplets of the first set with droplets of the second set wherein a droplet of the first set is fused with no more than one droplet of the second set.

Any technique known by the skilled person may be used to fuse a first droplet and a second droplet together to create a combined droplet. For example, opposite electric charges may be given to the first and second droplets (i.e., positive and negative charges, not necessarily of the same magnitude), which may increase the electrical interaction of the two droplets such that fusion or coalescence of the droplets can occur due to their opposite electric charges. For instance, an electric field may be applied to the droplets, the droplets may be passed through a capacitor, a chemical reaction may cause the droplets to become charged, etc.

Any technique known by the skilled person may be used to ensure that a droplet of the first set is fused with no more than one droplet of the second set. In particular, droplets may be paired through a pairing channel before to reach the coalescence point. The use of such channel is well-known by the skilled person in order to control fusion of microfluidic droplets. In embodiments wherein a pairing channel is used, droplets of the first and second sets should have different sizes. The pairing channel is a long channel having a width larger than the smallest droplets and narrower than the largest droplets. As a consequence, the small droplet catches the large one and pairs of droplets are formed at the exit of the channel. This configuration ensures that droplets properly pairwise prior to reaching the coalescence point. An exemplary embodiment of such pairing channel is illustrated in Figure 10.

In some embodiments, droplets of the first set and/or droplets of the second sets are generated on separate microfluidic system(s) and re-injected into the device. Preferably, droplets are spaced with oil streams and synchronized before to enter the pairing channel.

Preferably at least 60%, more preferably at least 80% of droplets, and even more preferably at least 95% of the first set are fused with a droplet of the second set.

After droplet fusion, UEI barcodes are incorporated into labelled molecular targets through restriction enzyme digestion and ligation.

In embodiments wherein droplets of the second set do not comprise UEI calibrators, UEI barcodes are incorporated into labelled molecular targets through restriction enzyme digestion and ligation of the compatible overhangs of labelled molecular targets and digested entity identification sequences comprising UEI barcodes.

In embodiments wherein droplets of the second set comprise UEI calibrators and said UEI calibrators comprise a overhang producing restriction site, (i) UEI calibrator barcodes and UEI barcodes on one hand, and (ii) UEI barcodes and labelled molecular targets on the other hand, may be assembled through restriction enzyme digestion and ligation of compatible overhangs of (i) UEI calibrators and entity identification sequences and of (ii) entity identification sequences and labelled molecular targets. In this embodiment, entity identification sequences are appended to both the labelled molecular targets and the UEI-calibrators through a digestion/ligation coupled reaction. An exemplary illustration of such embodiment is presented in Figure 7B. Alternatively, UEI calibrator barcodes, UEI barcodes and labelled molecular targets are assembled through restriction enzyme digestion and ligation of compatible overhangs of i) UEI calibrators and entity identification sequences and (ii) entity identification sequences and labelled molecular targets.

In embodiments wherein droplets of the second set comprise UEI calibrators and said UEI calibrators comprise two overhang producing restriction sites, UEI calibrator barcodes, UEI barcodes and labelled molecular targets are assembled to through restriction enzyme digestion and ligation of compatible overhangs of (i) UEI calibrators and labelled molecular targets on one hand and (ii) UEI calibrators and entity identification sequences on the other hand. In this embodiment, the entity identification sequence, the UEI-calibrator and the labelled molecular target together form a tripartite molecule. An exemplary illustration of such embodiment is presented in Figure 8.

In an embodiment, restriction sites comprised on entity identification sequences and UEI calibrators are identical and only one restriction enzyme is used to digest entity identification sequences and UEI calibrators. In embodiments wherein molecular identification DNA sequences comprise a restriction site generating an overhang, this restriction site and restriction sites comprised on entity identification sequences and UEI calibrators may be identical or different, preferably different.

In another embodiment, restriction sites comprised on entity identification sequences and UEI calibrators are different and two different restriction enzymes are used to digest entity identification sequences and UEI calibrators. The use of different restriction sites and of labels with compatible extremities ensures that a productive ligation event leads to the destruction of the restriction site. Therefore, the resulting chimeric molecule will not be a substrate of the restriction enzymes present in the mixture and the equilibrium is pulled toward the formation of the wished ligation products. In embodiments wherein molecular identification DNA sequences comprise a restriction site generating an overhang, this restriction site may be identical or different of the restriction site comprised on entity identification sequences or on UEI calibrators. Preferably, this restriction site is identical to the restriction site comprised on UEI calibrators.

Preferably, these restriction sites are non-palindromic in order to ensure directionality of the association of cell identification sequences, UEI calibrators and labelled molecular targets.

In embodiments wherein UEI sequence and UEI-calibrators are assembled through their compatible overhangs before amplification, i.e. before fusion of the two sets of droplets, the fragment comprising UEI sequence and UEI-calibrators can be ligated to labelled molecular targets through compatible overhangs of i) UEI calibrators and labelled molecular targets or (ii) entity identification sequences and labelled molecular targets.

Restriction enzymes, DNA ligase and optionally buffer may be added to the droplets after droplet fusion, e.g. by pico-injection or droplet fusion, or concomitantly to droplet fusion, e.g. by injection at a T-junction (see e.g. figure 10). Injection may be performed at the coalescence point where the presence of electrodes triggers both droplet fusion and enzyme delivery.

The emulsion may be then collected and incubated to allow digestion and ligation and thus association of UEI barcodes and labelled molecular targets, and UEI-calibrator barcodes when present.

In a particular embodiment wherein the molecular target is a nucleic acid, preferably a RNA, a sequencing primer annealing sequence may be added to labelled molecular target after incorporating UEI barcodes, and optionally UEI-calibrator barcodes. In particular, the method may further comprise, after incorporating UEI barcodes, and optionally UEI-calibrator barcodes, performing primer extension reaction using primers comprising from their 3'end to their 5'end, a region that hybridizes to complementary strands of molecular targets, i.e. to cDNA, and a sequencing primer annealing sequence.

Optionally, at their 5' extremity, the primers may further comprise a binding tag allowing the specific capture of primer extension reaction products. Preferably, the binding tag is biotin or digoxigenin, more preferably is biotin.

Primer extension reaction may be performed as described above and reaction mixture may be brought into the droplet using any known method such as pico-injection or droplet fusion. Alternatively, the primer extension reaction can be performed in bulk upon droplet breaking and content recovery.

After completion of the labelling process, droplets may be broken and their content may be recovered, i.e. droplet lysate, e.g. to be further analysed/sequenced.

### Analysis of molecular targets labelled with the method of the invention

In a further aspect, the present invention also relates to a method of quantifying one or several molecular targets from a plurality of entities with single-entity resolution, said method comprising
labelling said molecular targets according to the method of the invention as described above, capturing said labelled molecular targets comprising UMI, UEI, and optionally UEI calibrator barcodes,
amplifying sequences comprising UMI and UEI barcodes, and optionally UEI-calibrator barcodes
sequencing said amplified sequences.

All embodiments described above for the method of the invention are also encompassed in this aspect.

The step of capturing labelled molecular target comprising UMI, UEI, and optionally UEI calibrator barcodes, allows removing from the droplet lysate untargeted molecules, unreacted entity identification sequences and/or UEI calibrators when present. This step may be performed using any capture molecule which is able to specifically bind molecular targets such as an antibody or nucleic acid specific of a molecular target, attached to a support. The capture molecule may be directly or indirectly attached to the support. In particular, the capture molecule may comprise a binding tag, e.g. biotin, interacting with a partner, e.g. streptavidin, linked to the support. The capture molecule may be for example a biotinylated monoclonal antibody specific of a targeted protein, or synthetic DNA oligonucleotide specific of a cDNA produced from a targeted RNA. A binding tag can be added during the synthesis of the second cDNA strand by primer extension mentioned above. The support may be chosen in order to allow washing of unreacted molecules. Preferably the support is beads, more preferably streptavidin conjugated beads. In some preferred embodiments, the support is magnetic beads, in particular streptavidin conjugated magnetic beads.

The nucleic acids comprising (i) the molecular identification DNA sequence comprising the UMI barcode and optionally the type identifier and (ii) the entity identification sequence comprising the UEI barcode are then sequenced using any method known by the skilled person, preferably using a next generation sequencing method.

In some embodiments wherein the entity identification sequence, the UEI-calibrator and the labelled molecular target together form a tripartite molecule, the nucleic acids to be sequenced comprise i) the molecular identification DNA sequence comprising the UMI barcode and optionally the type identifier, (ii) the entity identification sequence comprising the UEI barcode and (iii) the UEI-calibrator barcode.

In some other embodiments wherein the entity identification sequences are separately associated with UEI-calibrators and with molecular identification DNA sequence, the nucleic acids to be sequenced comprise (i) the molecular identification DNA sequence comprising the UMI barcode and optionally the type identifier and the entity identification sequence comprising the UEI barcode, and (ii) the entity identification sequence comprising the UEI barcode and the UEI-calibrator barcode.

In some embodiments, wherein said nucleic acids do not comprise any sequencing primer annealing sequences, said sequences may be added before the sequencing step, preferably by DNA amplification using primers comprising said sequences or by ligation of oligonucleotides comprising said sequences.

Preferably, when the molecular target is RNA, a part of this RNA is also sequenced.

One of the main advantage of the present invention is the possibility of pooling all captured labelled molecular targets prior to the sequencing step, whatever the type of molecular targets (e.g. RNA or protein). Sequences may be then analyzed using any method known by the skilled person such as bioinformatics to cluster said sequences and determining the absolute quantification of molecular targets. Firstly, UEI and UEI-calibrator barcodes are used to cluster the set of UEI labeling the molecules originating from the same entity. Secondly, UEI barcodes are used to cluster molecules according to their entity of origin. Third, molecules are clustered according to their type and identity, optionally using the type Identifiers, and, finally, redundant sequences are eliminated using the UMI barcodes giving access to the absolute quantification of each molecule with single-entity resolution.

### Microfluidic devices

A further disclosure relates to a microfluidic device suitable for implementing at least one step of the methods of the invention.

All embodiments described above for the methods of the invention are also encompassed in this disclosure

As used herein, the term "microfluidic device", "microfluidic chip" or "microfluidic system" refers to a device, apparatus or system including at least one microfluidic channel.

The microfluidic system may be or comprise silicon-based chips and may be fabricated using a variety of techniques, including, but not limited to, hot embossing, molding of elastomers, injection molding, LIGA, soft lithography, silicon fabrication and related thin film processing techniques. Suitable materials for fabricating a microfluidic device include, but are not limited to, cyclic olefin copolymer (COC), polycarbonate, poly(dimethylsiloxane) (PDMS), poly(methyl methacrylate) (PMMA), and glass. Preferably, microfluidic devices are prepared by standard soft lithography techniques in PDMS and subsequent bonding to glass microscope slides. Due to the hydrophilic or hydrophobic nature of some materials, such as glass, which adsorbs some proteins and may inhibit certain biological processes, a passivating agent may be necessary. Suitable passivating agents are known in the art and include, but are not limited to silanes, fluorosilanes, parylene, *n*-dodecyl-β-D-maltoside (DDM), poloxamers such as Pluronics.

As used herein, the term "channel" refers to a feature on or in an article (e.g., a substrate) that at least partially directs the flow of a fluid. The term "microfluidic channel" refers to a channel having a cross-sectional dimension of less than 1 mm, typically less than 500 µm, 200 µm, 150 µm, 100 µm or 50 µm, and a ratio of length to largest cross-sectional dimension of at least 2:1, more typically at least 3: 1, 5:1, 10: 1 or more. It should be noted that the terms "microfluidic channel", microchannel" and "channel" are used interchangeably in this description. The channel can have any cross-sectional shape (circular, oval, triangular, irregular, square or rectangular, or the like). Preferably, the channel has a square or rectangular cross-sectional shape. The channel can be, partially or entirely, covered or uncovered. As used herein, the term "cross-sectional dimension" of a channel is measured perpendicular to the direction of fluid flow.

The microfluidic device of the invention comprises
- a first emulsion re-injection module or on-chip droplet generation module;
- a second emulsion re-injection module or on-chip droplet generation module
- a droplet-pairing module, and
- a module coupling droplet fusion to injection,
wherein emulsion re-injection modules and/or on-chip droplet generation modules are in fluid communication and upstream to the droplet-pairing module, the droplet-pairing module is in fluid communication and upstream to the module coupling droplet fusion to injection.

As used herein, the term "upstream" refers to components or modules in the direction opposite to the flow of fluids from a given reference point in a microfluidic system.

As used herein, the term "downstream" refers to components or modules in the direction of the flow of fluids from a given reference point in a microfluidic system.

In an embodiment, the microfluidic device of the invention comprises
two emulsion re-injection modules
a droplet-pairing module, and
a module coupling droplet fusion to injection.

An exemplary embodiment of such microfluidic device is presented in Figure 10.

In another embodiment, the microfluidic device of the invention comprises
an emulsion re-injection module and an on-chip droplet generation module,
a droplet-pairing module, and
a module coupling droplet fusion to injection.

In a further embodiment, the microfluidic device of the invention comprises
two on-chip droplet generation modules,
a droplet-pairing module, and
a module coupling droplet fusion to injection.

The emulsion re-injection module may be easily designed by the skilled person based on any known techniques. Typically, an emulsion re-injection module comprises a ψ-shaped structure where injected droplets are spaced by carrier oil supplying by at least one, preferably two side channels connected with the re-injection channel.

The module for generating droplets may be easily designed by the skilled person based on any known techniques. For example, emulsion droplets may be produced in the droplet generation module by any technique known by the skilled person such as drop-breakoff in co-flowing streams, cross-flowing streams in a T-shaped junction (see for example WO 2002/068104), and hydrodynamic flow-focussing (reviewed by Christopher and Anna, 2007, J. Phys. D: Appl. Phys. 40, R319-R336).

As explained above, the droplet-pairing module is a channel with dimensions allowing the contact between droplets of the two sets. In an embodiment, the width of the channel is about the diameter of the larger droplets and the depth of the channel is lower than the diameter of the larger droplets. In another embodiment, the depth of the channel is about the diameter of the larger droplets and the width of the channel is lower than the diameter of the larger droplets.

The length of the pairing channel has to be sufficient to obtain a contact between droplets of the first and second sets. Preferably, the time of contact is greater than 1 ms, preferably greater than 4 ms. As used herein, "the contact time τ" refers to the time in which paired droplets stay in physical contact before reaching the end of the pairing channel. Typically, the length of the pairing channel is ranging from 100 µm to 10 mm, preferably from 500 µm to 2 mm, and more preferably is about 1.5 mm.

The module coupling droplet fusion to injection is preferably a module wherein droplets, after pairing, are exposed to an electric field destabilizing their interface thanks to the proximity of electrodes, and are, in the same time, contacted with a stream injected in the channel. Destabilization of the interface then leads not only to droplet coalescence but also to infusion of the injected stream into droplets.

The microfluidic device may further comprise a collection module wherein fused droplets are recovered.

Optionally, the microfluidic device of the invention may comprise an inlet downstream to the module coupling droplet fusion to injection and upstream to the collection module. This inlet may be used to inject additional surfactant in order to increase the stability of fused droplets and to prevent any coalescence during the storage.

In another aspect, the present invention also relates to a kit comprising a microfluidic device according to the invention and as described above.

The kit may further comprise one or several microfluidic chips comprising an on-chip droplet generation module.

The kit of the invention may further comprise
- one or several probes as described above; and/or
- one or several entity identification sequences as described above; and/or
- one or several UEI-calibrators as described above; and/or
- one or several primers suitable to amplify entity identification sequences and/or UEI-calibrators; and/or
- an aqueous phase and/or an oil phase; and/or
- a leaflet providing guidelines to use such a kit.

All embodiments described above for the methods and the microfluidic device of the invention are also encompassed in this aspect.

In another aspect, the present invention also to a kit comprising
- one or several probes as described above; and/or
- one or several entity identification sequences as described above; and/or
- one or several UEI-calibrators as described above; and/or
- one or several primers suitable to amplify entity identification sequences and/or UEI-calibrators; and/or
- an aqueous phase and/or an oil phase; and/or
- one or several microfluidic devices, in particular one or several microfluidic devices as described above, and optionally
- a leaflet providing guidelines to use such a kit.

All embodiments described above for the methods and the microfluidic device of the invention are also encompassed in this aspect.

The present invention further relates to the use of a kit of the invention to label a plurality of molecular targets from a plurality of entities according to the method of the invention, or to quantify one or several molecular targets from a plurality of entities according to the method of the invention. All embodiments described above for the methods, the microfluidic device and the kit of the invention are also encompassed in this aspect.

As used herein, the verb "to comprise" is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there be one and only one of the elements. The indefinite article "a" or "an" thus usually means "at least one".

As used herein, the term "about" refers to a range of values ± 10% of the specified value. For example, "about 20" includes ± 10 % of 20, or from 18 to 22. Preferably, the term "about" refers to a range of values ± 5 % of the specified value.

### Examples

### I. Microfluidics chips fabrication and operation

In all the examples presented below microfluidic chips were fabricated using the same procedure and they were manipulated using on the same workstation.

### a. Microfluidic chips preparation and operation

Microfluidic devices were obtained using a classic replica molding process as described previously in (Mazutis et al., 2009). Briefly, devices were designed on Autocad (Autodesk 2014), negative photomasks were printed (Selba S.A.) and used to prepare molds by standard photolithography methods. SU8-2010 and SU8-2025 photoresist (MicroChem Corp.) were used to pattern 10 to 40 µm deep channels onto silicon wafers (Siltronix). Microfluidic devices were then fabricated in polydimethylsiloxane (PDMS, Silgard 184, Dow-Corning) using conventional soft lithography methods (Xia and Whitesides, 1998). Upon plasma bonding to a glass slide, channels were passivated with a solution of 1% (v/v) 1H, 1H, 2H, 2H-perfluorodecyltrichlorosilane (97%, ABCR GmbH and Co,) in HFE7500 (3M) and subsequently flushed with compressed air. Key dimensions and depth of microfluidic devices are given on concerned figures and in their captions.

Aqueous phases were loaded in I.D. 0.75 mm PTFE tubings (Thermo Scientifc) and oils were loaded in 2 mL Micrew Tubes (Thermo Scientific). Liquids were injected into microfluidic devices at constant and highly controlled flow-rates using a 7-bar MFCS™ pressure-driven flow controller (Fluigent) equipped with Flowells (7 µL/min flow-meters) allowing for operation in flow-rate controlled mode.

### b. Optical set-up, data acquisition and control system

The optical setup was based on an inverted microscope (Nikon Eclipse Ti-S) mounted on a vibration-dampening platform (Thorlabs B75150AE). The beams of a 488 nm laser (CrystaLaser DL488-050-O) and a 561 nm laser (Cobolt DPL 561-NM-100MW) were combined using a dichroic mirror (Semrock 2F495-DI03-2536). They were further combined with a 375 nm laser (CrystaLaser DL375-020-O) using a second dichroic mirror (Semrock Di02-R405-25x36) prior to shaping the resulting combined beam as lines using a pair of lenses (Semrock LJ1878L2-A and LJ1567L1-A) that was directed into the microscope objective (Nikon Super Plan Fluor 20x ELWD or Nikon Super Plan Fluor 40x ELWD) to be focused in the middle of the channel at the detection point. The emitted fluorescence was collected by the same objective and separated from the laser beams by a multi-edges dichroic mirror (Semrock Di01-R405/488/561/635-25x36). Blue (7-aminocoumarin-4-methanesulfonic acid) fluorescence was resolved from green (Syto9, FAM) and orange (Texas-Red) fluorescence by a third dichroic mirror (Semrock LM01-480-25). Then green fluorescence was separated from orange fluorescence by an additional dichroic mirror (Semrock FF562-Di03-25x36). Fluorescence was finally measured by three photomultiplier tubes (Hamamatsu H10722-20) equipped with bandpass filters (Semrock FF01-445/45-25, FF01-600/37-25 and FF03-525/50-25 for blue, green and orange detection respectively). Signal acquisition from the PMTs was performed using an intelligent data acquisition (DAQ) module featuring a user-programmable FPGA chip (National Instruments PCI-7851R) driven by internally developed firmware and software. To monitor the experiment, we used an additional dichroic mirror (Semrock FF665-Di02-25x36) to split light to a CCD camera (Allied Vision Technologies Guppy F-033). A long-pass filter (Semrock BLP01-664R-25) prevented potentially damaging reflections of the lasers into the camera.

### II. Sequences used in the examples

**Table 1: Sequences used in the examples**

| Molecule | SEQ ID n° | 5'- Sequence - 3' |
|---|---|---|
| Template UEI-DraIII | **1** | |
| UEI-Fwd | **2** | GAGCGGATAACAATTTCACACAGG |
| UEI-Rev | **3** | ACACGACGCTCTTCCGATC |
| Template Calibrator-N15-AlwNI | **4** | |
| Calib-Rev | **5** | GCCAGGGTTTTCCCAGTCACGAC |
| Illu-2-Fwd | **6** | TCGTCGGCAGCGTCAGATG |
| Template UEI-N15-DraIII* | **7** | |
| Template Calibrator-N15-AlwNI-1g | **8** | |
| Illul rev UEI | **9** | |
| Illul | **10** | GTCTCGTGGGCTCGGAGAT |
| Template-AlwNI-N 15-Calib-DraIII | **11** | |
| Calib-Fwd | **12** | GGAAGACGTAGCAAGGGAGTAGCC |
| RTmimicks | **13** | |
| antiSBACA- AlwN1 | **14** | GCAGCCAGCTGGCCTCGCTTCCGGGATCCGCGCAGACA |
| Template UEI-N15-DraIII-IIIu1 | **15** | |
| RNA III | **16** | |
| RT-UMI-RNAIII* | **18** | |
| SB-alone | **20** | CTGCGCGGATCCCGGAAGCGAGGCCAGCTGGCTGC |
| RNAIII-Fwd | **21** | |
| Gfp mRNA | **22** | |
| | | |
| gfp-mut2-RT-UMI-AlwNI* | **23** | |
| Gfp-Fwd-25nt | **24** | TGAA TT AGA TGGTGA TGTT AA TGGGC |
| Template-UEI-DraIII-N2x5 | **25** | |
| Template-UEI-DraIII-N4443 | **26** | |
| Template-Calibrator-AlwNI | **27** | |
| Template-Calibrator-AlwNI-N2x5 | **28** | |
| Template-Calibrator-AlwNI-N4443 | **29** | |
| Template-Calibrator-AlwNI-N15 | **30** | |
| Tag-NaBAb-AlwNI | **31** | |
| BG-M13-Fwd** | **32** | [BG]-isp18-GGGTTTTCCCAGTCACGACG* |
| Alexa488-M13-Rev | **33** | [Alexa488]-CACACAGGAAACAGCTATGACC |

| | | |
|---|---|---|
| All the oligonucleotides (but 15 obtained by primer extension, 16 and 22) were purchased from Integrated DNA Technologies (IDT). Random sequences corresponding to the UCI or the Calibrator are represented by N arrays and are underlined whereas the UMI is represented by an italicized N arrays. The sequence digested by AlwNI (CAGNNNCTG) and DraIII (CACNNNGTG) are shown in bold. * These oligonucleotides are 5' phosphorylated to allow their ligation with another DNA by T4 DNA ligase. ** [BG]: benzyl-guanine; ispl8: 18 carbon-long internal spacer. | | |

### Example 1: Cells preparation, individualization and lysis

In this example, we demonstrated that bacterial cells can be individualized in droplets and efficiently lysed using a detergent. *E. coli* bacteria (T7-Xpress Lys/I^{q}, GFP null strain New England Biolabs) transformed either with a plasmid carrying GFP gene under the control of T7 RNA polymerase promoter or a plasmid bearing an unrelated construct were used as model bacteria and will be later summarized as strains GFP+ and GFP- respectively. Note that both plasmids confer ampicillin resistance to the bacteria allowing for their co-culture.

### a. Preparation of the cell

A cell pre-culture (starter) was first obtained by inoculating a 2YT media supplemented with 0.1 mg/mL ampicillin and 2 % glucose, and incubating it over-night at 37°C and under agitation. 500 µL of this starter (OD₆₀₀ of 3.77) were then used to inoculate 20 mL of the same medium, and the bacteria were allowed to grow until the culture reached an OD₆₀₀ of 0.6. Next, the cells were fluorescently stained to allow for monitoring cells during the microfluidic steps. To do so, 2 mL of this culture were stained using 5 µM SYTO 9 (molecular probes by Life technologies, ref 34854) for 2 hours in the dark, following supplier recommendations. Cells were washed once with PBS buffer 1x before being pelleted for further experiments.

### b. Cells encapsulation and lysis

### Experimental procedure

Pellets of labelled cells were diluted to reach an OD₆₀₀ of 3.75 (giving a droplet occupancy of ∼ 20%) in a CutSmart® buffer 1x (50 mM Potassium acetate, 20 mM Tris acetate pH7.9 at 25°C, 10 mM Magnesium acetate and 0.1 mg/mL BSA, New England Biolabs) supplemented with 37.5 µg/mL dextran Texas red 70 000 MW (used as droplet tracker), and 0.05 % Pluronic F68. The mixture was then loaded into a 0.5 mL PCR tube containing a magnetic stirring bar (5 mm length and 2 mm diameter) closed by a plug of PDMS. One extremity of the system was connected to a Fluigent infusion device whereas the other extremity was connected to the droplet co-flow generator **(****Figure 11****, inlet 1).** During droplet production, the mixture was stirred by placing a stirring plate (Hei-Mix S, Heidolph instrument) aside the bacteria-containing tube to keep bacteria properly suspended. A solution of CutSmart® buffer 1x supplemented with B-PER™ lysis reagent (90% final concentration Thermo Scientific) was loaded into a length of PTFE tubing (I.D. 0.75 mm tubing; Thermo Scientifc) and connected to the Fluigent infusion device at one side of the tubing and to the droplet co-flow generator **(****Figure 11****, inlet 2)** at the other side of the tubing. Both aqueous solutions (bacteria suspension and lysis solution) were combined on-chip prior to being dispersed into a stream of Novec 7500 (3M) fluorinated oil supplemented with 3 % Krytox-Jeffamine 1000 diblock copolymer fluorosurfactant (Ryckelynck *et al* 2015) that was infused into the third inlet **(****Figure 11****, inlet 3).** 4 pL droplets were then produced at a rate of 800 droplets per second by infusing both aqueous phases at 200 nL/min and the oil phase at 1000 nL/min. Droplets were collected at the outlet of the chip **(****Figure 11****, outlet 4)** *via* a length of tubing into a 0.2 mL PCR tube closed by a plug of PDMS. Moreover, an identical experiment in which the lysis solution was exchanged for a lysis agent-free CutSmart® buffer 1x phase was used for a control experiment. Upon an incubation of 20 min at 25°C, droplet fluorescence was analyzed on-line by re-injecting the droplets into a droplet fluorescence analysis microfluidic device. Droplets contained into a 0.2 mL PCR tube closed by a plug of PDMS were reinjected into a droplet analyzer **(****Figure 12****, inlet 1)** where they were spaced by an oil stream **(****Figure 12****, inlet 2)** and their fluorescence content was analyzed by the optical set-up introduced above. The Texas Red contained into each droplet allows identifying a droplet as an orange peaks **(****Figure 13****).** Moreover, the staining of cell nucleic acids by Syto9 enables both detecting bacteria and appreciating their lysed/integer status. Indeed, the presence of an integer cell is indicated by a spike of green fluorescence into the droplet (orange peak; **Figure 13****,** top panel), whereas a lysed cell gives a more homogeneous labelling of lower intensity (e.g. second droplet from the left on **Figure 13****,** bottom panel). To further confirm this result, 10 µL of the droplets were loaded into a plastic Malassez hematimeter and imaged on an epi-fluorescence microscope both in bright-field and green fluorescence (ex/em = 470 nm/525 nm) mode **(****Figure 14****).** The green labelling of cell nucleic acids by Syto 9 allows discriminating intact bacteria (green rod shapes on **Figure 14****,** left panel) from lysed cells (homogenous green droplet staining on **Figure 14****,** right panel). This experiment showed that not only the presence of the lysis agent does not challenge droplets stability, but it also allows to efficiently lyse the cells and release their content into the droplets. Both approaches confirmed that cells can be isolated and lysed in droplets in conditions that do not compromise droplets stability. Moreover, the lysis conditions (i.e. CutSmat® buffer supplemented with B-PER™) are compatible with several molecular biology reactions such as reverse transcription, DNA digestion by restriction enzymes and DNA ligation. This example demonstrates the possibility of lysing cells into the droplets while preserving droplet integrity and being compatible with downstream molecular biology reactions.

### Example 2: Preparation of the Unique Identifier and droplet signatures

In this example, we show how Unique Identifiers (UIs) can be prepared both in bulk and in microfluidic droplets as well as how they can be used to generate a signature encoding droplet identity. A UI is made of a pair of random sequences (the UEI ("unique entity identifier") and the UEI Calibrator) surrounded by constant sequence regions encompassing restriction sites generating compatible extremities later used to recombine both sequences together **(****Figure 15****).** To form a UI, both UEI-Calibrator and UEI sequences should be first amplified in a duplex PCR prior to being recombined together through a specific restriction/ligation reaction. Then, the pool of UI contained into the same droplet constitute the signature of the droplet that can later be used to reassign a given encoded molecule to the droplet it originates from.

### a. Duplex PCR co-amplification of the two barcode sets

We first validated the possibility of performing a duplex PCR co-amplification of UEI-Calibrator and UEI sequences in tubes prior to transferring it in droplet microfluidic format.

### Experimental procedure

500 atto moles of one or both templates (i.e. UEI-DraIII (**1**) and AlwNI-Calibrator-DraIII (4)) diluted into 0.2 mg/mL yeast total RNA (Ambion) were introduced into a reaction mixture containing 0.2 µM of each forward (molecules **2** and **6**) and reverse (molecules **3** and **5**) primers, 0.2 mM of each dNTP, 0.1 % Pluronic F68, 0.67 mg/mL dextran Texas Red (Invitrogen), 6 nM of a Taq polymerase produced in the laboratory and 1x CutSmart® buffer (50 mM Potassium acetate, 20 mM Tris acetate pH7.9 at 25°C, 10 mM Magnesium acetate and 0.1 mg/mL BSA) in a final volume of 50 µL. The mixture was then thermocycled with an initial denaturation step of 30 sec at 95°C followed by 25 cycles of 5 sec at 95°C and 30 sec at 65°C.

### Results

Upon amplification, PCR products with the expected size (62 base pairs for UEI-DraIII and 142 base pairs for AlwNI-Calibrator-DraIII) were readily observed on 8% polyacrylamide native gel. Whereas, a single band was observed when only one of the template was present **(****Figure 16****, lane 1** and **2),** two distinguished bands were seen when both templates were mixed together **(****Figure 16****, lane 3),** confirming that both templates can be efficiently co-amplified. In this section only the Calibrators carried a randomized region, which explains the slightly smeary aspect of the band corresponding to the Calibrator. This smear was attributed to the formation of heteroduplexes between the strands of two different Calibrators sharing the same constant regions but having different randomized ones.

### b. Amplification of the PCR in water-in-oil droplet

We next verified that the co-amplification can efficiently occur in droplets.

### Experimental procedure

14 atto moles (a concentration allowing for having a theoretical average of 4 template DNA molecules per droplet) of each template (i.e. Template UEI-N15-DraIII **(7)** and Template AlwNI-N15-Calibrator-DraIII (**8**)) diluted into 0.2 mg/mL yeast total RNA (Ambion) were introduced in 200 µL of reaction mixture containing 0.2 µM of each forward (molecules **2** and **6**) and reverse (molecules **3** and **5**) primers, 0.2 mM of each dNTP, 0.1 % Pluronic F68, 0.67 mg/mL dextran Texas Red (Invitrogen), 6 nM of a Taq polymerase produced in the laboratory and 1x CutSmart® buffer (50 mM Potassium acetate, 20 mM Tris acetate pH7.9 at 25°C, 10 mM Magnesium acetate and 0.1 mg/mL BSA. The mixture was then loaded into a length of PTFE tubing (I.D. 0.75 mm tubing; Thermo Scientifc) and connected to the Fluigent infusion device at one side of the tubing while the other the other side was connected to a 40 µm deep droplet generator **(****Figure 17****, inlet 1).** An oil phase made of Novec 7500 supplemented with 3 % Krytox-Jeffamine 1000 diblock copolymer fluorosurfactant (Ryckelynck *et al* 2015) was also infused into the chip **(****Figure 17****, inlet 2)** and used to produce 100 pL droplets at a rate of 1600 droplets per second by infusing oil and aqueous phases at 1500 nL/min and 1550 nL/min respectively. Droplets were collected for 11 min *via* a length of tubing **(****Figure 17****, outlet 3)** into a 0.2 mL PCR tube closed by a plug of PDMS. The tube was then placed in a thermocycler and the emulsion was subjected to an initial denaturation step of 30 sec at 95°C followed by 25 cycles of 5 sec at 95°C and 30 sec at 65°C. Upon thermocycling, the emulsion was broken using 50 µL of 1H, 1H, 2H, 2H, perfluoro-1-octanol (Sigma-Aldrich) and 5 µL were loaded on an 8% native PAGE.

### Results

Gel analysis confirmed that the duplex PCR amplification properly worked in water-in-oil droplets **(****Figure 16****, lane 5)** and with the same apparent efficiency than in bulk **(****Figure 16****, lane 4).** Moreover, the analysis revealed that compartmentalizing the reaction into droplets allowed obtaining better resolved bands, likely by limiting recombination, the formation of heteroduplexes and other PCR artefacts linked to the presence of the randomized regions on both barcodes (UEI-Calibrators and UEIs).

### c. UI-based signature

Forming the UI requires recombining together the UEI-Calibrator and the UEI through a restriction/ligation coupled reaction. To do so, we choose two non-palindromic restriction sites producing compatible 3' overhangs. AlwNI (5'-CAGNNN/CTG-3') and DraIII (5'-CACNNN/GTG-3') are two enzymes digesting sequences (bolded in **Table 1**) fulfilling these criteria. Digestion at these sites generates two compatible sequences that can recombine together and form new sequences (i.e. 5'-CAGNNNGTG-3' and 5'-CACNNNCTG-3') that can no longer be digested by the enzymes, pulling therefore the equilibrium toward the formation of recombined molecules.

### Experimental procedure

The duplex PCR established in **section b** was repeated using template molecules **7** and **8** and amplification primers **3, 5** and **6.** However, primer **2** was exchanged for primer **9** that allowed introducing Illu-1 sequence, an adaptor sequence later used for next generation sequencing analysis. To evaluate to what extend the relative amount of both barcodes can affect recombination efficiency, so UI formation, we performed a set of experiments using two relative concentrations of UEI-Calibrators and UEI in the droplets. This ratio was changed by varying the concentration of the corresponding primers in the PCR mixture. However, the diversity of the barcodes was preserved by initiating each experiment with the same average number of template per PCR droplet. Therefore, in a first reaction set 14 atto moles of each template diluted into 0.2 mg/mL yeast total RNA (Ambion) were mixed with 0.2 µM of each primer (**3, 5, 6** and **10**) giving a final ratio UEI-Calibrator/UEI of 1/1. In a second set of reactions, 14 atto moles of each template diluted into 0.2 mg/mL yeast total RNA (Ambion) were mixed with 0.2 µM of primers **3** and **10** and 0.02 µM of primers **5** and **6,** giving a final ratio UEI-Calibrator/UEI of 0.1/1. Each template/primers mixture was then introduced into 200 µL of reaction mixture containing 0.2 mM of each dNTP, 0.1 % Pluronic F68, 0.67 mg/mL dextran Texas Red (Invitrogen), 6 nM of a Taq polymerase produced in the laboratory and 1x CutSmart® buffer (50 mM Potassium acetate, 20 mM Tris acetate pH7.9 at 25°C, 10 mM Magnesium acetate and 0.1 mg/mL BSA). The mixture was dispersed into 100 pL droplets as above and the emulsion was collected for 20 min in a 0.2 mL tube closed by a plug of PDMS and thermocycled as before. Upon thermocycling, the emulsion was reinjected into a droplet picoinjector **(****Figure 18****, inlet 1)** at 500 nL/min and the droplets were spaced by a stream of Novec 7500 oil supplemented with 2 % Krytox-Jeffamine 1000 diblock copolymer fluorosurfactant infused at 1600 nL/min through a second inlet **(****Figure 18****, inlet 2).** A mixture of CutSmart™buffer 1x (50 mM Potassium acetate, 20 mM Tris acetate pH7.9 at 25°C, 10 mM Magnesium acetate and 0.1 mg/mL BSA), supplemented with 1 mM rATP, 7 mM DTT, 3 U/µL DraIII HF (New England Biolabs), 3 U/µL AlwN1 (New England Biolabs), 30 U/µL T4 DNA ligase (New England Biolabs), 20 µM coumarin acetic (used as injection tracker) and 0.1% pluronic F68 was prepared. The mixture was loaded into a length of PTFE tubing (I.D. 0.75 mm tubing; Thermo Scientifc) and connected to the Fluigent infusion device at one side of the tubing and to the third inlet of the chip **(****Figure 18****, inlet 3)** at the other side. Then, 25 pL of the enzyme mixture were delivered to each 100 pL droplet by infusing the "enzyme" phase at 150 nL/min while energizing the electrodes facing the injection point with a squared AC field (400 V, 30 Hz) obtained by a function generator connected to an high voltage amplifier (TREK Model 623B). Droplets were collected **(****Figure 18****, outlet 4)** in a 0.5 mL tube under mineral oil for 45 minutes prior to being incubated overnight at 37°C to allow digestion and ligation to occur. In parallel, an identical reaction in which enzymes were omitted was performed as a control. Upon incubation, the emulsion was broken with 50 µL of 1H, 1H, 2H, 2H, perfluoro-1-octanol (Sigma-Aldrich) and the labelling efficiency was assessed by quantitative PCR with the kit SsoFast Evagreen Supermix (Bio-Rad) supplemented with primers **6** and **10.** The mixture was thermocycled in a CFX qPCR machine (Bio-Rad) with an initial denaturation step of 30 sec at 95°C followed by 40 cycles of 5 sec at 95°C and 30 sec at 60°C. At the end of the process, the Ct value was determined for each condition **(****Figure 19****, Top and middle panels)** and the quality of the amplified material was verified by loading an aliquot of each qPCR reaction onto a native polyacrylamide gel **(****Figure 19****, bottom left panel).** After having verified the quality of the DNA, ∼ 1000 droplets from the emulsion containing the 1/1 UEI-Calibrator/UEI ratio were transferred in a new tube where they were broken with 50 µL of 1H, 1H, 2H, 2H, perfluoro-1-octanol (Sigma-Aldrich) and the released DNA was recovered in 1x CutSmart™ buffer. The DNA was then indexed with primers N503 and N702 using Nextera index kit (ref FC-121-1011, Illumina) following supplier specifications. To limit the risk of unwanted mutations during the indexing step, the reaction was performed using Phusion DNA polymerase (ThermoScientific). The band containing indexed DNA was then purified on a 1% agarose gel electrophoresis and the DNA recovered with the kit Wizard SV Gel and PCR clean up system (Promega) **(****Figure 19****, right panel).** Last, the resulting library was loaded onto an Illumina V2-300 cycles flow-cell and the DNA was sequenced on a MiSeq device (Illumina).

Finally, the sequences were analyzed using a Python-based bioinformatics algorithm **(****Figure 20****).** The algorithm works in 3 main steps. First, raw sequences obtained from the sequencer are quality-filtered and those with a Q score below 30 are removed from the pool. Moreover, sequences presenting mutations in the non-randomized region or showing an inappropriate length are also removed from the pool. Second, UEI-Calibrator and UEI sequences are extracted and pairs coming from the same droplet are clustered together. Briefly, all the UEIs associated with a given UEI-Calibrator are clustered together. Then, all the UEI-calibrator sharing the UEIs contained into the same cluster are also clustered together. At the end of the process, clusters of UIs (pairs of UEI-Calibrators and UEIs) are obtained and form a signature of the droplet. Finally, in a third step, the signatures can be used to reassign each molecule from a pool to the droplet it originates from.

### Results

The UI signature readily forms only in the presence of restriction/ligation enzymes. Indeed, the qPCR analysis revealed that whatever the UEI-Calibrator/UEI ratio used, more than 10 additional amplification cycles (delta Ct > 10) are required the reach the threshold in the absence of recombination enzymes (**Figure 19****, Top panel,** compare columns - and + enzymes), indicating that there is at least a thousand times less recombined material in the absence of enzymes. Moreover, electrophoresis gel analysis (**Figure 19****, left panel**) showed that specific band of the expected size is obtained only in the presence of the enzymes (lanes 2, 4 and 7). Therefore, UI formation is highly controlled as it occurs only in the presence of specific enzymes and does not form spontaneously form during the PCR reaction, which limits the risk of forming UI in non-specific way. Not only this recombination was found to work in tubes (**Figure 19****, left panel,** lanes 1 to 4) but it also works in droplets **(****Figure 19****, left panel,** lanes 5 to 7) starting from PCR amplification products also prepared in droplets. As in tubes, the presence of UI in droplets requires the presence of the specific restriction/ligation enzymes (compare lanes 6 and 7).

Further analyzing the droplet-contained UI by Next Generation Sequencing together with the bioinformatics algorithm shown on **Figure 20****,** allowed us to reassign each UEI and each UEI-Calibrator to the droplet it originates from (**Figure 21**). Indeed, the use of a barcode made of 15 randomized (theoretical diversity of 10⁹ different variants) region makes unlikely to find the same UEI or the same UEI-Calibrator in more than one droplet in the emulsion (made at most of a few millions of droplets), making this reassignment faithful. The number of different UEI and UEI-Calibrator per droplet followed a Poisson distribution (**Figure 21****, left**; see also **Figure 23**) as expected for objects randomly distributed into compartments such as droplets. This confirms that the proper confinement of the information was preserved all along the process and that the UI were generated in the droplets and not after droplet breaking. Note that in this example, the average number of different UEI-Calibrator per droplet was only 1 while 4 were expected. However, complementary experiments presented hereafter (section d) allowed readjusting this parameter and further confirming the Poisson-driven behavior of the molecules.

Clustering the different UIs contained in each droplet allows obtaining a signature unique to each droplet. Interestingly, the signatures were evenly represented within the pool of sequences **(****Figure 21****, right),** indicating that no significant droplet-to-droplet bias occurs during UI preparation.

### d. Adjusting UEI diversity

To further confirm that both UEI-Calibrators and UEIs distributes into the droplets following a Poisson distribution, as well as to properly adjust the content of each type of barcodes, the experiment was performed at three different average number (lambda values) of barcode per droplet.

### Experimental procedure

As before, a duplex PCR was performed but using different starting amount of template molecules **7** and **8.** To test the lambda 1.3 condition (i.e. an average of 1.3 UEI-Calibrator and 1.3 UEI per droplet), 5 atto moles of each template (i.e. Template UEI-N15-DraIII **(7)** and Template AlwNI-N15-Calibrator-DraIII (**8**)) diluted into 0.2 mg/mL yeast total RNA (Ambion) were introduced into 200 µL of a reaction mixture containing 0.2 µM of each forward (molecules **2** and **6**) and reverse (molecules **3** and **5**) primers, 0.2 mM of each dNTP, 0.1 % Pluronic F68, 0.67 mg/mL dextran Texas Red (Invitrogen), 6 nM of a Taq polymerase produced in the laboratory and 1x CutSmart® buffer (50 mM Potassium acetate, 20 mM Tris acetate pH7.9 at 25°C, 10 mM Magnesium acetate and 0.1 mg/mL BSA. To test the lambda 4 (i.e. an average of 4 different UEI-Calibrators and 4 different UEIs per droplet) and lambda 12 (i.e. an average of 12 different UEI-Calibrators and 12 different UEIs per droplet) conditions, the starting amount of each template introduced in the reaction mixture was respectively raised to 14 atto moles and 56 atto moles. 100 pL droplets were generated at a frequency of 300 droplets per second using the same chip as before **(****Figure 17****)** and the emulsion was collected and thermocycled as in **section c.** Finally, as above, an enzyme mixture was picoinjected into each droplet and the emulsion was incubated to allow the recombination to occur. Moreover, a control reaction in which restriction/ligation enzymes were omitted was performed in parallel with the lambda 1.3 condition. Upon incubation, the proper recombination was again verified by qPCR (using primers **6** and **10**) and gel electrophoresis **(****Figure 22****).** Moreover, for each condition, ∼ 1000 droplets were transferred into a new tube where they were broken by adding 20 µL of 1H, 1H, 2H, 2H, perfluoro-1-octanol (Sigma-Aldrich) and the released DNA was recovered in 200 µL of PCR mixture containing Q5 DNA polymerase (New England Biolabs) and its buffer at the recommended concentration, 0.2 mM of each dNTP and a Nextera primer pair (N703 and N502 to index the lambda 1.3 condition; N705 and N504 to index the lambda 4 condition; N706 and N517 to index the lambda 12 condition; Illumina) at the recommended concentration. Indexing was performed by thermocycling the mixture using the program recommended by the manufacturer and the amplification product was loaded on a 1% agarose gel). Upon electrophoresis, the band containing the indexed DNA was sliced from the gel and the DNA was recovered with the kit Wizard SV Gel and PCR clean up system (Promega). Finally, the resulting library was loaded into an Illumina V2-300 cycles flow-cell and a high-throughput sequencing was performed on a MiSeq device (Illumina). Then, sequencing data were analyzed using our bioinformatics algorithm **(****Figure 20****)** and the distribution profiles of both UEI-Calibrators and UEIs were established for each condition **(****Figure 23****).**

### Results

Preparing UIs in droplets while varying the initial number of UEI-Calibrator and UEI template per droplet (lambda value) did not significantly challenged the process *per se.* Indeed, whatever the starting lambda value (from 1.3 to 12), very close Ct values were obtained when quantifying the amount of DNA amplified and recombined into the droplets at the of the process (reactions 2 to 3 on **Figure 22**). With respect to the enzyme-free control (reaction 1 on **Figure 22**), up to 20 less amplification cycles were required to reach the threshold when enzymes were added, indicating that there was up to a million times more UI formed in the presence of enzymes. Analysis on gel, confirmed that the proper reaction product (indicated by an arrow on **Figure 22**) was observed only in the presence of the enzyme (lanes 2 to 4), whereas only a small parasitic amplification product was obtained in the absence of enzymes (lane 1).

Analyzing the sequence content of an aliquot of 1000 droplets from each condition with our bioinformatics algorithm revealed that, as expected, changing the starting amount of UEI-Calibrator and UEI template per droplet directly impacts the distribution of both barcodes in the droplets **(****Figure 23****)** and that the higher the starting number of different UEI-Calibrator and UEI per droplet the higher the number of different UI forming droplet signature. Therefore, adjusting the initial concentration of both templates allows adjusting the diversity of UI per droplet (so the complexity of the signature) in a predictable way.

### Example 3: Labelling and quantifying DNA using UI/signature

Since UIs are obtained through a digestion/ligation process, they can also be appended to another DNA molecule provided it possesses a site compatible with one of the restriction products. This DNA could be either a reverse transcription product or a DNA attached to another molecule such as a protein. In this example, we demonstrate the possibility of attaching UI to another DNA and to later use our approach to reassign each DNA molecule to the droplet it originates from as well as quantifying the DNA content of each droplet. Therefore, in this example we aimed to: i) isolate and PCR co-amplify UEI-Calibrator and UEI templates; ii) supplement each UEI-Calibrator/UEI-containing droplet with a DNA displaying an extremity compatible with one of the restriction products; iii) add restriction/ligation enzymes to each droplet to form UIs through recombination and attach them at the extremity of the target DNA molecule; iv) index and sequence the library in high throughput regimes. Furthermore, a random sequence of 8 nucleotides forming a Unique Molecular Identifier (UMI) was inserted into the target DNA to allow the later counting of the molecules contained in each droplet.

### a. UEI formation and DNA labelling in tubes

Prior to performing DNA labelling in droplets, we first verified the whole reaction process in tubes. In this example, we used a partially double stranded DNA mimicking a reverse transcription product **(****Figure 24****).** This molecule is made of a long molecule **(13)** whom the 3' part is kept single stranded and bears a randomized region of 8 nucleotides (UMI) at its 5' part followed by a constant region forming a double strand by pairing with the molecule **14.** This pairing generates a 3' overhang compatible with the DraIII cleavage product of the UEI-Calibrator moiety of the UIs. Moreover, the 5' phosphorylated end of **13** makes it substrate of DNA ligases.

### Experimental procedure

UI components (UEI-Calibrators and UEIs) were first generated as in Example 2 by a duplex PCR co-amplification. To do so, 500 atto moles of template DNAs **7** and **11** were first PCR amplified in a volume of 200 µL as described in **Example 2 section a** using 0.2 µM of primers **2, 3, 5** and **12.** Target DNA was prepared by annealing together 1.25 pmol of the molecule **13** and 1.25 pmol of the molecule **14** in 20 µL of 1x CutSmart® buffer, 0.25 mM dNTP, 10 mM DTT, 0.1 µM FAM, 0.15 mg/mL dextran Texas Red. The enzyme mixture was prepared by supplementing a 1x CutSmart® buffer with 6 mM rATP, 60 mM DTT, 8 U/µL DraIII HF, 8 U/µL AlwN1, 80 U/µL T4 DNA ligase, 55 µM coumarin acetic and 0.3% Pluronic F68. 10 µL of duplex PCR were then mixed with 4 µL of target DNA solution and 3 µL of Enzyme mixture and the resulting mixture was incubated overnight at 37°C. As a control, the same reaction was performed but restriction enzymes and the ligase were omitted from the enzyme mixture. Upon incubation, an aliquot of each reaction was analyzed by quantitative PCR using the kit Sso-Fast Evagreen (Bio-Rad) supplemented in primers **3** and **6.** After the qPCR, an aliquot of each reaction was analyzed on polyacrylamide gel electrophoresis.

### Results

The annealing of molecule **13** and **14** form a target DNA displaying a 3' overhang compatible with the DraIII product generated upon UEI-Calibrator digestion. Mixing together this target DNA with co-amplified UEI-Calibrators and UEIs as well as with restriction and ligation enzymes allows the formation of UIs and their attachment to the target DNA, therefore directly encoding it. qPCR analysis indicates that more than 17 additional amplification cycles (delta Ct > 17) are required the reach the threshold in the absence of recombination enzymes (**Figure 25****,** compare columns - and + enzymes), indicating that there is at least a 160,000-times less UI-encoded DNA molecules in the absence of enzymes. Moreover, gel electrophoresis analysis (**Figure 25****, right panel**) showed that specific band of the expected size is obtained only in the presence of the enzymes (lane 2).

Therefore, UIs can be used to encode a DNA displaying an overhang compatible with one of the restriction site present in the UI.

### b. UI formation and DNA labelling in droplet

We next transposed the DNA labelling into the droplet microfluidic format. As in Example 2, UEI-Calibrators and UEIs were co-amplified in droplets using a duplex PCR prior to adding the DNA to label and the enzyme mixture to each droplet.

### Experimental procedure

An extended version of the molecule 7 encompassing the sequencing adapter Illu1 was first prepared by primer extension. Briefly, 10 pmols of the template 7 were mixed with 50 pmols of primer **9** in a 50 µL reaction mixture containing 0.2 mM of each dNTP, 1U of Phusion DNA polymerase (ThermoScientific) and the corresponding buffer at the recommended working concentration. The mixture was then incubated for 5 minutes at 98°C and 10 minutes at 72°C. Extension product was then purified on an agarose gel and the DNA recovered with the Wizard SV Gel and PCR clean up system (Promega). The extended molecule is now called Template UEI-N15-DraIII-Illu1 and corresponds to molecule **15.** 14 atto moles (a concentration allowing for having an average of 4 template DNA molecules per droplet) of each template (i.e. Template UEI-N15-DraIII-Illu1 (**15**) and Template-AlwNI-N15-Calib-DraIII (**11**)) diluted into 0.2 mg/mL yeast total RNA (Ambion) were introduced into a reaction mixture containing 0.2 µM of each forward (molecules **2** and **5**) and reverse (molecules **10** and **12**) primers, 0.2 mM of each dNTP, 0.1 % Pluronic F68, 0.67 mg/mL dextran Texas Red (Invitrogen), 6 nM of a Taq polymerase produced in the laboratory and 1x CutSmart® buffer (50 mM Potassium acetate, 20 mM Tris acetate pH7.9 at 25°C, 10 mM Magnesium acetate and 0.1 mg/mL BSA) in a final volume of 200 µL. The mixture was then loaded into a length of PTFE tubing (I.D. 0.75 mm tubing; Thermo Scientifc) and connected to the Fluigent infusion device at one side of the tubing while the other the other side was connected to a 40 µm deep droplet generator **(****Figure 17****, inlet 1).** An oil phase made of Novec 7500 supplemented with 3 % Krytox-Jeffamine 1000 diblock copolymer fluorosurfactant (Ryckelynck *et al* 2015) was also infused into the chip **(****Figure 17****, inlet 2)** and used to produce 100 pL droplets at a rate of 1600 droplets per second by infusing oil and aqueous phases at 1500 nL/min and 1550 nL/min respectively. Droplets were collected **(****Figure 17****, outlet 3)** for 11 min *via* a length of tubing into a 0.2 mL PCR tube closed by a plug of PDMS. The tube was then closed, placed into a thermocycler and the emulsion was subjected to an initial denaturation step of 30 sec at 95°C followed by 25 cycles of 5 sec at 95°C and 30 sec at 65°C. Target DNA was prepared by mixing and annealing together 33 femto moles of oligonucleotide **13** (corresponding to 1000 DNA molecule per 20 pL) and 66 femto moles of oligonucleotide **14** in 400 µL of solution containing a 1x CutSmart® buffer, 0.25 mM dNTP, 10 mM DTT, 0.1 µM FAM (droplet tracker fluorescent dye), 0.15 mg/mL dextran Texas red, 0.005 % Pluronic F68. The mixture was then loaded into a length of PTFE tubing (I.D. 0.75 mm tubing; Thermo Scientifc) and connected to the Fluigent infusion device at one side of the tubing while the other the other side was connected to a 15 µm deep droplet generator **(****Figure 17****, inlet 1).** An oil phase made of Novec 7500 supplemented with 3 % Krytox-Jeffamine 1000 diblock copolymer fluorosurfactant was also infused into the chip **(****Figure 17****, inlet 2)** and used to produce 20 pL droplets at a rate of 1600 droplets per second by infusing oil and aqueous phases at 1200 nL/min and 900 nL/min respectively. Droplets were collected **(****Figure 17****, outlet 3)** into a 0.2 mL PCR tube closed by a plug of PDMS for *via* a length of tubing. The enzyme mixture was prepared by mixing together 9 mM rATP, 30 mM DTT, 3 U/µL DraIII HF (New England Biolabs), 3 U/µL AlwN1 (New England Biolabs), 15 U/µL T4 DNA ligase (New England Biolabs), 20 µM coumarin acetic (injection tracker) and 0.1% pluronic F68 in a 1x CutSmart® buffer. The enzyme mixture was loaded into a length of PTFE tubing (I.D. 0.75 mm tubing; Thermo Scientifc) and connected to the chip **(****Figure 27****, inlet 5).** Both emulsions (UEI-Calibrator/UEI-containing 100 pL droplets and target DNA-containing 20 pL droplets) were combined, were fused and supplemented with the enzyme mixture on a Barcoding microfluidic device **(****Figure 27****).** UEI-Calibrator/UEI-containing 100 pL droplets were reinjected **(****Figure 27****, inlet 1)** at a frequency of ∼ 100 droplets per second by infusing the emulsion at 400 nL/min while spacing them with a stream of oil (Novec 7500 supplemented with 2 % Krytox-Jeffamine 1000 diblock copolymer fluorosurfactant) infused into the chip **(****Figure 27****, inlet 2)** at a flow-rate of 1300 nL/min. Target DNA-containing 20 pL droplets were reinjected **(****Figure 27****, inlet 3)** at a frequency of ∼ 100 droplets per second by infusing the emulsion at 100 nL/min while spacing them with a stream of oil (Novec 7500 supplemented with 2 % Krytox-Jeffamine 1000 diblock copolymer fluorosurfactant) infused into the chip **(****Figure 27****, inlet 4)** at a flow-rate of 1200 nL/min. Pairs of droplets were allowed to form while the droplets were circulating into a short delay line. Pair-wised droplets were then fused when passing in front of an electrode pair to which a squared AC field (800 V, 30 Hz) was applied using a function generator connected to a high voltage amplifier (TREK Model 623B). At the same time, 25 pL of enzyme mixture was delivered to each droplet by injecting the enzyme mixture into the chip **(****Figure 27****, inlet 5)** and infusing it at 100 nL/min. Fused and picoinjected droplets were collected **(****Figure 27****, outlet 6)** for 20 minutes in a 0.5 mL tube under mineral oil. The emulsion was then incubated in a thermocycler and subjected to 15 repeats of the program: incubate for 15 min at 37°C and 45 min at 16°C. Upon incubation, 1000 droplets were transferred in a new tube where they were broken by adding 20 µL of 1H, 1H, 2H, 2H, perfluoro-1-octanol (Sigma-Aldrich) and the released DNA was recovered in 200 µL of PCR mixture containing Q5 DNA polymerase (New England Biolabs) and its buffer at the recommended concentration, 0.2 mM of each dNTP and Nextera primers N701 and N502 (Illumina) at the recommended concentration. Indexing was performed by thermocycling the mixture using the program recommended by the manufacturer and the amplification product was loaded on a 1% agarose gel **(****Figure 28****).** Upon electrophoresis, the band containing the indexed DNA was then recovered and the DNA isolated with the kit Wizard SV Gel and PCR clean up system (Promega). Finally, the resulting library was loaded onto an Illumina V2-300 cycles flow-cell and the DNA was sequenced on a MiSeq device (Illumina). Upon sequencing, data were analyzed using an up-graded version of our bioinformatics algorithm **(****Figure 29****).** Indeed, first, droplet signatures were established as previously (see **Example 2-section c** and **Figure 20**). Next, these signatures were used to cluster the QC-filtered sequences and reassign each cluster to the droplet it originates from. Then, all the sequences sharing the same UMI sequence (stretch of 8 randomized nucleotides carried by the target DNA) were collapsed into a single sequence. Finally, counting the number of different UMIs contained into each droplet gave access to the absolute quantification of the DNA content of each droplet **(****Figure** 30).

### Results

In this example, we prepared an emulsion made of 20 pL droplets, each containing on average ∼ 1000 target DNA molecules. In parallel, UEI-Calibrator/UEI-containing droplets were prepared as described in Example 2. Finally, using our Barcoding microfluidic device **(****Figure 27****)** each target DNA-containing droplet was fused to a single UEI-Calibrator/UEI-containing and received a controlled amount of restriction/ligation enzyme mixture. Upon sequencing analysis of the content of ∼ 1000 droplets we applied an up-graded version of our bioinformatics algorithm **(****Figure 29****)** and succeed in: i) identifying the different UIs contained in the sample and cluster them into droplet signatures and ii) use these signatures to reassign each sequence to the droplet it originates from and finally iii) count the target DNA copy number present into each droplet **(****Figure 30****).** The analysis led us to identify ∼ 1200 different droplets, a value approaching closely the 1000 expected droplets. Moreover, an average of ∼ 200 copies of the target DNA were found in each droplet, again a value closing approaching the theoretical droplet content (expected to be ∼ 1000 copies per droplet). DNA copy number distribution was very tight, indicating a high homogeneity of the barcoding process (i.e. formation and addition of UIs) among the different droplets. Each sequence-UMI-UI was found at least 10 times in the sequence pool, indicating that the library was analyzed with a 10-times coverage. This indicates that the tight distribution of the droplets content does results from the saturation of the methods and is rather representative of the actual content of the emulsion. Taken together, the results presented in this example show that our methodological and analytical algorithm allows for quantifying the DNA content of a droplet in reproducible way by attaching an UI to it.

### Example 4: Converting RNA into cDNA encodable by an UI

Since our methodology allows for labelling and counting the DNA molecule contained into the droplets of an emulsion, we next investigated if RNA can be converted, in the droplets, into a cDNA that could be used as a substrate of our labelling technology. To generate such cDNA, we designed composite reverse transcription (RT) primers made of i) double-stranded 5' part terminated by a 3' overhang compatible with the restriction site present at the extremity of the UI; followed by ii) a single-stranded region containing 8 random nucleotides and working as Unique Molecular Identifier (UMI) and iii) terminated by 3' single-stranded region annealing specifically to the target RNA **(****Figure 31****).** In this example, we show that such molecule can be used to prime reverse transcription of an RNA as well as attachment point for an UI.

### a. Reverse transcription of RNAs in droplets

To test the possibility of reverse transcribing RNAs in the droplets, we choose the highly structured (therefore challenging to reverse transcribe) RNA III from *Staphylococcus aureus* (Benito *et al*, 2000). The RNA was prepared by *in vitro* transcription using T7 RNA polymerase using the same procedure described in (Ryckelynck *et al*, 2015). Upon transcription, RNA was purified on a size exclusion column Nap5 (GE-Healthcare) and quantified using a Nanodrop device. To prevent unwanted reverse transcription of the RNA prior to its encapsulation in droplets, RNA and RT reagents were first emulsified separately and mixed together through droplet fusion (Mazutis *et al*, 2009). Moreover, to evaluate the sensitivity of the reverse transcription process, we prepared emulsions containing 10, 100 or 1000 RNA molecules per droplet. Then the reverse transcription was added to each droplet and the RT allowed to proceed prior to analyzing the produced cDNA.

### Experimental procedure

0.6, 6 or 60 femto moles of RNA-III (allowing for having respectively, 10, 100 or 1000 RNA molecule per 2 pL droplet) were introduced into a 100 µL of a solution containing CutSmart® buffer, 1.5 mg/mL Dextran Texas Red (Invitrogen), 0.25% Pluronic F68. The mixture was then loaded into a length of PTFE tubing (I.D. 0.75 mm tubing; Thermo Scientifc) and connected to the Fluigent infusion device at one side of the tubing while the other the other side was connected to a 10 µm deep droplet generator **(****Figure 32****, inlet 1).** An oil phase made of Novec 7500 supplemented with 3 % Krytox-Jeffamine 1000 diblock copolymer fluorosurfactant was also infused into the chip **(****Figure 32****, inlet 2)** and used to produce 2 pL droplets at a rate of 9000 droplets per second by infusing oil and aqueous phases at 1450 nL/min and 700 nL/min respectively. Droplets were collected **(****Figure 32****, outlet 3)** *via* a length of tubing into a 0.2 mL PCR tube closed by a plug of PDMS. 200 µL of reverse transcription mixture were prepared by supplementing a CutSmart® buffer prepared at the recommended concentration with 1.25 pmols of RT-UMI-RNAIII primer (molecule **18**), 2.5 pmol of AntiSBACA primer (molecule **14**), 0.25 mM of each dNTP, 10 mM DTT, 0.1 µM FAM (droplet tracker) and 125 U of Reverse Transcriptase Maxima RNase H minus (Thermo Scientific). The mixture was loaded into a length of PTFE tubing (I.D. 0.75 mm tubing; Thermo Scientifc) and connected to the Fluigent infusion device at one side of the tubing while the other the other side was connected to a 15 µm deep droplet fusion device **(****Figure 33****, inlet 1).** An oil phase made of Novec 7500 supplemented with 2 % Krytox-Jeffamine 1000 diblock copolymer fluorosurfactant was also infused into the chip **(****Figure 33****, inlet 2)** and used to produce 18 pL droplets at a frequency of 1200 droplets per second by infusing oil and aqueous phases at 1280 nL/min and 800 nL/min respectively. Moreover, the 2 pL droplets containing the RNA were also reinjected into the same chip **(****Figure 33****, inlet 3)** at a frequency of ∼ 1200 droplets per second by infusing them at 140 nL/min and spacing them with a stream of surfactant-free Novec 7500 fluorinated oil (3M) infused into the chip **(****Figure 33****, inlet 4)** at 1400 nL/min. Pairs of droplet were formed and fused by a squared AC field (450 V, 30 Hz) that was applied to built-in electrodes and obtained from a function generator connected to a high voltage amplifier (TREK Model 623B). Fused droplets were then collected **(****Figure 33****, outlet 5)** under mineral oil. In parallel, the same reaction was performed in bulk by mixing 1 volume of RNA dilution with 9 volumes of reverse transcription mixture and by incubating both the bulk mixture and the emulsions for 1 hour at 55°C. Furthermore, a second control reaction in which the reverse transcriptase was omitted was also performed. Upon incubation, emulsions were broken using of 1H, 1H, 2H, 2H, perfluoro-1-octanol (Sigma-Aldrich) and the aqueous phases were recovered. cDNA obtained from the different conditions (bulk or emulsion; 10, 100 or 1000 RNAs per droplet) was then analyzed by qPCR using the SsoFast Evagreen Supermix kit (Bio-Rad) supplemented with the primers **20** and **21.** Moreover, the amplification products were analyzed on an 8% native polyacrylamide gel.

### Results

In this example, emulsions of 2 pL droplets containing each 10, 100 or 1000 molecules of RNA-III were produced and fused to 18 pL droplets containing an RT mixture. As a control, the same experiment was performed in a bulk format. Upon incubation, qPCR analysis revealed that RT occurred with the same efficiency both in bulk and emulsified format **(****Figure 34****).** Indeed, in both formats Ct values obtained with bulk and emulsified reactions were very close and significantly higher than that of a control reaction where the reverse transcriptase was omitted. Moreover, the analysis on gel confirmed that the qPCR product obtained in both conditions had the expected size, whereas only a low size PCR side product was obtained with the negative control. Interestingly, in the condition where the RNA was the most diluted (10 molecules per 2 pL) gave a detectable signal corresponding to an amplification product of the expected size only in emulsion demonstrating the higher sensitivity offered by the droplet format and suggesting that if a target RNA is present in at least 10, it should be possible to convert it into a cDNA displaying an extremity compatible with the grafting of a UI. Indeed, in this example we validated the use of an RT primer possessing the sequences elements allowing for efficiently grafting an UI using the strategy validated in Example 3 (compare **Figure 24** and **31**).

### Example 5: Exploring alternative format of UEI-Calibrator and UEI sequences

We noticed that, when working at low concentration of target molecules, some non-specific recombination events occur between some primers and the randomized regions of the UEI-Calibrators and the UEIs. We therefore explored alternative sequences to the simple stretch of 15 contiguous randomized nucleotides. In this example, we reverse transcribed a purified gfp mRNA prior to subjecting the obtained cDNA the labeling by UI produced from templates: i) deprived of randomized regions; ii) or bearing a stretch of 15 contiguous randomized nucleotides (N15); iii) or bearing a stretch of semi-randomized nucleotides where 5 randomized dinucleotides are spaced by constant dinucleotides (N2x5); iv) or bearing a stretch of semi-randomized nucleotides where 4 randomized trinucleotides are spaced by constant trinucleotides (N4443).

### Reverse transcription of the GFP RNA and UI addition

### Experimental procedure

gfp mRNA was prepared by *in vitro* transcription using T7 RNA polymerase using the same procedure described in (Ryckelynck *et al*, 2015). Upon transcription, RNA was purified on a size exclusion column Nap5 (GE-Healthcare) and quantified using a Nanodrop device. 16.67 femto moles of purified gfp mRNA were reverse transcribed in 20 µL CutSmart® buffer prepared at the recommended concentration and supplemented with 1.25 pmol of RT primer (molecule **23**), 1.25 pmol of the complementary oligonucleotide (molecule **24**), 1 mM dNTP, 10 mM DTT, 10 U of AMV reverse transcriptase, 0.1 µM FAM. The mixture was then incubated 1 hour at 42°C. 500 atto moles of pairs of template UEI-Calibrators and UEIs (i.e. **1/27, 25/28, 26/29** and **7/30**) were co-amplified in 200 µL of amplification mixture supplemented with 0.2 µM of each primer (**2**, **5**, **10** and **12**) as described in **Example 3-section a.** Upon thermocycling, 10 µL duplex PCR were mixed with 4 µL of reverse transcribed reaction and 3 µL of Enzyme mixture (1x CutSmart® buffer with 6 mM rATP, 60 mM DTT, 8 U/µL DraIII HF, 8 U/µL AlwN1, 80 U/µL T4 DNA ligase, 55 µM coumarin acetic and 0.3% pluronic F68). The mixture was then subjected to 5 cycles of temperature: 15 min at 37°C and 45 min at 16°C. At the end of the incubation, 1 µL of each reaction was amplified by PCR using the kit Sso-Fast Evagreen (Bio-Rad) supplemented in primers **3** and **6** and the amplification products were analyzed on a 1% agarose gel **(****Figure 35****).**

### Results & conclusions

The target RNA (gfp mRNA) was properly reversed transcribed and the 4 types of barcodes (UEI-Calibrator/UEI) were appended to the resulting cDNA. Whereas barcodes deprived of randomized regions or affording N2x5 semi-randomized regions gave homogeneous PCR products (lanes 1 and 2 on **Figure 35**), more smeary bands were observed with the N4443 and N15 regions. Moreover, secondary amplification products of smaller size tend to significantly accumulate with N4443 and N15. Therefore, semi-randomized barcodes such as the N2x5 represent an attractive alternative to the more conventional N15 barcode design.

### Example 6: Labelling proteins with UIs

To extend the use of the technology to molecules other than nucleic acids, we validated in this example the capacity of our method to encode a DNA fragment covalently attached to a protein. To this end, we cloned a fusion gene made of protein A and SNAP tag (New England Biolabs) coding regions. The resulting gene was overexpressed in *E. coli* and the resulting protein (NaBAb) was purified. In this example, we first verified that a unique fragment of DNA could be covalently attached to the protein and that it did not interfere with the capacity of the protein to interact with antibodies *via* its protein A moiety. Then, we tested if a UI could be attached to the DNA strand appended to the protein.

### a. Labelling antibodies with a single DNA per antibody

We prepared a construct in which the sequence coding for the protein A was placed in fusion with a His-tagged version of the SNAP-tag (New England Biolabs) and placed the construct on an expression plasmid. The corresponding protein was then overproduced in E. *coli* and purified by affinity chromatography. The SNAP domain is a catalytic module reacting with benzyl-guanine-displaying substrate molecules. Upon reaction, a single molecule of substrate is covalently and irreversibly attached to the SNAP module **(****Figure 36****).** Moreover, this protein modified with a single DNA molecule should also be able to strongly associate with antibodies *via* its protein A domain. In this work, we show that our fusion protein is indeed able to be labelled by a single DNA molecule and that the resulting complex is still able to interact with antibodies.

### Experimental procedure

A template DNA (molecule **31**) possessing a unique AlwNI as well as a UMI of 8 randomized positions was PCR amplified using a primer modified with a benzyl-guanine (BG) group (molecule **32**) allowing for grafting the DNA on the SNAP module of NaBAb and a primer modified with an Alexa-488 fluorescent group (molecule **33**) to fluorescently label the DNA. 10 pmols of template **31** were mixed with 1 nmol of each primer (**32** and **33**) in 1 mL of PCR solution containing 0.2 mM of each dNTP, 20 U of Q5 DNA polymerase (New England Biolabs) and the corresponding buffer at the recommended concentration. The mixture was then placed in thermocycler and subjected to an initial denaturation step of 30 sec at 98°C followed by 25 cycles of 10 sec at 98°C, 10 sec at 55°C and 30 sec at 72°C. Finally, the program was concluded by a final extension step of 2 min at 72°C. Amplification products were the purified using the kit Wizard SV Gel and PCR clean up system (Promega) and quantified with a Nanodrop device. 200 pmols of BG/Alexa488 dually labelled DNA were then mixed with 180 pmol of purified NaBAb in 1 mL of CutSmart® buffer (New England Biolabs) diluted at the recommended concentration (1x) and supplemented with 1 mM DTT. The same experiment in which NaBAb was omitted was performed in parallel and used as control. The mixture was incubated for an hour at 37°C and an aliquot was analyzed on polyacrylamide gel electrophoresis and Alexa488 fluorescence revealed **(****Figure 37****, left panel).**

In a second experiment, 27 pmols of purified NaBAb were mixed with 20 pmols of BG/Alexa488 dually labelled DNA in 40 µL of CutSmart® buffer (New England Biolabs) diluted at the recommended concentration (1x) and supplemented with 1 mM DTT. Moreover, the mixture was also supplemented with 225, 112, 62.5 µg/mL of total Human IgG (Sigma Aldrich). Reactions in which IgG and/or the dually labelled DNA were omitted were used as control. The mixture was incubated for an hour at 37°C and an aliquot was analyzed on polyacrylamide gel electrophoresis and Alexa488 fluorescence revealed **(****Figure 37****, right panel).**

### Results & conclusions

Incubating BG/Alexa488 dually labelled DNA with a slight excess of NaBAb allowed grafting all the DNA onto the SNAP module of NaBAb as attested by the complete up-shift of the DNA band on the gel **(****Figure 37****, left panel).** Forming the NaBAb-DNA covalent complex in the presence of IgG showed that NaBAb-DNA/IgG ternary complex can readily form **(****Figure 37****, right panel, lanes 3 to 5).** Moreover, considering the average molecular weight of IgG to be ∼ 150 kDa one can see that the 27 pmols of NaBAb-DNA complex are completely shifted in the presence of an equimolar amount of IgG (125mg/mL or 30 pmols IgG, lane 4) whereas only half of the complex is shifted using twice less IgG. These data indicate that not only NaBAb can be covalently linked to a single DNA molecule, but mixing it stoichiometrically with an IgG allows forming a NaBAb-DNA/IgG ternary complex in which one antibody molecule is predominantly labelled with a single DNA molecule.

### b. Labelling NaBAb-DNA complex with UEIs

We investigated the possibility of labelling the NaBAb-DNA complex by UIs using the labelling strategy presented in Example 3.

### Experimental procedure

500 atto moles of template UEI-Calibrators and UEIs (molecules **11** and **15**) were co-amplified in 200 µL of PCR mixture supplemented with 0.2 µM of each primer (**2, 5, 10** and **12**) as described in **Example 3-section a.** Upon thermocycling, 10 µL duplex PCR were added to 10 µL of CutSmart® buffer (New England Biolabs) diluted at the recommended concentration (1x) supplemented with 2 pmol of NaBAb-DNA complex (prepared as described in **section a** of this Example), 2 mM rATP, 20 mM DTT, 2.5 DraIII HF (New England Biolabs), 2.5 U/µL AlwNl (New England Biolabs), 25 U/µL T4 DNA ligase (New England Biolabs), 10 µM coumarin acetic and 0.1% pluronic F68. The mixture was then subjected to 5 cycles of temperature: 15 min at 37°C and 45 min at 16°C. In parallel, we performed a control reaction in which restriction/ligation enzymes were omitted. At the end of the incubation, 1 µL of each reaction was analyzed by quantitative PCR using the kit Sso-Fast Evagreen (Bio-Rad) supplemented in primers **6** and **10,** and the amplification products were analyzed on a 1% agarose gel **(****Figure 38****).**

### Results & conclusions

In this section, we showed that a DNA covalently associated to the NaBAb protein can serve as UI acceptor using or restriction/ligation strategy. Indeed, in absence of the enzymes more than 26 additional amplification cycles (delta Ct > 26) were required the reach the threshold (**Figure 38****,** compare columns - and + enzymes), indicating that there is at least a 33 million-times less UI-target DNA formed in the absence of enzyme. Moreover, gel electrophoresis analysis **(****Figure 38****)** showed that specific band of the expected size is obtained only in the presence of the enzymes (lane 2).

In conclusion, in this example, we demonstrated that the NaBAb fusion protein can be used to specifically label IgG with a unique DNA molecule and that, even though it is covalently attached to the NaBAb protein, this DNA can further serve as UI acceptor using our restriction/ligation strategy. Moreover, since the recombination occurs in native conditions preserving the association of non-covalent complexes (e.g. antibody/antigen), the combined use of NaBAb-DNA/IgG complex and of UI encoding can be used to label and encoded IgG-recognized targets (e.g. protein) and quantify them by Next Generation sequencing (e.g. MiSeq).

### General summary & conclusions

The different examples reported here demonstrate the complete technical feasibility of the method of the invention. Indeed, we demonstrated that the two components (the UEI-Calibrator and the UEI) of Unique Identifiers (UIs) can be efficiently co-amplified in a duplex PCR prior to being recombined together by restriction/ligation reactions to form UIs both in bulk **(Example 2.a)** and emulsified reactions **(Example 2.b).** The pool of UI contained in droplet can then be used as signature allowing for unambiguously assigning the sequences contained in a pool of Next Generation Sequencing data to the droplet they originate from **(Example 2.c).** Moreover, changing the initial number of different template UEI-Calibrators and UEIs was shown to allow adjusting the complexity of the signature **(Example 2.d).**

In addition, we demonstrated that these UI can be further attached to a target DNA molecule displaying a 3' overhang compatible with a restriction site present on the UI both in bulk **(Example 3.a)** and in droplets **(Example 3.b).** Again, the use UI-based signature allowed to unambiguously assign each DNA sequence to the droplet it originated with a great droplet-to-droplet reproducibility.

Moreover, we showed that RNA can be efficiently reverse transcribed into cDNA in droplet **(Example 4.a)** using reverse transcription primers either competent for being later coupled with an UI **(Example 4.b)** or conjugated to an UI prior to being used for reverse transcribing the target RNA **(Example 5).** In both cases, similar yield of RT and UI coupling were obtained. Our encoding strategy is not limited to nucleic acids since we showed that our UI grafting strategy can also be applied to IgG labeled with a single DNA molecule possessing a restriction site making it compatible with our restriction/ligation approach **(Example 6).** This last scenario makes possible to encode non-nucleic acids targets such as proteins in a way that preserve non-covalent biological complexes (e.g. antibody/antigen complexes).

Finally, we showed that cells can be isolated and lysed into small 4 pL droplets **(Example 1).** Not only the material can be released from the cells, but the size of the droplets makes the directly compatible with the use of the droplet fusion device **(****Figure 33****).** Therefore, cell extract can be directly supplemented with the reagents allowing for reverse transcribing target RNAs and/or antibodies coupled to a single DNA molecule. Upon reverse transcription/antigen capture, a different set of UEI-Calibrators/UEIs can then be delivered to each droplet together with the restriction/ligation enzymes using the Barcoding chip **(****Figure** 27) validated in Example 3. Finally, upon indexing and next generation sequencing the use of our bioinformatics algorithm **(****Figure** 29) allows to reassign each sequence (initially corresponding to an RNA, a protein or another molecule) to the droplet it originates from and this way allows for quantifying the content of each cell.

### References

Benito, Y., et al. RNA, 2000. 6(5): p. 668-79.
Brown, R. B. & Audet, J. R. Soc. Interface 5, S131-S138 (2008)
Islam et al. Nat. Protoc. 2012;7:813-828.
Kintses et al. Chem. Biol. 2012. 19, 1001-1009
Klein et al., Cell. 2015. 161, 1187-1201
Macosko et al. Cell. 2015, 161, 1202-1214
Mazutis, L., et al., Analytical Chemistry, 2009. 81(12): p. 4813-21.
Novak et al. Angew. Chem. Int. Ed. 50, 390-395 (2011)
Rau et al. Appl. Phys. Lett. 2004. 84, 2940-2942
Rotem et al., PLoS ONE, 2015, 10(5): e0116328
Ryckelynck, M., et al., RNA, 2015. 21(3): p. 458-69.
Siegel, A.C., et al., Advanced Materials, 2007. 19(5): p. 727-733.
Streets et al. Proc. Natl. Acad. Sci. USA, 2014, 111, 7048-7053
Xia, Y.N. and G.M. Whitesides, Soft lithography. Angewandte Chemie-International Edition, 1998. 37(5): p. 551-575.

## Claims

1. A method of labelling a plurality of molecular targets from a plurality of entities while preserving the integrity of the single-entity information, said method comprising
providing a first set of emulsion droplets comprising droplets containing labelled molecular targets, wherein each of these droplets contains a plurality of molecular targets originating from no more than one entity and wherein, in each of these droplets, each molecular target is labelled with a molecular identification DNA sequence comprising (i) a unique molecular identification (UMI) barcode which is different for each molecular target and (ii) an overhang or an overhang producing restriction site,;
providing a second set of emulsion droplets comprising droplets containing entity identification sequences, wherein each of these droplets contains at least one entity identification sequence which is a DNA sequence comprising a unique entity identification (UEI) barcode which is different for each droplet of the second set, and an overhang producing restriction site;
fusing droplets of the first set with droplets of the second set wherein a droplet of the first set is fused with no more than one droplet of the second set; and
ligating UEI barcodes to labelled molecular targets after restriction enzyme digestion, and
optionally breaking the emulsion,
wherein the entity is a cell, a particle or an emulsion droplet.

2. The method of claim 1, wherein the method further comprises
encapsulating a plurality of entities within emulsion droplets, each droplet containing no more than one entity, and optionally lysing said entities within the droplets to release molecular targets;
labelling said molecular targets with probes, each probe comprising
a capture moiety capable of specific binding or ligation to a molecular target or to an adaptor linked to said molecular target, and
a DNA moiety comprising (i) a region proximal to the capture moiety and comprising the unique molecular identification (UMI) sequence and (ii) a region distal from the capture moiety and comprising an overhang or an overhang producing restriction site,
thereby obtaining the first set of emulsion droplets.

3. The method of claim 1 or 2, wherein the method further comprises encapsulating a plurality of entity identification sequences within emulsion droplets, each droplet containing no more than one entity identification sequence, with an amplification reaction mixture, and
amplifying the entity identification sequences within droplets
thereby obtaining the second set of emulsion droplets.

4. The method of claim 1 or 2, wherein the method further comprises
encapsulating a plurality of entity identification sequences within emulsion droplets in the presence of UEI-calibrators, wherein at least some droplets comprise one or several entity identification sequences and one or several UEI-calibrators, and wherein said UEI-calibrators are DNA sequences comprising a unique calibrator barcode which is different for each UEI-calibrator and for each droplet, and one or two overhang producing restriction sites; and
amplifying entity identification sequences and/or UEI-calibrators within droplets;
thereby obtaining the second set of emulsion droplets.

5. The method of claim 4, wherein, after fusion of droplets of the first and second sets, (i) UEI calibrator barcodes and UEI barcodes and (ii) UEI barcodes and labelled molecular targets are assembled through restriction enzyme digestion and ligation of compatible overhangs of (i) UEI calibrators and entity identification sequences and of (ii) entity identification sequences and labelled molecular targets.

6. The method of claim 4, wherein, after fusion of droplets of the first and second sets, UEI calibrator barcodes ,UEI barcodes and labelled molecular targets are assembled through restriction enzyme digestion and ligation of compatible overhangs of (i) UEI calibrators and labelled molecular targets and (ii) UEI calibrators and entity identification sequences, or of i) UEI calibrators and entity identification sequences and (ii) entity identification sequences and labelled molecular targets.

7. The method of claim 4, wherein entity identification sequences and UEI-calibrators are assembled through their compatible overhangs before amplification and, after fusion of droplets of the first and second sets, the amplified fragment comprising UEI calibrator and UEI barcodes is ligated to labelled molecular targets through compatible overhangs of i) UEI calibrators and labelled molecular targets or (ii) entity identification sequences and labelled molecular targets.

8. The method of any of claims 1 to 7, wherein at least some of molecular targets are nucleic acids and at least some probes comprise
a capture moiety which is a single stranded DNA region which drives the specific recognition of a nucleic acid molecular target through conventional Watson-Crick base-pairing interactions and
a DNA moiety comprising a 3' single stranded region comprising the unique molecular identification (UMI) sequence and a 5'double-stranded region comprising the overhang or overhang producing restriction site.

9. The method of claim 8, wherein said nucleic acid molecular targets are labelled using said probes as priming sites for a DNA polymerase synthetizing complementary strands of molecular targets.

10. The method of claim 8 or 9, wherein at least some of molecular targets are RNA molecules and the DNA polymerase is a reverse transcriptase.

11. The method of any of claims 2 to 10, wherein at least some probes comprise a capture moiety which is
(i) a binding moiety that specifically binds to a molecular target and is directly bound to the DNA moiety,
(ii) a chimeric protein comprising a first domain that specifically binds to a molecular target and a second domain that binds to the DNA moiety, or
(iii) a binding moiety that binds specifically to a molecular target and a protein bridge, said protein bridge comprising a first domain that binds to the binding moiety and a second domain that binds to the DNA moiety.

12. The method of claim 11, wherein (i) the binding moiety or the first domain of the chimeric protein is selected from the group consisting of an antibody, a ligand of a ligand/anti-ligand couple, a peptide aptamer, a nucleic acid aptamer, a protein tag, or a chemical probe (e.g. suicide substrate, activity-based probes ABP) reacting specifically with a molecular target or a class of molecular targets, preferably is an antibody, (ii) the first domain of the protein bridge is an immunoglobulin-binding bacterial protein, preferably is domains A to E of protein A, and/or (iii) the second domain of the protein bridge or the chimeric protein is selected from the group consisting of SNAP-tag, CLIP-tag or Halo-Tag, preferably is a SNAP-tag.

13. The method of any of claims 2 to 12, wherein at least some probes comprise a capture moiety comprising an antibody moiety specific to a molecular target and a protein bridge, said protein bridge comprising a first domain that binds to a Fc region of the antibody moiety and a second domain that binds to the DNA moiety, preferably a SNAP-tag.

14. A method of quantifying one or several molecular targets from a plurality of entities with single-entity resolution, said method comprising
labelling said molecular targets according to the method of any of claims 1 to 13;
capturing said labelled molecular targets,
amplifying sequences comprising UMI and UEI barcodes, and optionally UEI-calibrator barcodes
sequencing amplified sequences.

15. The method of any of claims 1 to 14, wherein the entity is a particle or an emulsion droplet exposing molecular targets on its outer surface, and the method further comprises
- labelling said molecular targets with probes, each probe comprising
a capture moiety capable of specific binding or ligation to a molecular target or to an adaptor linked to said molecular target, and
a DNA moiety comprising (i) a region proximal to the capture moiety and comprising the unique molecular identification (UMI) sequence and (ii) a region distal from the capture moiety and comprising an overhang or an overhang producing restriction site, and
- encapsulating entities attached to labelled molecular targets within emulsion droplets, each droplet containing no more than one entity,
thereby obtaining the first set of emulsion droplets.

16. Use of a kit to label a plurality of molecular targets from a plurality of entities according to the method of any of claims 1 to 13 and 15, or to quantify one or several molecular targets from a plurality of entities with single-entity resolution according to the method of claim 14 or 15, wherein the kit comprises
- a microfluidic device and/or,
- one or several probes as defined in claim 2; and/or
- one or several entity identification sequences as defined in claim 1; and/or
- one or several UEI-calibrators as defined in claim 4; and/or
- one or several primers suitable to amplify entity identification sequences and/or UEI-calibrators; and/or
- an aqueous phase and/or an oil phase; and optionally
- a leaflet providing guidelines to use such a kit.

## Patentansprüche

1. Ein Verfahren zur Markierung einer Vielzahl an molekularen Zielen von einer Vielzahl an Einheiten unter Bewahrung der Integrität der Einheitsinformation, wobei das Verfahren umfasst:
Bereitstellen eines ersten Satzes an Emulsionstropfen, umfassend molekulare Ziele enthaltende Tropfen, wobei jeder dieser Tropfen eine Vielzahl an molekularen Zielen enthält, die von nicht mehr als einer Einheit stammen, und wobei, in jedem dieser Tropfen, jedes molekulare Ziel mit einer molekularen Identifizierungs-DNA-Sequenz markiert ist, umfassend (i) einen eindeutigen molekularen Identifizierungs-(UMI)-Barcode, der für jedes molekulare Ziel verschieden ist, und (ii) einen Überhang oder einen Überhang erzeugende Restriktionsstelle;
Bereitstellen eines zweiten Satzes an Emulsionstropfen, umfassend Einheitsidentifizierungssequenzen enthaltende Tropfen, wobei jeder dieser Tropfen wenigstens eine Einheitsidentifizierungssequenz enthält, die eine DNA-Sequenz ist, umfassend einen eindeutigen Einheitsidentifizierungs-(UEI)-Barcode, der für jeden Tropfen des zweiten Satzes verschieden ist, und einen Überhang erzeugende Restriktionsstelle;
Fusionieren von Tropfen des ersten Satzes mit Tropfen des zweiten Satzes, wobei ein Tropfen des ersten Satzes mit nicht mehr als einem Tropfen des zweiten Satzes fusioniert wird; und
Ligieren von UEI-Barcodes an markierte molekulare Ziele nach Restriktionsenzymverdau, und gegebenenfalls Brechen der Emulsion,
wobei die Einheit eine Zelle, ein Partikel oder ein Emulsionstropfen ist.

2. Das Verfahren nach Anspruch 1, wobei das Verfahren außerdem umfasst:
Verkapseln einer Vielzahl an Einheiten in Emulsionstropfen, wobei jeder Tropfen nicht mehr als eine Einheit umfasst und gegebenenfalls Lysieren der Einheiten in den Tropfen, um molekulare Ziele freizusetzen;
Markieren der molekularen Ziele mit Sonden, wobei jede Sonde umfasst eine Einfangkomponente, die in der Lage ist, an ein molekulares Ziel oder an einen Adaptor, der an das molekulare Ziel gebunden ist, zu binden oder zu ligieren, und
eine DNA-Komponente, umfassend (i) eine Region proximal der Einfangkomponente und umfassend die eindeutige molekulare Identifizierungs-(UMI)-Sequenz und (ii) eine Region distal der Einfangkomponente und umfassend einen Überhang oder einen Überhang erzeugende Restriktionsstelle,
wobei dadurch der erste Satz an Emulsionstropfen erhalten wird.

3. Das Verfahren nach Anspruch 1 oder 2, wobei das Verfahren außerdem Verkapselung einer Vielzahl an Einheitsidentifizierungssequenzen in Emulsionstropfen, wobei jeder Tropfen nicht mehr als eine Einheitsidentifizierungssequenz enthält, mit einer Amplifikationsreaktionsmischung umfasst, und
Amplifizieren der Einheitsidentifizierungssequenzen in den Tropfen,
wobei dadurch der zweite Satz an Emulsionstropfen erhalten wird.

4. Das Verfahren nach Anspruch 1 oder 2, wobei das Verfahren außerdem umfasst:
Verkapselung einer Vielzahl an Einheitsidentifizierungssequenzen in Emulsionstropfen in Gegenwart von UEI-Kalibratoren, wobei wenigstens einige Tropfen eine oder mehrere Einheitsidentifizierungssequenzen und einen oder mehrere UEI-Kalibratoren umfassen, und wobei die UEI-Kalibratoren DNA-Sequenzen sind, die einen eindeutigen Kalibrator-Barcode, der für jeden UEI-Kalibrator und für jeden Tropfen verschieden ist, und eine oder zwei Überhang erzeugende Restriktionsstellen umfassen; und
Amplifizieren von Einheitsidentifizierungssequenzen und/oder UEI-Kalibratoren in den Tropfen; wobei dadurch der zweite Satz an Emulsionstropfen erhalten wird.

5. Das Verfahren nach Anspruch 4, wobei, nach Fusion von Tropfen des ersten Satzes und zweiten Satzes, (i) UEI-Kalibrator-Barcodes und UEI-Barcodes und (ii) UEI-Barcodes und markierte molekulare Ziele durch Restriktionsenzymverdau und Ligation kompatibler Überhänge von (i) UEI-Kalibratoren und Einheitsidentifizierungssequenzen und von (ii) Einheitsidentifizierungssequenzen und markierten molekularen Zielen zusammengefügt werden.

6. Das Verfahren nach Anspruch 4, wobei, nach Fusion von Tropfen des ersten Satzes und zweiten Satzes, UEI-Kalibrator-Barcodes, UEI-Barcodes und markierte molekulare Ziele durch Restriktionsenzymverdau und Ligation kompatibler Überhänge von (i) UEI-Kalibratoren und markierten molekularen Zielen und (ii) UEI-Kalibratoren und Einheitsidentifizierungssequenzen oder von (i) UEI-Kalibratoren und Einheitsidentifizierungssequenzen und (ii) Einheitsidentifizierungssequenzen und markierten molekularen Zielen zusammengefügt werden.

7. Das Verfahren nach Anspruch 4, wobei Einheitsidentifizierungssequenzen und UEI-Kalibratoren durch ihre kompatiblen Überhänge vor Amplifikation zusammengefügt werden und, nach Fusion von Tropfen des ersten Satzes und zweiten Satzes, das amplifizierte Fragment, umfassend UEI-Kalibrator und UEI-Barcodes, an markierte molekulare Ziele durch kompatible Überhänge von (i) UEI-Kalibratoren und markierten Zielen oder (ii) Einheitsidentifizierungssequenzen und markierten Zielen ligiert wird.

8. Das Verfahren nach einem der Ansprüche 1 bis 7, wobei wenigstens einige molekularen Ziele Nukleinsäuren sind und wenigstens einige Sonden umfassen:
eine Einfangkomponente, die eine einzelsträngige DNA-Region ist, welche die spezifische Erkennung eines molekularen Nukleinsäure-Ziels durch herkömmliche Watson-Crick-Basenpaarung-Interaktionen steuert,
eine DNA-Komponente, umfassend eine einzelsträngige 3'-Region, umfassend die eindeutige molekulare Identifizierungs-(UMI)-Sequenz, und eine doppelsträngige 5'-Region, umfassend den Überhang oder die Überhang erzeugende Restriktionsstelle.

9. Das Verfahren nach Anspruch 8, wobei die molekularen Nukleinsäure-Ziele unter Verwendung besagter Sonden als Primer-Anlagerungsstellen für eine DNA-Polymerase markiert werden, die Komplementärstränge von molekularen Zielen synthetisiert.

10. Das Verfahren nach Anspruch 8 oder 9, wobei wenigstens einige molekulare Ziele RNA-Moleküle sind und die DNA-Polymerase eine reverse Transkriptase ist.

11. Das Verfahren nach einem der Ansprüche 2 bis 10, wobei wenigstens einige Sonden eine Einfangkomponente umfassen, die
(i) eine bindende Komponente ist, die spezifisch an ein molekulares Ziel bindet und direkt an die DNA-Komponente gebunden ist,
(ii) eine chimäres Protein ist, umfassend eine erste Domäne, die spezifisch an ein molekulares Ziel bindet, und eine zweite Domäne, die an die DNA-Komponente bindet, oder
(iii) eine bindende Komponente ist, die spezifisch an ein molekulares Ziel und an eine Proteinbrücke bindet, wobei die Proteinbrücke eine erste Domäne, die an die bindende Komponente bindet, und eine zweite Domäne umfasst, die an die DNA-Komponente bindet.

12. Das Verfahren nach Anspruch 11, wobei (i) die bindende Komponente oder die erste Domäne des chimären Proteins aus der Gruppe ausgewählt ist, bestehend aus einem Antikörper, einem Liganden eines Ligand/Anti-Ligand-Paares, einem Peptid-Aptamer, einem Nukleinsäure-Aptamer, einem Protein-Tag oder einer chemischen Sonde (z.B. Suizidsubstrat, aktivitätsbasierte Sonden, ABP), die spezifisch mit einem molekularen Ziel oder einer Klasse molekularer Ziele reagieren, vorzugsweise ein Antikörper ist, (ii) die erste Domäne der Proteinbrücke ein Immunglobulin-bindendes bakterielles Protein ist, vorzugsweise Domänen A bis E von Protein A ist, und/oder (iii) die zweite Domäne der Proteinbrücke oder des chimären Proteins aus der Gruppe ausgewählt ist, bestehend aus SNA-Tag, CLIP-Tag oder Halo-Tag, vorzugsweise ein SNAP-Tag ist.

13. Das Verfahren nach einem der Ansprüche 2 bis 12, wobei wenigstens einige Sonden eine Einfangkomponente umfassen, die eine Antikörperkomponente, die für ein molekulares Ziel spezifisch ist, und eine Proteinbrücke umfasst, wobei die Proteinbrücke eine erste Domäne, die an eine Fc-Region der Antikörperkomponente bindet, und eine zweite Domäne, die an die DNA-Komponente bindet, vorzugsweise ein SNAP-Tag umfasst.

14. Ein Verfahren zur Quantifizierung eines oder mehrerer molekularer Ziele von einer Vielzahl an Einheiten mit Einheitsauflösung, wobei das Verfahren umfasst:
Markieren der molekularen Ziele gemäß dem Verfahren nach einem der Ansprüche 1 bis 13; Einfangen der markierten molekularen Ziele,
Amplifizieren von Sequenzen, umfassend UMI- und UEI-Barcodes und gegebenenfalls UEI-Kalibrator-B arcodes
Sequenzieren amplifizierter Sequenzen.

15. Das Verfahren nach einem der Ansprüche 1 bis 14, wobei die Einheit ein Partikel oder ein Emulsionstropfen ist, das/der molekulare Ziele auf seiner Außenseite exponiert, und das Verfahren außerdem umfasst:
- Markieren der molekularen Ziele mit Sonden, wobei jede Sonde umfasst:
eine Einfangkomponente, die in der Lage ist, an ein molekulares Ziel oder an einen Adaptor, der an das molekulare Ziel gebunden ist, zu binden oder zu ligieren, und
eine DNA-Komponente, umfassend (i) eine Region proximal der Einfangkomponente und umfassend die eindeutige molekulare Identifizierungs-(UMI)-Sequenz und (ii) eine Region distal der Einfangkomponente und umfassend einen Überhang oder einen Überhang erzeugende Restriktionsstelle, und
- Verkapselung von Einheiten, die an den markierten molekularen Zielen in Emulsionstropfen gebunden sind, wobei jeder Tropfen nicht mehr als eine Einheit enthält,
wobei dadurch der erste Satz an Emulsionstropfen erhalten wird.

16. Verwendung eines Kits zur Markierung einer Vielzahl an molekularen Zielen von einer Vielzahl an Einheiten gemäß dem Verfahren nach einem der Ansprüche 1 bis 13 und 15 oder zur Quantifizierung eines oder mehrerer molekularer Ziele von einer Vielzahl an Einheiten mit Einheitsauflösung gemäß dem Verfahren nach Anspruch 14 oder 15, wobei das Kit umfasst:
- eine Mikrofluidvorrichtung und/oder
- eine oder mehrere Sonden, definiert wie in Anspruch 2; und/oder
- eine oder mehrere Einheitsidentifizierungssequenzen, definiert wie in Anspruch 1; und/oder
- einen oder mehrere UEI-Kalibratoren, definiert wie in Anspruch 4; und/oder
- einen oder mehrere Primer, die zum Amplifizieren von Einheitsidentifizierungssequenzen und/oder UEI-Kalibratoren geeignet sind; und/oder
- eine wässrige Phase und/oder Ölphase; und gegebenenfalls
- eine Packungsbeilage mit Anleitungen zur Verwendung eines derartigen Kits.

## Revendications

1. Procédé de marquage d'une pluralité de cibles moléculaires provenant d'une pluralité d'entités tout en préservant l'intégrité de l'information au niveau de l'entité unique, ledit procédé comprenant
la fourniture d'un premier ensemble de gouttelettes d'émulsion comprenant des gouttelettes contenant des cibles moléculaires marquées, dans lequel chacune de ces gouttelettes contient une pluralité de cibles moléculaires provenant d'au plus une entité et dans lequel, dans chacune de ces gouttelettes, chaque cible moléculaire est marquée avec une séquence ADN d'identification moléculaire comprenant (i) un code- barres d'identification moléculaire unique (UMI) qui est différent pour chaque cible moléculaire et (ii) un surplomb ou un site de restriction produisant un surplomb;
la fourniture d'un deuxième ensemble de gouttelettes d'émulsion comprenant des gouttelettes contenant des séquences d'identification d'entité, dans lequel chacune de ces gouttelettes contient au moins une séquence d'identification d'entité qui est une séquence d'ADN comprenant un code-barres d'identification d'entité unique (UEI) qui est différent pour chaque gouttelette du deuxième ensemble, et un site de restriction produisant un surplomb ;
la fusion de gouttelettes du premier ensemble avec des gouttelettes du second ensemble, dans laquelle une gouttelette du premier ensemble est fusionnée avec au plus une gouttelette du second ensemble ; et
la ligature des codes-barres UEI à des cibles moléculaires marquées après digestion par enzyme de restriction,
et
éventuellement la cassure de l'émulsion,
où l'entité est une cellule, une particule ou une gouttelette d'émulsion.

2. Procédé selon la revendication 1, dans lequel le procédé comprend en outre
l'encapsulation d'une pluralité d'entités dans des gouttelettes d'émulsion, chaque gouttelette ne contenant pas plus d'une entité, et éventuellement la lyse desdites entités dans les gouttelettes pour libérer des cibles moléculaires ;
le marquage desdites cibles moléculaires avec des sondes, chaque sonde comprenant
un élément de capture capable de se lier ou de se ligaturer spécifiquement à une cible moléculaire ou à un adaptateur lié à ladite cible moléculaire, et
un élément ADN comprenant (i) une région proximale à l'élément de capture et comprenant la séquence d'identification moléculaire unique (UMI) et (ii) une région distale de l'élément de capture et comprenant un surplomb ou un site de restriction produisant un surplomb,
obtenant ainsi le premier ensemble de gouttelettes d'émulsion.

3. Procédé selon la revendication 1 ou 2, dans lequel le procédé comprend en outre l'encapsulation d'une pluralité de séquences d'identification d'entités dans des gouttelettes d'émulsion, chaque gouttelette ne contenant pas plus d'une séquence d'identification d'entité, avec un mélange réactionnel d'amplification, et
l'amplification des séquences d'identification d'entités dans les gouttelettes obtenant ainsi le deuxième ensemble de gouttelettes d'émulsion.

4. Procédé selon la revendication 1 ou 2, dans lequel le procédé comprend en outre
l'encapsulation d'une pluralité de séquences d'identification d'entités dans des gouttelettes d'émulsion en présence de calibrateurs-UEI, dans lequel au moins certaines gouttelettes comprennent une ou plusieurs séquences d'identification d'entités et un ou plusieurs calibrateurs-UEI, et dans lequel lesdits calibrateurs-UEI sont des séquences d'ADN comprenant un code-barres de calibrateur unique qui est différent pour chaque calibrateur-UEI et pour chaque gouttelette, et un ou deux sites de restriction produisant un surplomb ; et
l'amplification des séquences d'identification d'entités et/ou des calibrateurs-UEI dans les gouttelettes ;
obtenant ainsi le deuxième ensemble de gouttelettes d'émulsion.

5. Procédé selon la revendication 4, dans lequel, après fusion des gouttelettes des premier et deuxième ensembles, (i) les codes-barres des calibrateurs-UEI et les codes-barres UEI et (ii) les codes-barres UEI et les cibles moléculaires marquées sont assemblés par digestion par enzyme de restriction et ligature des surplombs compatibles (i) des calibrateurs-UEI et des séquences d'identification d'entités et (ii) des séquences d'identification d'entités et des cibles moléculaires marquées.

6. Procédé selon la revendication 4, dans lequel, après fusion des gouttelettes des premier et deuxième ensembles, les codes-barres des calibrateurs-UEI, les codes-barres UEI et les cibles moléculaires marquées sont assemblés par digestion par enzyme de restriction et ligature des surplombs compatibles (i) des calibrateurs-UEI et des cibles moléculaires marquées et (ii) des calibrateurs-UEI et des séquences d'identification d'entités, ou (i) des calibrateurs-UEI et des séquences d'identification d'entités et (ii) des séquences d'identification d'entités et des cibles moléculaires marquées.

7. Procédé selon la revendication 4, dans lequel les séquences d'identification d'entités et les calibrateurs-UEI sont assemblés par leurs surplombs compatibles avant amplification et, après fusion des gouttelettes du premier et du second ensembles, le fragment amplifié comprenant le calibrateur-UEI et les codes-barres UEI est ligaturé à des cibles moléculaires marquées par les surplombs compatibles i) des calibrateurs-UEI et des cibles moléculaires marquées ou ii) des séquences d'identification d'entités et des cibles moléculaires marquées.

8. Procédé selon l'une quelconque des revendications 1 à 7, dans lequel au moins certaines des cibles moléculaires sont des acides nucléiques et au moins certaines sondes comprennent
un élément de capture qui est une région d'ADN simple brin qui entraîne la reconnaissance spécifique d'une cible moléculaire d'acide nucléique par les interactions classiques d'appariement de bases de Watson-Crick et
un élément ADN comprenant une région simple brin 3' comprenant la séquence moléculaire unique d'identification (UMI) et une région double brin 5' comprenant le surplomb ou le site de restriction produisant le surplomb.

9. Procédé selon la revendication 8, dans lequel lesdites cibles moléculaires d'acide nucléique sont marquées en utilisant lesdites sondes comme sites d'amorçage pour une ADN polymérase synthétisant des brins complémentaires de cibles moléculaires.

10. Procédé selon la revendication 8 ou 9, dans lequel au moins certaines des cibles moléculaires sont des molécules d'ARN et l'ADN polymérase est une transcriptase inverse.

11. Procédé selon l'une quelconque des revendications 2 à 10, dans lequel au moins certaines sondes comprennent un élément de capture qui est
(i) un élément de liaison qui se lie spécifiquement à une cible moléculaire et est directement lié à l'élément ADN,
(ii) une protéine chimérique comprenant un premier domaine qui se lie spécifiquement à une cible moléculaire et un second domaine qui se lie à l'élément ADN, ou
(iii) un élément de liaison qui se lie spécifiquement à une cible moléculaire et un pont protéique, ledit pont protéique comprenant un premier domaine qui se lie à l'élément de liaison et un second domaine qui se lie à l'élément ADN.

12. Procédé selon la revendication 11, dans lequel (i) l'élément de liaison ou le premier domaine de la protéine chimérique est choisi dans le groupe constitué par un anticorps, un ligand d'un couple ligand/anti-ligand, un aptamère peptidique, un aptamère d'acide nucléique, une étiquette protéique ou une sonde chimique (par exemple un substrat suicide, des sondes ABP basées sur l'activité) réagissant spécifiquement avec une cible moléculaire ou une classe de cibles moléculaires, de préférence est un anticorps, (ii) le premier domaine du pont protéique est une protéine bactérienne liant l'immunoglobuline, de préférence est les domaines A à E de la protéine A, et/ou (iii) le second domaine du pont protéique ou de la protéine chimérique est choisi dans le groupe constitué par une étiquette SNAP, une étiquette CLIP ou une étiquette Halo, de préférence est une étiquette SNAP.

13. Procédé selon l'une quelconque des revendications 2 à 12, dans lequel au moins certaines sondes comprennent un élément de capture comprenant un élément d'anticorps spécifique d'une cible moléculaire et un pont protéique, ledit pont protéique comprenant un premier domaine qui se lie à une région Fc de l'élément d'anticorps et un second domaine qui se lie à l'élément ADN, de préférence une étiquette SNAP.

14. Procédé de quantification d'une ou plusieurs cibles moléculaires provenant d'une pluralité d'entités avec une résolution au niveau de l'entité unique, ledit procédé comprenant
le marquage desdites cibles moléculaires selon le procédé de l'une quelconque des revendications 1 à 13;
la capture desdites cibles moléculaires marquées,
l'amplification des séquences comprenant des codes-barres UMI et UEI, et éventuellement des codes-barres de calibrateur-UEI
le séquençage des séquences amplifiées.

15. Procédé selon l'une quelconque des revendications 1 à 14, dans lequel l'entité est une particule ou une gouttelette d'émulsion exposant des cibles moléculaires sur sa surface externe, et le procédé comprend en outre
- le marquage desdites cibles moléculaires avec des sondes, chaque sonde comprenant
un élément de capture capable de se lier ou de se ligaturer spécifiquement à une cible moléculaire ou à un adaptateur lié à ladite cible moléculaire, et
un élément d'ADN comprenant (i) une région proximale de l'élément de capture et comprenant la séquence d'identification moléculaire unique (UMI) et (ii) une région distale de l'élément de capture et comprenant un surplomb ou un site de restriction produisant un surplomb, et
- l'encapsulation des entités attachées à des cibles moléculaires marquées dans des gouttelettes d'émulsion, chaque gouttelette ne contenant pas plus d'une entité,
obtenant ainsi le premier ensemble de gouttelettes d'émulsion.

16. Utilisation d'un kit pour marquer une pluralité de cibles moléculaires provenant d'une pluralité d'entités selon le procédé de l'une quelconque des revendications 1 à 13 et 15, ou pour quantifier une ou plusieurs cibles moléculaires provenant d'une pluralité d'entités avec une résolution au niveau de l'entité unique selon le procédé de la revendication 14 ou 15, dans laquelle le kit comprend
- un appareil microfluidique et/ou,
- une ou plusieurs sondes telles que définies dans la revendication 2 ; et/ou
- une ou plusieurs séquences d'identification d'entités telles que définies dans la revendication 1 ; et/ou
- un ou plusieurs calibrateurs-UEI tels que définis dans la revendication 4 ; et/ou
- une ou plusieurs amorces adaptées pour amplifier les séquences d'identification d'entités et/ou les calibrateurs-UEI ; et/ou
- une phase aqueuse et/ou une phase huileuse ; et éventuellement
- une brochure fournissant des lignes directrices pour l'utilisation d'un tel kit.
